# EUROPEAN PATENT SPECIFICATION

(11) **EP 3 833 347 B1**
(45) Date of publication and mention of the grant of the patent: **21.10.2026**
(21) Application number: 19848392.7
(22) Date of filing: 06.08.2019
(51) Int. Cl.: A61K 31/44, A61K 31/4427, C07D 213/79, A61P 35/00

(54) **HISTONE DEMETHYLASE 5 INHIBITORS AND USES THEREOF**
HISTON-DEMETHYLAS-5-INHIBITOREN UND VERWENDUNGEN DAVON
INHIBITEURS D'HISTONE DÉMÉTHYLASE 5 ET UTILISATIONS CORRESPONDANTES

(30) Priority: 06.08.2018 US 201862715122 P; 07.08.2018 US 201862715687 P; 08.02.2019 US 201962803332 P
(43) Date of publication of application: 16.06.2021
(73) Proprietor: Dana-Farber Cancer Institute, Inc., Boston, MA 02215 (US)
(72) Inventor: QI, Jun, Sharon, MA 02067 (US); PARK, Paul, M., Waltham, MA 02453 (US); PEI, Chengkui, Brighton, MA 02135 (US)
(74) Representative: Kraus & Lederer PartGmbB
(86) International application number: PCT/US2019/045259
(87) International publication number: WO 2020/033377

(56) References cited:
- WO-A1-2014/053491
- WO-A1-2014/131777
- WO-A1-95/29907
- US-A1- 2008 312 237
- US-A1- 2016 168 144
- US-A1- 2017 305 920
- US-A1- 2017 320 827
- US-B2- 9 919 998

## Description

### RELATED APPLICATIONS

The present application claims priority under 35 U.S.C. § 119(e) to U.S. provisional applications: U.S.S.N. 62/715,122, filed August 6, 2018, U.S.S.N. 62/715,687, filed August 7, 2018, and U.S.S.N. 62/803,332 filed February 8, 2019.

### BACKGROUND OF THE INVENTION

The overexpression of certain histone lysine demethylases (KDM's) (e.g., KDM's in the JmjC KDM2-7 subfamily) has been linked to cancer. KDM's have also been linked to cell cycle control.

KDM5A (Lysine demethylase 5A; also known as JARID1A/RBP2) is one of the Jumonji C domain-containing demethylases, which recognizes and removes histone H3 lysine 4 di- and tri-methylation (H3K4me2 and H3K4me3), epigenetic marks of transcriptional gene activation. KDM5A is not only involved in development and differentiation but also involved in tumorigenesis, metastasis, and drug resistance in various cancers, either dependently or independently of its catalytic function. KDM5A is highly expressed in multiple myeloma (MM) cell lines and primary MM samples, and higher KDM5A expression is associated with poor prognosis.

WO 2014/053491 discloses compounds capable of modulating the activity of histone demethylases (HDMEs), useful for the treatment of diseases in which genomic dysregulation is involved in the pathogenesis, e.g. cancer.

WO 2014/131777 also discloses compounds capable of modulating the activity of histone demethylases (HDMEs), useful for the treatment of diseases in which genomic dysregulation is involved in the pathogenesis, e.g. cancer.

Thus, in view of the significant role of histone demethylase KDM's (*e.g*., KDM5, which is a family of histone demethylases) in cell cycle control and in various diseases (*e.g.*, cancer), it is important to develop modulators of the activity of these histone demethylases, including selective modulators (*e.g*., selective inhibitors) of KDM's, for use as research tools as well as therapeutic agents in the treatment of various diseases including proliferative diseases such as cancer, and/ or cardiovascular disease.

### SUMMARY OF THE INVENTION

The present invention is set out in the appended set of claims.

In particular, the present invention relates in one aspect to a compound of Formula **(I-B):** 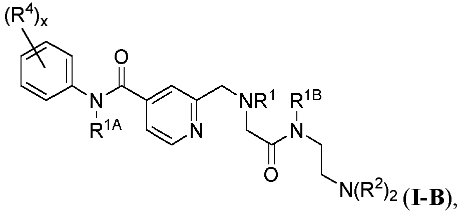 or a pharmaceutically acceptable salt, solvate, hydrate, polymorph, co-crystal, tautomer, stereoisomer, or isotopically labeled derivative thereof, wherein:
R¹ is hydrogen, optionally substituted alkyl, or a nitrogen protecting group;
R^{1A} is hydrogen, optionally substituted acyl, optionally substituted alkyl, optionally substituted carbocyclyl, or a nitrogen protecting group;
R^{1B} is hydrogen, optionally substituted alkyl, optionally substituted carbocyclyl, or a nitrogen protecting group;
each instance of R² is independently optionally substituted alkyl or a nitrogen protecting group;
each R⁴ is independently optionally substituted alkyl, optionally substituted alkenyl, optionally substituted alkynyl, optionally substituted carbocyclyl, optionally substituted heterocyclyl, optionally substituted aryl, optionally substituted heteroaryl, -OR^{A}, -N(R^{B})₂, or -SR^{A}, or optionally two instances of R⁴ are taken together with their intervening atoms to form a substituted or unsubstituted heterocyclic or substituted or unsubstituted heteroaryl ring; and x is 0, 1, 2, 3, 4, or 5; wherein the nitrogen protecting group is Bn, BOC, Cbz, Fmoc, trifluoroacetyl, triphenylmethyl, acetyl, or Ts.

The present invention further relates to the compound as defined above, or a pharmaceutically acceptable salt, solvate, hydrate, polymorph, co-crystal, tautomer, stereoisomer, or isotopically labeled derivative, thereof, which is 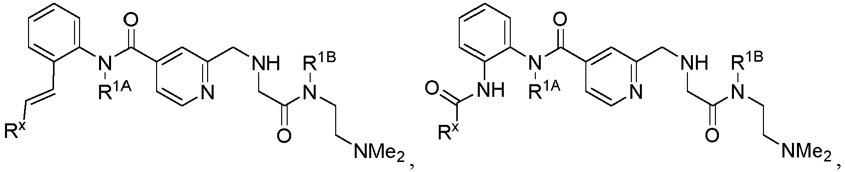 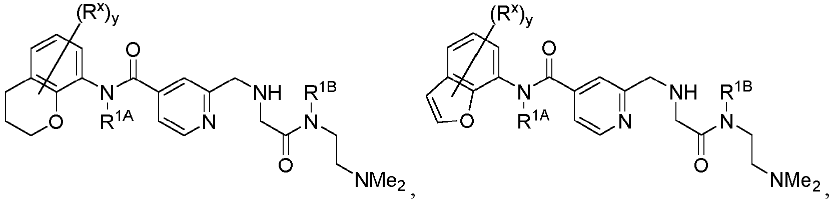 or 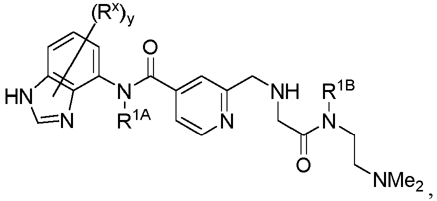 wherein:
R^{x} is optionally substituted acyl, optionally substituted alkyl, -O(optionally substituted alkyl), or -NO₂; and
y is 0, 1, 2, 3, 4, 5, 6, 7, 8, or 9, as valency permits.

The present invention further relates to the compound as defined above, or a pharmaceutically acceptable salt, solvate, hydrate, polymorph, co-crystal, tautomer, stereoisomer, or isotopically labeled derivative thereof, wherein at least one instance of R⁴ is optionally substituted alkenyl, wherein at least one instance of R⁴ is -OR^{A}, and R^{A} is hydrogen, optionally substituted C₁₋₆ alkyl, or optionally substituted aryl, or wherein at least one instance of R⁴ is -NHC(=O)R^{x}, and R^{x} is optionally substituted C₁₋₆ alkyl or optionally substituted alkenyl.

The present invention further relates to the compound as defined above or a pharmaceutically acceptable salt, solvate, hydrate, polymorph, co-crystal, tautomer, stereoisomer, or isotopically labeled derivative, thereof, wherein x is 0, 1 or 2.

The present invention further relates to the compound as defined above or a pharmaceutically acceptable salt, solvate, hydrate, polymorph, co-crystal, tautomer, stereoisomer, or isotopically labeled derivative thereof, wherein
R¹ is hydrogen, or
R^{1B} is optionally substituted C₁₋₆ alkyl or optionally substituted carbocyclyl; or
at least one instance of R² is optionally substituted C₁₋₆ alkyl; or
at least one instance of R² is unsubstituted methyl; or both instances of R² are unsubstituted methyl.

The present invention further relates to the compound as defined above or a pharmaceutically acceptable salt, solvate, hydrate, polymorph, co-crystal, tautomer, stereoisomer, or isotopically labeled derivative thereof, wherein
R¹ is hydrogen and R^{1B} is optionally substituted C₁₋₆ alkyl or optionally substituted carbocyclyl; or
R¹ is hydrogen, R^{1B} is optionally substituted C₁₋₆ alkyl or optionally substituted carbocyclyl and at least one instance of R² is optionally substituted C₁₋₆ alkyl.

The present invention further relates to the compound as defined above or a pharmaceutically acceptable salt, solvate, hydrate, polymorph, co-crystal, tautomer, stereoisomer, or isotopically labeled derivative thereof, wherein
R¹ is hydrogen, R^{1B} is optionally substituted C₁₋₆ alkyl or optionally substituted carbocyclyl and at least one instance of R² is unsubstituted methyl; or
R¹ is hydrogen, R^{1B} is optionally substituted C₁₋₆ alkyl or optionally substituted carbocyclyl and both instances of R² are unsubstituted methyl

The present invention in particular relates to a compound which is 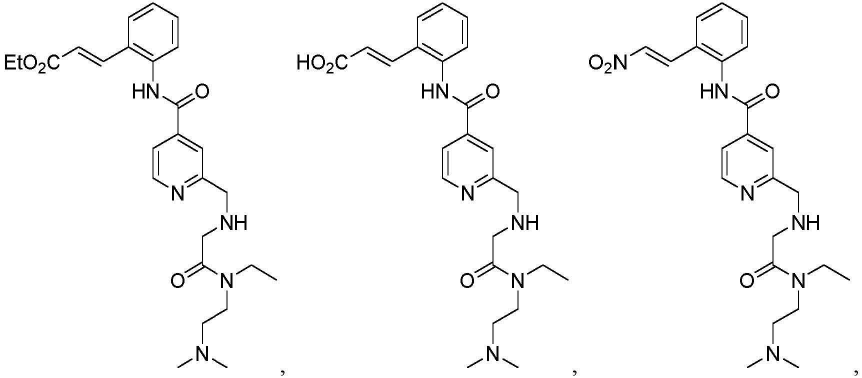 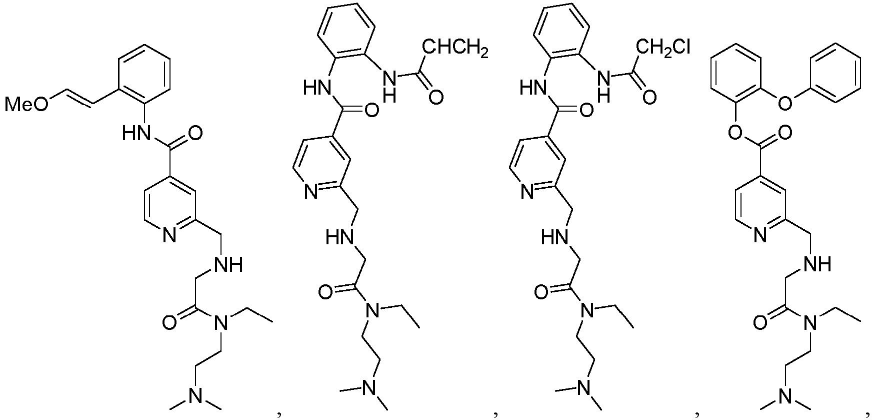 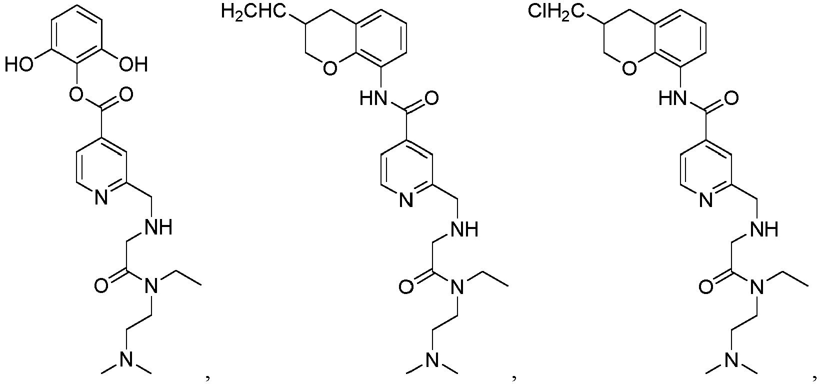 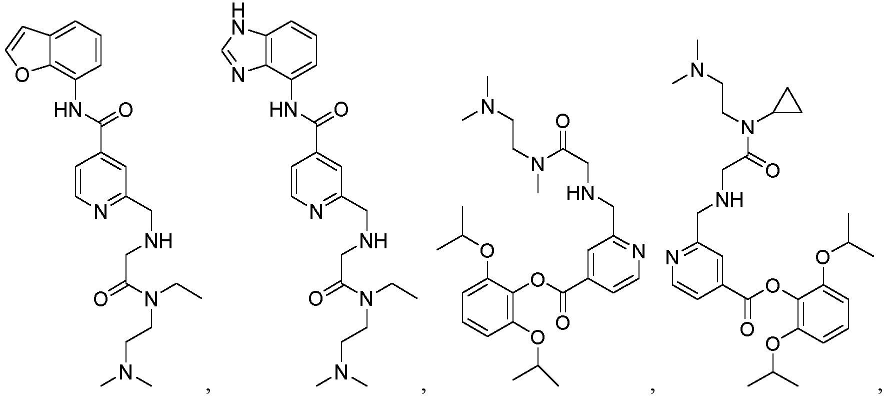 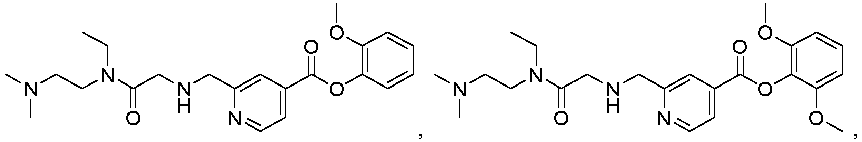 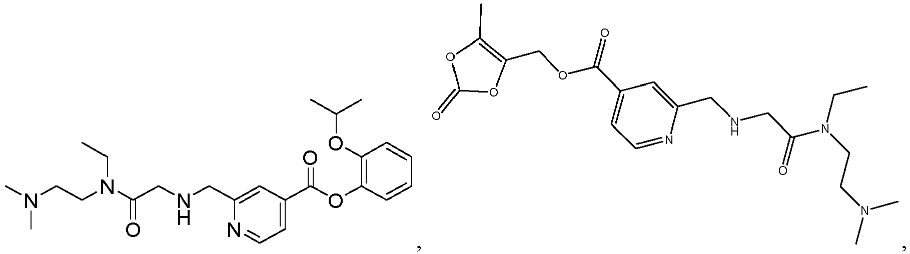 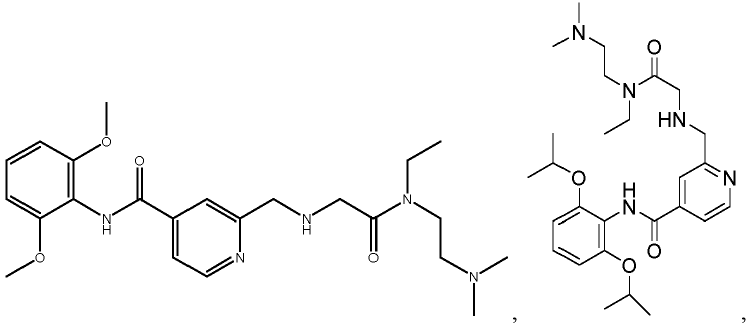 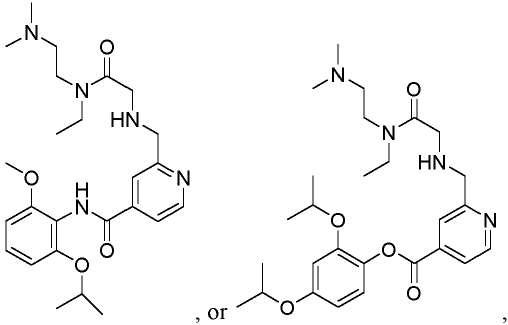 or a pharmaceutically acceptable salt, solvate, hydrate, polymorph, co-crystal, tautomer, stereoisomer, or isotopically labeled derivative thereof.

The present invention further relates to the compound as defined above, which is: 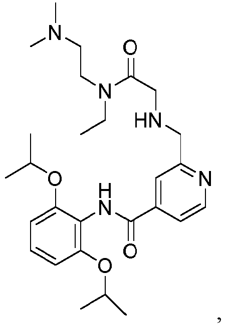 or a pharmaceutically acceptable salt thereof.

The present invention further relates to the compound as defined above, which is 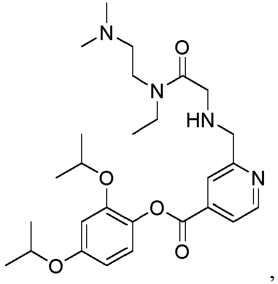 or a pharmaceutically acceptable salt thereof

In another aspect, the present invention relates to compounds of Formula **(II):** 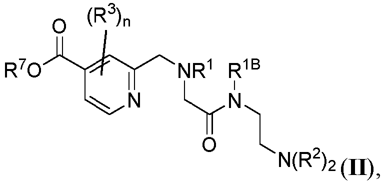 and pharmaceutically acceptable salts, solvates, hydrates, polymorphs, co-crystals, tautomers, stereoisomers, isotopically labeled derivatives, thereof, wherein
R¹ is hydrogen, optionally substituted alkyl, or a nitrogen protecting group;
R^{1B} is hydrogen, methyl , optionally substituted carbocyclyl, or a nitrogen protecting group;
each instance of R² is independently optionally substituted alkyl or a nitrogen protecting group;
each instance of R³ is independently halogen, optionally substituted acyl, optionally substituted alkyl, optionally substituted alkenyl, optionally substituted alkynyl, optionally substituted carbocyclyl, optionally substituted heterocyclyl, optionally substituted aryl, optionally substituted heteroaryl, -CN, -SCN, -NO₂, -N₃, -OR^{A}, -N(R^{B})₂, or -SR^{A};
each instance of R^{A} is independently hydrogen, optionally substituted acyl, optionally substituted alkyl, optionally substituted alkenyl, optionally substituted alkynyl, optionally substituted carbocyclyl, optionally substituted heterocyclyl, optionally substituted aryl, optionally substituted heteroaryl;
each instance of R^{B} is independently hydrogen, optionally substituted acyl, optionally substituted alkyl, optionally substituted alkenyl, optionally substituted alkynyl, optionally substituted carbocyclyl, optionally substituted heterocyclyl, optionally substituted aryl, optionally substituted heteroaryl, or a nitrogen protecting group; or optionally two instances of R^{B} are taken together with their intervening atoms to form a substituted or unsubstituted heterocyclic or substituted or unsubstituted heteroaryl ring;
n is 0, 1, 2, or 3; and
R⁷ is ethyl or propyl;
wherein the nitrogen protecting group is Bn, BOC, Cbz, Fmoc, trifluoroacetyl, triphenylmethyl, acetyl, or Ts;
provided that the compound is not of the formula:

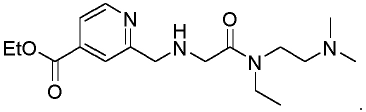

The present invention further relates to the compound as defined above, wherein the compound is of the formula: 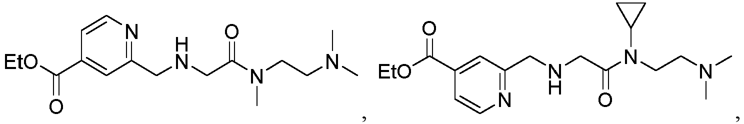 or a pharmaceutically acceptable salt, solvate, hydrate, polymorph, co-crystal, tautomer, stereoisomer, isotopically labeled derivative thereof.

In another aspect, the present invention provides a pharmaceutical composition comprising a compound described herein, or a pharmaceutically acceptable salt, solvate, hydrate, polymorph, co-crystal, tautomer, stereoisomer, or isotopically labeled derivative, and optionally a pharmaceutically acceptable excipient.

The pharmaceutical composition according to the invention is for use in treating a proliferative disease in which KDM5 plays a role, wherein a therapeutically effective amount of said compound or pharmaceutical composition is to be administered to a subject.

In certain embodiments, the pharmaceutical composition according to the invention is for use in treating a proliferative disease, wherein the proliferative disease is a cancer selected from a carcinoma, a sarcoma, lung cancer, breast cancer, liver cancer, pancreatic cancer, gastric cancer, ovarian cancer, colon cancer, leukemia, and multiple myeloma, wherein the lung cancer is non-small cell lung cancer, and the sarcoma is Ewing sarcoma.

The details of one or more embodiments of the present disclosure are set forth herein. Other features, objects, and advantages of the present disclosure will be apparent from the Detailed Description, Examples, Figures, and Claims.

### DEFINITIONS

Definitions of specific functional groups and chemical terms are described in more detail below. The chemical elements are identified in accordance with the Periodic Table of the Elements, CAS version, Handbook of Chemistry and Physics, 75th Ed., inside cover, and specific functional groups are generally defined as described therein. Additionally, general principles of organic chemistry, as well as specific functional moieties and reactivity, are described in Thomas Sorrell, Organic Chemistry, University Science Books, Sausalito, 1999; Smith and March, March 's Advanced Organic Chemistry, 5th Edition, John Wiley & Sons, Inc., New York, 2001; Larock, Comprehensive Organic Transformations, VCH Publishers, Inc., New York, 1989; and Carruthers, Some Modern Methods of Organic Synthesis, 3rd Edition, Cambridge University Press, Cambridge, 1987. The disclosure is not intended to be limited in any manner by the exemplary listing of substituents described herein.

Compounds described herein can comprise one or more asymmetric centers, and thus can exist in various isomeric forms, e.g., enantiomers and/or diastereomers. For example, the compounds described herein can be in the form of an individual enantiomer, diastereomer, or geometric isomer, or can be in the form of a mixture of stereoisomers, including racemic mixtures and mixtures enriched in one or more stereoisomer. Isomers can be isolated from mixtures by methods known to those skilled in the art, including chiral high pressure/performance liquid chromatography ("HPLC") and the formation and crystallization of chiral salts; or isomers can be prepared by asymmetric syntheses. See, for example, Jacques et al., Enantiomers, Racemates and Resolutions (Wiley Interscience, New York, 1981); Wilen et al., Tetrahedron 33:2725 (1977); Eliel, Stereochemistry of Carbon Compounds (McGraw-Hill, NY, 1962); and Wilen, Tables of Resolving Agents and Optical Resolutions p. 268 (E.L. Eliel, Ed., Univ. of Notre Dame Press, Notre Dame, IN 1972). The present disclosure additionally encompasses compounds described herein as individual isomers substantially free of other isomers, and alternatively, as mixtures of various isomers.

When a range of values is listed, it is intended to encompass each value and sub-range within the range. For example, "C₁₋₆" is intended to encompass C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅, and C₅₋₆.

"Hydrocarbon chain" refers to a substituted or unsubstituted divalent alkyl, alkenyl, or alkynyl group. A hydrocarbon chain includes at least one chain, each node ("carbon unit") of which including at least one carbon atom, between the two radicals of the hydrocarbon chain. For example, hydrocarbon chain -C^{A}H(C^{B}H₂C^{C}H₃)- includes only one carbon unit C^{A}. The term "Cₓ hydrocarbon chain," wherein x is a positive integer, refers to a hydrocarbon chain that includes x number of carbon unit(s) between the two radicals of the hydrocarbon chain. If there is more than one possible value of x, the smallest possible value of x is used for the definition of the hydrocarbon chain. By way of non-limiting example, -CH(C₂H₅)- is a C₁ hydrocarbon chain, and 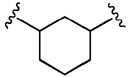 is a C₃ hydrocarbon chain. When a range of values is used, *e.g.,* a C₁₋₆ hydrocarbon chain, the meaning of the range is as described herein. A hydrocarbon chain may be saturated (*e.g*., -(CH₂)₄-). A hydrocarbon chain may also be unsaturated and include one or more C=C and/or C≡C bonds anywhere in the hydrocarbon chain. For instance, -CH=CH-(CH₂)₂-, - CH₂-C≡C-CH₂-, and -C≡C-CH=CH- are all non-limiting examples of a unsubstituted and unsaturated hydrocarbon chain. In certain embodiments, the hydrocarbon chain is unsubstituted (*e.g.,* -(CH₂)₄-). In certain embodiments, the hydrocarbon chain is substituted (*e.g.,* -CH(C₂H₅)- and -CF₂-). Any two substituents on the hydrocarbon chain may be joined to form an optionally substituted carbocyclyl, optionally substituted heterocyclyl, optionally substituted aryl, or optionally substituted heteroaryl ring. For instance, 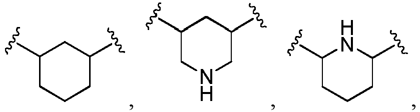 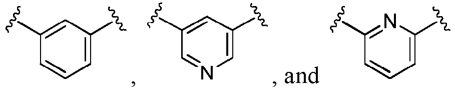 are all non-limiting examples of a hydrocarbon chain. In contrast, in certain embodiments 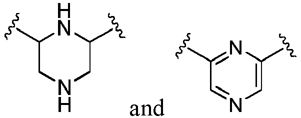 are not within the scope of the hydrocarbon chains described herein.

"Alkyl" refers to a radical of a straight-chain or branched saturated hydrocarbon group having from 1 to 20 carbon atoms ("C₁₋₂₀ alkyl"). In some embodiments, an alkyl group has 1 to 10 carbon atoms ("C₁₋₁₀ alkyl"). In some embodiments, an alkyl group has 1 to 9 carbon atoms ("C₁₋₉ alkyl"). In some embodiments, an alkyl group has 1 to 8 carbon atoms ("C₁₋₈ alkyl"). In some embodiments, an alkyl group has 1 to 7 carbon atoms ("C₁₋₇ alkyl"). In some embodiments, an alkyl group has 1 to 6 carbon atoms ("C₁₋₆ alkyl"). In some embodiments, an alkyl group has 1 to 5 carbon atoms ("C₁₋₅ alkyl"). In some embodiments, an alkyl group has 1 to 4 carbon atoms ("C₁₋₄ alkyl"). In some embodiments, an alkyl group has 1 to 3 carbon atoms ("C₁₋₃ alkyl"). In some embodiments, an alkyl group has 1 to 2 carbon atoms ("C₁₋₂ alkyl"). In some embodiments, an alkyl group has 1 carbon atom ("C₁ alkyl"). In some embodiments, an alkyl group has 2 to 6 carbon atoms ("C₂₋₆ alkyl"). Examples of C₁₋₆ alkyl groups include, but are not limited to, methyl (C₁), ethyl (C₂), *n*-propyl (C₃), isopropyl (C₃), *n*-butyl (C₄), *tert-butyl* (C₄), *sec-*butyl (C₄), *iso-*butyl (C₄), *n*-pentyl (C₅), 3-pentanyl (C₅), amyl (C₅), neopentyl (C₅), 3-methyl-2-butanyl (C₅), tertiary amyl (C₅), and *n*-hexyl (C₆). Additional non-limiting examples of alkyl groups include *n*-heptyl (C₇), *n*-octyl (C₈) and the like. Unless otherwise specified, each instance of an alkyl group is independently optionally substituted, *i.e.,* unsubstituted (an "unsubstituted alkyl") or substituted (a "substituted alkyl") with one or more substituents. In certain embodiments, the alkyl group is unsubstituted C₁₋₁₀ alkyl (*e.g.,* -CH₃). In certain embodiments, the alkyl group is substituted C₁₋₁₀ alkyl.

"Alkenyl" refers to a radical of a straight-chain or branched hydrocarbon group having from 2 to 20 carbon atoms, one or more carbon-carbon double bonds, and no triple bonds ("C₂₋₂₀ alkenyl"). In some embodiments, an alkenyl group has 2 to 10 carbon atoms ("C₂₋₁₀ alkenyl"). In some embodiments, an alkenyl group has 2 to 9 carbon atoms ("C₂₋₉ alkenyl"). In some embodiments, an alkenyl group has 2 to 8 carbon atoms ("C₂₋₈ alkenyl"). In some embodiments, an alkenyl group has 2 to 7 carbon atoms ("C₂₋₇ alkenyl"). In some embodiments, an alkenyl group has 2 to 6 carbon atoms ("C₂₋₆ alkenyl"). In some embodiments, an alkenyl group has 2 to 5 carbon atoms ("C₂₋₅ alkenyl"). In some embodiments, an alkenyl group has 2 to 4 carbon atoms ("C₂₋₄ alkenyl"). In some embodiments, an alkenyl group has 2 to 3 carbon atoms ("C₂₋₃ alkenyl"). In some embodiments, an alkenyl group has 2 carbon atoms ("C₂ alkenyl"). The one or more carbon-carbon double bonds can be internal (such as in 2-butenyl) or terminal (such as in 1-butenyl). Examples of C₂₋₄ alkenyl groups include ethenyl (C₂), 1-propenyl (C₃), 2-propenyl (C₃), 1-butenyl (C₄), 2-butenyl (C₄), butadienyl (C₄), and the like. Examples of C₂₋₆ alkenyl groups include the aforementioned C₂₋₄ alkenyl groups as well as pentenyl (C₅), pentadienyl (C₅), hexenyl (C₆), and the like. Additional examples of alkenyl include heptenyl (C₇), octenyl (C₈), octatrienyl (C₈), and the like. Unless otherwise specified, each instance of an alkenyl group is independently optionally substituted, *i.e.,* unsubstituted (an "unsubstituted alkenyl") or substituted (a "substituted alkenyl") with one or more substituents. In certain embodiments, the alkenyl group is unsubstituted C₂₋₁₀ alkenyl. In certain embodiments, the alkenyl group is substituted C₂₋₁₀ alkenyl.

"Alkynyl" refers to a radical of a straight-chain or branched hydrocarbon group having from 2 to 20 carbon atoms, one or more carbon-carbon triple bonds, and optionally one or more double bonds ("C₂₋₂₀ alkynyl"). In some embodiments, an alkynyl group has 2 to 10 carbon atoms ("C₂₋₁₀ alkynyl"). In some embodiments, an alkynyl group has 2 to 9 carbon atoms ("C₂₋₉ alkynyl"). In some embodiments, an alkynyl group has 2 to 8 carbon atoms ("C₂₋₈ alkynyl"). In some embodiments, an alkynyl group has 2 to 7 carbon atoms ("C₂₋₇ alkynyl"). In some embodiments, an alkynyl group has 2 to 6 carbon atoms ("C₂₋₆ alkynyl"). In some embodiments, an alkynyl group has 2 to 5 carbon atoms ("C₂₋₅ alkynyl"). In some embodiments, an alkynyl group has 2 to 4 carbon atoms ("C₂₋₄ alkynyl"). In some embodiments, an alkynyl group has 2 to 3 carbon atoms ("C₂₋₃ alkynyl"). In some embodiments, an alkynyl group has 2 carbon atoms ("C₂ alkynyl"). The one or more carbon-carbon triple bonds can be internal (such as in 2-butynyl) or terminal (such as in 1-butynyl). Examples of C₂₋₄ alkynyl groups include, without limitation, ethynyl (C₂), 1-propynyl (C₃), 2-propynyl (C₃), 1-butynyl (C₄), 2-butynyl (C₄), and the like. Examples of C₂₋₆ alkenyl groups include the aforementioned C₂₋₄ alkynyl groups as well as pentynyl (C₅), hexynyl (C₆), and the like. Additional examples of alkynyl include heptynyl (C₇), octynyl (C₈), and the like. Unless otherwise specified, each instance of an alkynyl group is independently optionally substituted, *i.e.,* unsubstituted (an "unsubstituted alkynyl") or substituted (a "substituted alkynyl") with one or more substituents. In certain embodiments, the alkynyl group is unsubstituted C₂₋₁₀ alkynyl. In certain embodiments, the alkynyl group is substituted C₂₋₁₀ alkynyl.

"Carbocyclyl" or "carbocyclic" refers to a radical of a non-aromatic cyclic hydrocarbon group having from 3 to 10 ring carbon atoms ("C₃₋₁₀ carbocyclyl") in the non-aromatic ring system. In some embodiments, a carbocyclyl group has 3 to 8 ring carbon atoms ("C₃₋₈ carbocyclyl"). In some embodiments, a carbocyclyl group has 3 to 6 ring carbon atoms ("C₃₋₆ carbocyclyl"). In some embodiments, a carbocyclyl group has 3 to 6 ring carbon atoms ("C₃₋₆ carbocyclyl"). In some embodiments, a carbocyclyl group has 5 to 10 ring carbon atoms ("C₅₋₁₀ carbocyclyl"). Exemplary C₃₋₆ carbocyclyl groups include, without limitation, cyclopropyl (C₃), cyclopropenyl (C₃), cyclobutyl (C₄), cyclobutenyl (C₄), cyclopentyl (C₅), cyclopentenyl (C₅), cyclohexyl (C₆), cyclohexenyl (C₆), cyclohexadienyl (C₆), and the like. Exemplary C₃₋₈ carbocyclyl groups include, without limitation, the aforementioned C₃₋₆ carbocyclyl groups as well as cycloheptyl (C₇), cycloheptenyl (C₇), cycloheptadienyl (C₇), cycloheptatrienyl (C₇), cyclooctyl (C₈), cyclooctenyl (C₈), bicyclo[2.2.1]heptanyl (C₇), bicyclo[2.2.2]octanyl (C₈), and the like. Exemplary C₃₋₁₀ carbocyclyl groups include, without limitation, the aforementioned C₃₋₈ carbocyclyl groups as well as cyclononyl (C₉), cyclononenyl (C₉), cyclodecyl (C₁₀), cyclodecenyl (C₁₀), octahydro-1*H*-indenyl (C₉), decahydronaphthalenyl (C₁₀), spiro[4.5]decanyl (C₁₀), and the like. As the foregoing examples illustrate, in certain embodiments, the carbocyclyl group is either monocyclic ("monocyclic carbocyclyl") or contain a fused, bridged or spiro ring system such as a bicyclic system ("bicyclic carbocyclyl") and can be saturated or can be partially unsaturated. "Carbocyclyl" also includes ring systems wherein the carbocyclic ring, as defined above, is fused with one or more aryl or heteroaryl groups wherein the point of attachment is on the carbocyclic ring, and in such instances, the number of carbons continue to designate the number of carbons in the carbocyclic ring system. Unless otherwise specified, each instance of a carbocyclyl group is independently optionally substituted, *i.e.,* unsubstituted (an "unsubstituted carbocyclyl") or substituted (a "substituted carbocyclyl") with one or more substituents. In certain embodiments, the carbocyclyl group is unsubstituted C₃₋₁₀ carbocyclyl. In certain embodiments, the carbocyclyl group is a substituted C₃₋₁₀ carbocyclyl.

In some embodiments, "carbocyclyl" is a monocyclic, saturated carbocyclyl group having from 3 to 10 ring carbon atoms ("C₃₋₁₀ cycloalkyl"). In some embodiments, a cycloalkyl group has 3 to 8 ring carbon atoms ("C₃₋₈ cycloalkyl"). In some embodiments, a cycloalkyl group has 3 to 6 ring carbon atoms ("C₃₋₆ cycloalkyl"). In some embodiments, a cycloalkyl group has 5 to 6 ring carbon atoms ("C₅₋₆ cycloalkyl"). In some embodiments, a cycloalkyl group has 5 to 10 ring carbon atoms ("C₅₋₁₀ cycloalkyl"). Non-limiting examples of C₅₋₆ cycloalkyl groups include cyclopentyl (C₅) and cyclohexyl (C₆). Non-limiting examples of C₃₋₆ cycloalkyl groups include the aforementioned C₅₋₆ cycloalkyl groups as well as cyclopropyl (C₃) and cyclobutyl (C₄). Non-limiting examples of C₃₋₈ cycloalkyl groups include the aforementioned C₃₋₆ cycloalkyl groups as well as cycloheptyl (C₇) and cyclooctyl (C₈). Unless otherwise specified, each instance of a cycloalkyl group is independently unsubstituted (an "unsubstituted cycloalkyl") or substituted (a "substituted cycloalkyl") with one or more substituents. In certain embodiments, the cycloalkyl group is unsubstituted C₃₋₁₀ cycloalkyl. In certain embodiments, the cycloalkyl group is substituted C₃₋₁₀ cycloalkyl.

"Heterocyclyl" or "heterocyclic" refers to a radical of a 3- to 10-membered non-aromatic ring system having ring carbon atoms and 1 to 4 ring heteroatoms, wherein each heteroatom is independently selected from the group consisting of nitrogen, oxygen, sulfur, boron, phosphorus, and silicon (*i.e.,* a "3-10 membered heterocyclyl"). In heterocyclyl groups that contain one or more nitrogen atoms, the point of attachment can be a carbon or nitrogen atom, as valency permits. A heterocyclyl group can either be monocyclic ("monocyclic heterocyclyl") or a fused, bridged or spiro ring system such as a bicyclic system ("bicyclic heterocyclyl"), and can be saturated or can be partially unsaturated. Heterocyclyl bicyclic ring systems can include one or more heteroatoms in one or both rings. "Heterocyclyl" also includes ring systems wherein the heterocyclic ring, as defined above, is fused with one or more carbocyclyl groups wherein the point of attachment is either on the carbocyclyl or heterocyclic ring, or ring systems wherein the heterocyclic ring, as defined above, is fused with one or more aryl or heteroaryl groups, wherein the point of attachment is on the heterocyclic ring, and in such instances, the number of ring members continue to designate the number of ring members in the heterocyclic ring system. Unless otherwise specified, each instance of heterocyclyl is independently optionally substituted, *i.e.,* unsubstituted (an "unsubstituted heterocyclyl") or substituted (a "substituted heterocyclyl") with one or more substituents. In certain embodiments, the heterocyclyl group is unsubstituted 3-10 membered heterocyclyl. In certain embodiments, the heterocyclyl group is substituted 3-10 membered heterocyclyl.

In some embodiments, a heterocyclyl group is a 5-10 membered non-aromatic ring system having ring carbon atoms and 1-4 ring heteroatoms, wherein each heteroatom is independently selected from the group consisting of nitrogen, oxygen, sulfur, boron, phosphorus, and silicon ("5-10 membered heterocyclyl"). In some embodiments, a heterocyclyl group is a 5-8 membered non-aromatic ring system having ring carbon atoms and 1-4 ring heteroatoms, wherein each heteroatom is independently selected from the group consisting of nitrogen, oxygen, boron, silicon, phosphorous, and sulfur ("5-8 membered heterocyclyl"). In some embodiments, a heterocyclyl group is a 5-6 membered non-aromatic ring system having ring carbon atoms and 1-4 ring heteroatoms, wherein each heteroatom is independently selected from the group consisting of nitrogen, oxygen, boron, silicon, phosphorous, and sulfur ("5-6 membered heterocyclyl"). In some embodiments, the 5-6 membered heterocyclyl has 1-3 ring heteroatoms selected from nitrogen, oxygen, and sulfur. In some embodiments, the 5-6 membered heterocyclyl has 1-2 ring heteroatoms selected from nitrogen, oxygen, and sulfur. In some embodiments, the 5-6 membered heterocyclyl has one ring heteroatom selected from nitrogen, oxygen, and sulfur.

Exemplary 3-membered heterocyclyl groups containing one heteroatom include, without limitation, aziridinyl, oxiranyl, and thiiranyl. Exemplary 4-membered heterocyclyl groups containing one heteroatom include, without limitation, azetidinyl, oxetanyl and thietanyl. Exemplary 5-membered heterocyclyl groups containing one heteroatom include, without limitation, tetrahydrofuranyl, dihydrofuranyl, tetrahydrothiophenyl, dihydrothiophenyl, pyrrolidinyl, dihydropyrrolyl, and pyrrolyl-2,5-dione. Exemplary 5-membered heterocyclyl groups containing two heteroatoms include, without limitation, dioxolanyl, oxasulfuranyl, disulfuranyl, and oxazolidin-2-one. Exemplary 5-membered heterocyclyl groups containing three heteroatoms include, without limitation, triazolinyl, oxadiazolinyl, and thiadiazolinyl. Exemplary 6-membered heterocyclyl groups containing one heteroatom include, without limitation, piperidinyl, tetrahydropyranyl, dihydropyridinyl, and thianyl. Exemplary 6-membered heterocyclyl groups containing two heteroatoms include, without limitation, piperazinyl, morpholinyl, dithianyl, and dioxanyl. Exemplary 6-membered heterocyclyl groups containing two heteroatoms include, without limitation, triazinanyl. Exemplary 7-membered heterocyclyl groups containing one heteroatom include, without limitation, azepanyl, oxepanyl and thiepanyl. Exemplary 8-membered heterocyclyl groups containing one heteroatom include, without limitation, azocanyl, oxecanyl and thiocanyl. Exemplary 5-membered heterocyclyl groups fused to a C₆ aryl ring (also referred to herein as a 5,6-bicyclic heterocyclic ring) include, without limitation, indolinyl, isoindolinyl, dihydrobenzofuranyl, dihydrobenzothienyl, benzoxazolinonyl, and the like. Exemplary 6-membered heterocyclyl groups fused to an aryl ring (also referred to herein as a 6,6-bicyclic heterocyclic ring) include, without limitation, tetrahydroquinolinyl, tetrahydroisoquinolinyl, and the like.

"Aryl" refers to a radical of a monocyclic or polycyclic (*e.g*., bicyclic or tricyclic) 4n+2 aromatic ring system (*e.g*., having 6, 10, or 14 pi electrons shared in a cyclic array) having 6-14 ring carbon atoms and zero heteroatoms provided in the aromatic ring system ("C₆₋₁₄ aryl"). In some embodiments, an aryl group has six ring carbon atoms ("C₆ aryl"; *e.g*., phenyl). In some embodiments, an aryl group has ten ring carbon atoms ("C₁₀ aryl"; *e.g*., naphthyl such as 1-naphthyl and 2-naphthyl). In some embodiments, an aryl group has fourteen ring carbon atoms ("C₁₄ aryl"; *e.g*., anthracyl). "Aryl" also includes ring systems wherein the aryl ring, as defined above, is fused with one or more carbocyclyl or heterocyclyl groups wherein the radical or point of attachment is on the aryl ring, and in such instances, the number of carbon atoms continue to designate the number of carbon atoms in the aryl ring system. Unless otherwise specified, each instance of an aryl group is independently optionally substituted, *i.e.,* unsubstituted (an "unsubstituted aryl") or substituted (a "substituted aryl") with one or more substituents. In certain embodiments, the aryl group is unsubstituted C₆₋₁₄ aryl. In certain embodiments, the aryl group is substituted C₆₋₁₄ aryl.

"Aralkyl" is a subset of alkyl and aryl and refers to an optionally substituted alkyl group substituted by an optionally substituted aryl group. In certain embodiments, the aralkyl is optionally substituted benzyl. In certain embodiments, the aralkyl is benzyl. In certain embodiments, the aralkyl is optionally substituted phenethyl. In certain embodiments, the aralkyl is phenethyl.

"Heteroaryl" refers to a radical of a 5-10 membered monocyclic or bicyclic 4n+2 aromatic ring system (*e.g*., having 6 or 10 pi electrons shared in a cyclic array) having ring carbon atoms and 1-4 ring heteroatoms provided in the aromatic ring system, wherein each heteroatom is independently selected from the group consisting of nitrogen, oxygen and sulfur ("5-10 membered heteroaryl"). In heteroaryl groups that contain one or more nitrogen atoms, the point of attachment can be a carbon or nitrogen atom, as valency permits. Heteroaryl bicyclic ring systems can include one or more heteroatoms in one or both rings. "Heteroaryl" includes ring systems wherein the heteroaryl ring, as defined above, is fused with one or more carbocyclyl or heterocyclyl groups wherein the point of attachment is on the heteroaryl ring, and in such instances, the number of ring members continue to designate the number of ring members in the heteroaryl ring system. "Heteroaryl" also includes ring systems wherein the heteroaryl ring, as defined above, is fused with one or more aryl groups wherein the point of attachment is either on the aryl or heteroaryl ring, and in such instances, the number of ring members designates the number of ring members in the fused (aryl/heteroaryl) ring system. Bicyclic heteroaryl groups wherein one ring does not contain a heteroatom (*e.g*., indolyl, quinolinyl, carbazolyl, and the like) the point of attachment can be on either ring, *i.e.,* either the ring bearing a heteroatom (*e.g.,* 2-indolyl) or the ring that does not contain a heteroatom (*e.g*., 5-indolyl).

**In** some embodiments, a heteroaryl group is a 5-10 membered aromatic ring system having ring carbon atoms and 1-4 ring heteroatoms provided in the aromatic ring system, wherein each heteroatom is independently selected from the group consisting of nitrogen, oxygen, and sulfur. In some embodiments, a heteroaryl group is a 5-8 membered aromatic ring system having ring carbon atoms and 1-4 ring heteroatoms provided in the aromatic ring system, wherein each heteroatom is independently selected from the group consisting of nitrogen, oxygen, and sulfur ("5-8 membered heteroaryl"). In some embodiments, a heteroaryl group is a 5-6 membered aromatic ring system having ring carbon atoms and 1-4 ring heteroatoms provided in the aromatic ring system, wherein each heteroatom is independently selected from the group consisting of nitrogen, oxygen, and sulfur ("5-6 membered heteroaryl"). In some embodiments, the 5-6 membered heteroaryl has 1-3 ring heteroatoms selected from nitrogen, oxygen, and sulfur. In some embodiments, the 5-6 membered heteroaryl has 1-2 ring heteroatoms selected from nitrogen, oxygen, and sulfur. In some embodiments, the 5-6 membered heteroaryl has 1 ring heteroatom selected from nitrogen, oxygen, and sulfur. Unless otherwise specified, each instance of a heteroaryl group is independently optionally substituted, *i.e.,* unsubstituted (an "unsubstituted heteroaryl") or substituted (a "substituted heteroaryl") with one or more substituents. In certain embodiments, the heteroaryl group is unsubstituted 5-14 membered heteroaryl. In certain embodiments, the heteroaryl group is substituted 5-14 membered heteroaryl.

Exemplary 5-membered heteroaryl groups containing one heteroatom include, without limitation, pyrrolyl, furanyl and thiophenyl. Exemplary 5-membered heteroaryl groups containing two heteroatoms include, without limitation, imidazolyl, pyrazolyl, oxazolyl, isoxazolyl, thiazolyl, and isothiazolyl. Exemplary 5-membered heteroaryl groups containing three heteroatoms include, without limitation, triazolyl, oxadiazolyl, and thiadiazolyl. Exemplary 5-membered heteroaryl groups containing four heteroatoms include, without limitation, tetrazolyl. Exemplary 6-membered heteroaryl groups containing one heteroatom include, without limitation, pyridinyl. Exemplary 6-membered heteroaryl groups containing two heteroatoms include, without limitation, pyridazinyl, pyrimidinyl, and pyrazinyl. Exemplary 6-membered heteroaryl groups containing three or four heteroatoms include, without limitation, triazinyl and tetrazinyl, respectively. Exemplary 7-membered heteroaryl groups containing one heteroatom include, without limitation, azepinyl, oxepinyl, and thiepinyl. Exemplary 5,6-bicyclic heteroaryl groups include, without limitation, indolyl, isoindolyl, indazolyl, benzotriazolyl, benzothiophenyl, isobenzothiophenyl, benzofuranyl, benzoisofuranyl, benzimidazolyl, benzoxazolyl, benzisoxazolyl, benzoxadiazolyl, benzthiazolyl, benzisothiazolyl, benzthiadiazolyl, indolizinyl, and purinyl. Exemplary 6,6-bicyclic heteroaryl groups include, without limitation, naphthyridinyl, pteridinyl, quinolinyl, isoquinolinyl, cinnolinyl, quinoxalinyl, phthalazinyl, and quinazolinyl.

"Heteroaralkyl" is a subset of alkyl and heteroaryl and refers to an optionally substituted alkyl group substituted by an optionally substituted heteroaryl group.

"Partially unsaturated" refers to a group that includes at least one double or triple bond. A "partially unsaturated" ring system is further intended to encompass rings having multiple sites of unsaturation but is not intended to include aromatic groups (*e.g*., aryl or heteroaryl groups) as defined herein. Likewise, "saturated" refers to a group that does not contain a double or triple bond, *i.e.,* contains all single bonds.

Alkyl, alkenyl, alkynyl, carbocyclyl, heterocyclyl, aryl, and heteroaryl groups, which are divalent bridging groups are optionally further referred to using the suffix -ene, *e.g*., alkylene, alkenylene, alkynylene, carbocyclylene, heterocyclylene, arylene, and heteroarylene.

The term "optionally substituted" refers to substituted with one or more optional substituents or unsubstituted.

Alkyl, alkenyl, alkynyl, carbocyclyl, heterocyclyl, aryl, and heteroaryl groups are optionally substituted (*e.g*., "substituted" or "unsubstituted" alkyl, "substituted" or "unsubstituted" alkenyl, "substituted" or "unsubstituted" alkynyl, "substituted" or "unsubstituted" carbocyclyl, "substituted" or "unsubstituted" heterocyclyl, "substituted" or "unsubstituted" aryl or "substituted" or "unsubstituted" heteroaryl group). In general, the term "substituted", whether preceded by the term "optionally" or not, means that at least one hydrogen present on a group (*e.g.,* a carbon or nitrogen atom) is replaced with a permissible substituent, *e.g.,* a substituent which upon substitution results in a stable compound, *e.g.,* a compound which does not spontaneously undergo transformation such as by rearrangement, cyclization, elimination, or other reaction. Unless otherwise indicated, a "substituted" group has a substituent at one or more substitutable positions of the group, and when more than one position in any given structure is substituted, the substituent is either the same or different at each position. The term "substituted" is contemplated to include substitution with all permissible substituents of organic compounds, any of the substituents described herein that results in the formation of a stable compound. The present disclosure contemplates any and all such combinations in order to arrive at a stable compound. For purposes of this disclosure, heteroatoms such as nitrogen may have hydrogen substituents and/or any suitable substituent as described herein which satisfy the valencies of the heteroatoms and results in the formation of a stable moiety.

Exemplary carbon atom substituents include, but are not limited to, halogen, -CN, -NO₂, -N₃, -SO₂H, -SO₃H, -OH, -OR^{aa}, -ON(R^{bb})₂, -N(R^{bb})₂, -N(R^{bb})₃⁺X⁻, -N(OR^{cc})R^{bb}, -SH, -SR^{aa}, -SSR^{cc}, -C(=O)R^{aa}, -CO₂H, -CHO, -C(OR^{cc})₂, -CO₂R^{aa}, -OC(=O)R^{aa}, -OCO₂R^{aa}, -C(=O)N(R^{bb})₂, -OC(=O)N(R^{bb})₂, -NR^{bb}C(=O)R^{aa}, -NR^{bb}CO₂R^{aa}, -NR^{bb}C(=O)N(R^{bb})₂, -C(=NR^{bb})R^{aa}, -C(=NR^{bb})OR^{aa}, -OC(=NR^{bb})R^{aa}, -OC(=NR^{bb})OR^{aa}, -C(=NR^{bb})N(R^{bb})₂, -OC(=NR^{bb})N(R^{bb})₂, -NR^{bb}C(=NR^{bb})N(R^{bb})₂, -C(=O)NR^{bb}SO₂R^{aa}, -NR^{bb}SO₂R^{aa}, -SO₂N(R^{bb})₂, -SO₂R^{aa}, -SO₂OR^{aa}, -OSO₂R^{aa}, -S(=O)R^{aa}, -OS(=O)R^{aa}, -Si(R^{aa})₃, -OSi(R^{aa})₃ -C(=S)N(R^{bb})₂, -C(=O)SR^{aa}, -C(=S)SR^{aa}, -SC(=S)SR^{aa}, -SC(=O)SR^{aa}, -OC(=O)SR^{aa}, -SC(=O)OR^{aa}, -SC(=O)R^{aa}, -P(=O)(R^{aa})₂, -P(=O)(OR^{cc})₂, -OP(=O)(R^{aa})₂, -OP(=O)(OR^{cc})₂, -P(=O)(N(R^{bb})₂)₂, -OP(=O)(N(R^{bb})₂)₂, -NR^{bb}P(=O)(R^{aa})₂, -NR^{bb}P(=O)(OR^{cc})₂, -NR^{bb}P(=O)(N(R^{bb})₂)₂, -P(R^{cc})₂, -P(OR^{cc})₂, -P(R^{cc})₃⁺X⁻, -P(OR^{cc})₃⁺X⁻, -P(R^{cc})₄, -P(OR^{cc})₄, -OP(R^{cc})₂, -OP(R^{cc})₃⁺X⁻, -OP(OR^{cc})₂, -OP(OR^{cc})₃⁺X⁻, -OP(R^{cc})₄, -OP(OR^{cc})₄, -B(R^{aa})₂, -B(OR^{cc})₂, -BR^{aa}(OR^{cc}), C₁₋₁₀ alkyl, C₁₋₁₀ perhaloalkyl, C₂₋₁₀ alkenyl, C₂₋₁₀ alkynyl, heteroC₁₋₁₀ alkyl, heteroC₂₋₁₀ alkenyl, heteroC₂₋₁₀ alkynyl, C₃₋₁₀ carbocyclyl, 3-14 membered heterocyclyl, C₆₋₁₄ aryl, and 5-14 membered heteroaryl, wherein each alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{dd} groups; wherein X⁻ is a counterion;
or two geminal hydrogens on a carbon atom are replaced with the group =O, =S, =NN(R^{bb})₂, =NNR^{bb}C(=O)R^{aa}, =NNR^{bb}C(=O)OR^{aa}, =NNR^{bb}S(=O)₂R^{aa}, =NR^{bb}, or =NOR^{cc};
each instance of R^{aa} is, independently, selected from C₁₋₁₀ alkyl, C₁₋₁₀ perhaloalkyl, C₂₋₁₀ alkenyl, C₂₋₁₀ alkynyl, heteroC₁₋₁₀ alkyl, heteroC₂₋₁₀alkenyl, heteroC₂₋₁₀alkynyl, C₃₋₁₀ carbocyclyl, 3-14 membered heterocyclyl, C₆₋₁₄ aryl, and 5-14 membered heteroaryl, or two R^{aa} groups are joined to form a 3-14 membered heterocyclyl or 5-14 membered heteroaryl ring, wherein each alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{dd} groups;
each instance of R^{bb} is, independently, selected from hydrogen, -OH, -OR^{aa}, -N(R^{cc})₂, -CN, -C(=O)R^{aa}, -C(=O)N(R^{cc})₂, -CO₂R^{aa}, -SO₂R^{aa}, -C(=NR^{cc})OR^{aa}, -C(=NR^{cc})N(R^{cc})₂, -SO₂N(R^{cc})₂, -SO₂R^{cc}, -SO₂OR^{cc}, -SOR^{aa}, -C(=S)N(R^{cc})₂, -C(=O)SR^{cc}, -C(=S)SR^{cc}, -P(=O)(R^{aa})₂, -P(=O)(OR^{cc})₂, -P(=O)(N(R^{cc})₂)₂, C₁₋₁₀ alkyl, C₁₋₁₀ perhaloalkyl, C₂₋₁₀ alkenyl, C₂₋₁₀ alkynyl, heteroC₁₋₁₀alkyl, heteroC₂₋₁₀alkenyl, heteroC₂₋₁₀alkynyl, C₃₋₁₀ carbocyclyl, 3-14 membered heterocyclyl, C₆₋₁₄ aryl, and 5-14 membered heteroaryl, or two R^{bb} groups are joined to form a 3-14 membered heterocyclyl or 5-14 membered heteroaryl ring, wherein each alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{dd} groups; wherein X⁻ is a counterion;
each instance of R^{cc} is, independently, selected from hydrogen, C₁₋₁₀ alkyl, C₁₋₁₀ perhaloalkyl, C₂₋₁₀ alkenyl, C₂₋₁₀ alkynyl, heteroC₁₋₁₀ alkyl, heteroC₂₋₁₀ alkenyl, heteroC₂₋₁₀ alkynyl, C₃₋₁₀ carbocyclyl, 3-14 membered heterocyclyl, C₆₋₁₄ aryl, and 5-14 membered heteroaryl, or two R^{cc} groups are joined to form a 3-14 membered heterocyclyl or 5-14 membered heteroaryl ring, wherein each alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{dd} groups;
each instance of R^{dd} is, independently, selected from halogen, -CN, -NO₂, -N₃, -SO₂H, -SO₃H, -OH, -OR^{ee}, -ON(R^{ff})₂, -N(R^{ff})₂, -N(R^{ff})₃⁺X⁻, -N(OR^{ee})R⁷, -SH, -SR^{ee}, -SSR^{ee}, -C(=O)R^{ee}, -CO₂H, -CO₂R^{ee}, -OC(=O)R^{ee}, -OCO₂R^{ee}, -C(=O)N(R^{ff})₂, -OC(=O)N(R^{ff})₂, -NR^{ff}C(=O)R^{ee}, -NR^{ff}CO₂R^{ee}, -NR^{ff}C(=O)N(R^{ff})₂, -C(=NR^{ff})OR^{ee}, -OC(=NR^{ff})R^{ee}, -OC(=NR^{ff})OR^{ee}, -C(=NR^{ff})N(R^{ff})₂, -OC(=NR^{ff})N(R^{ff})₂, -NR^{ff}C(=NR^{ff})N(R^{ff})₂, -NR^{ff}SO₂R^{ee}, -SO₂N(R^{ff})₂, -SO₂R^{ee}, -SO₂OR^{ee}, -OSO₂R^{ee}, -S(=O)R^{ee}, -Si(R^{ee})₃, -OSi(R^{ee})₃, -C(=S)N(R^{ff})₂, -C(=O)SR^{ee}, -C(=S)SR^{ee}, -SC(=S)SR^{ee}, -P(=O)(OR^{ee})₂, -P(=O)(R^{ee})₂, -OP(=O)(R^{ee})₂, -OP(=O)(OR^{ee})₂, C₁₋₆ alkyl, C₁₋₆ perhaloalkyl, C₂₋₆ alkenyl, C₂₋₆ alkynyl, heteroC₁₋₆alkyl, heteroC₂₋₆alkenyl, heteroC₂₋₆alkynyl, C₃₋₁₀ carbocyclyl, 3-10 membered heterocyclyl, C₆₋₁₀ aryl, 5-10 membered heteroaryl, wherein each alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{gg} groups, or two geminal R^{dd} substituents can be joined to form =O or =S; wherein X⁻ is a counterion;
each instance of R^{ee} is, independently, selected from C₁₋₆ alkyl, C₁₋₆ perhaloalkyl, C₂₋₆ alkenyl, C₂₋₆ alkynyl, heteroC₁₋₆ alkyl, heteroC₂₋₆alkenyl, heteroC₂₋₆ alkynyl, C₃₋₁₀ carbocyclyl, C₆₋₁₀ aryl, 3-10 membered heterocyclyl, and 3-10 membered heteroaryl, wherein each alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{gg} groups;
each instance of R^{ff} is, independently, selected from hydrogen, C₁₋₆ alkyl, C₁₋₆ perhaloalkyl, C₂₋₆ alkenyl, C₂₋₆ alkynyl, heteroC₁₋₆alkyl, heteroC₂₋₆alkenyl, heteroC₂₋₆alkynyl, C₃₋₁₀ carbocyclyl, 3-10 membered heterocyclyl, C₆₋₁₀ aryl and 5-10 membered heteroaryl, or two R^{ff} groups are joined to form a 3-10 membered heterocyclyl or 5-10 membered heteroaryl ring, wherein each alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{gg} groups; and
each instance of R^{gg} is, independently, halogen, -CN, -NO₂, -N₃, -SO₂H, -SO₃H, -OH, -OC₁₋₆ alkyl, -ON(C₁₋₆ alkyl)₂, -N(C₁₋₆ alkyl)₂, -N(C₁₋₆ alkyl)₃⁺X⁻, -NH(C₁₋₆ alkyl)₂⁺X⁻, -NH₂(C₁₋₆ alkyl) ⁺X⁻, -NH₃⁺X⁻, -N(OC₁₋₆ alkyl)(C₁₋₆ alkyl), -N(OH)(C₁₋₆ alkyl), -NH(OH), -SH, -SC₁₋₆ alkyl, -SS(C₁₋₆ alkyl), -C(=O)(C₁₋₆ alkyl), -CO₂H, -CO₂(C₁₋₆ alkyl), -OC(=O)(C₁₋₆ alkyl), -OCO₂(C₁₋₆ alkyl), -C(=O)NH₂, -C(=O)N(C₁₋₆ alkyl)₂, -OC(=O)NH(C₁₋₆ alkyl), -NHC(=O)( C₁₋₆ alkyl), -N(C₁₋₆ alkyl)C(=O)( C₁₋₆ alkyl), -NHCO₂(C₁₋₆ alkyl), -NHC(=O)N(C₁₋₆ alkyl)₂, -NHC(=O)NH(C₁₋₆ alkyl), -NHC(=O)NH₂, -C(=NH)O(C₁₋₆ alkyl), -OC(=NH)(C₁₋₆ alkyl), -OC(=NH)OC₁₋₆ alkyl, -C(=NH)N(C₁₋₆ alkyl)₂, -C(=NH)NH(C₁₋₆ alkyl), -C(=NH)NH₂, -OC(=NH)N(C₁₋₆ alkyl)₂, -OC(NH)NH(C₁₋₆ alkyl), -OC(NH)NH₂, -NHC(NH)N(C₁₋₆ alkyl)₂, -NHC(=NH)NH₂, -NHSO₂(C₁₋₆ alkyl), -SO₂N(C₁₋₆ alkyl)₂, -SO₂NH(C₁₋₆ alkyl), -SO₂NH₂, -SO₂C₁₋₆ alkyl, -SO₂OC₁₋₆ alkyl, -OSO₂C₁₋₆ alkyl, -SOC₁₋₆ alkyl, -Si(C₁₋₆ alkyl)₃, -OSi(C₁₋₆ alkyl)₃ -C(=S)N(C₁₋₆ alkyl)₂, C(=S)NH(C₁₋₆ alkyl), C(=S)NH₂, -C(=O)S(C₁₋₆ alkyl), -C(=S)SC₁₋₆ alkyl, -SC(=S)SC₁₋₆ alkyl, -P(=O)(OC₁₋₆ alkyl)₂, -P(=O)(C₁₋₆ alkyl)₂, -OP(=O)(C₁₋₆ alkyl)₂, -OP(=O)(OC₁₋₆ alkyl)₂, C₁₋₆ alkyl, C₁₋₆ perhaloalkyl, C₂₋₆ alkenyl, C₂₋₆ alkynyl, heteroC₁₋₆alkyl, heteroC₂₋₆alkenyl, heteroC₂₋₆alkynyl, C₃₋₁₀ carbocyclyl, C₆₋₁₀ aryl, 3-10 membered heterocyclyl, 5-10 membered heteroaryl; or two geminal R^{gg} substituents can be joined to form =O or =S; wherein X⁻ is a counterion.

A "counterion" or "anionic counterion" is a negatively charged group associated with a positively charged group in order to maintain electronic neutrality. An anionic counterion may be monovalent (i.e., including one formal negative charge). An anionic counterion may also be multivalent (i.e., including more than one formal negative charge), such as divalent or trivalent. Exemplary counterions include halide ions (*e.g.,* F⁻, Cl⁻, Br, I ), NO₃⁻, ClO4⁻, OH⁻, H₂PO₄⁻, HCO₃⁻, HSO₄⁻, sulfonate ions (*e.g.,* methansulfonate, trifluoromethanesulfonate, *p-*toluenesulfonate, benzenesulfonate, 10-camphor sulfonate, naphthalene-2-sulfonate, naphthalene-1-sulfonic acid-5-sulfonate, ethan-1-sulfonic acid-2-sulfonate, and the like), carboxylate ions (e.g., acetate, propanoate, benzoate, glycerate, lactate, tartrate, glycolate, gluconate, and the like), BF₄⁻, PF₄⁻, PF₆⁻, AsF₆-, SbF₆-, B[3,5-(CF₃)₂C₆H₃]₄]⁻, B(C₆F₅)4⁻, BPh₄⁻, Al(OC(CF₃)₃)₄⁻, and carborane anions (*e.g*., CB₁₁H₁₂⁻ or (HCB₁₁Me₅Br₆)⁻). Exemplary counterions which may be multivalent include CO₃²⁻, HPO₄²⁻, PO₄³⁻, B₄O₇²⁻, SO₄²⁻, S₂O₃²⁻, carboxylate anions (e.g., tartrate, citrate, fumarate, maleate, malate, malonate, gluconate, succinate, glutarate, adipate, pimelate, suberate, azelate, sebacate, salicylate, phthalates, aspartate, glutamate, and the like), and carboranes.

"Halo" or "halogen" refers to fluorine (fluoro, -F), chlorine (chloro, -Cl), bromine (bromo, -Br), or iodine (iodo, -I).

The term "acyl" refers to a group having the general formula -C(=O)R^{X1}, -C(=O)OR^{X1}, - C(=O)-O-C(=O)R^{X1}, -C(=O)SR^{X1}, -C(=O)N(R^{X1})₂, -C(=S)R^{X1}, -C(=S)N(R^{X1})₂, and - C(=S)S(R^{X1}), -C(=NR^{X1})R^{X1}, -C(=NR^{X1})OR^{X1}, -C(=NR^{X1})SR^{X1}, and -C(=NR^{X1})N(R^{X1})₂, wherein R^{X1} is hydrogen; halogen; substituted or unsubstituted hydroxyl; substituted or unsubstituted thiol; substituted or unsubstituted amino; substituted or unsubstituted acyl, cyclic or acyclic, substituted or unsubstituted, branched or unbranched aliphatic; cyclic or acyclic, substituted or unsubstituted, branched or unbranched heteroaliphatic; cyclic or acyclic, substituted or unsubstituted, branched or unbranched alkyl; cyclic or acyclic, substituted or unsubstituted, branched or unbranched alkenyl; substituted or unsubstituted alkynyl; substituted or unsubstituted aryl, substituted or unsubstituted heteroaryl, aliphaticoxy, heteroaliphaticoxy, alkyloxy, heteroalkyloxy, aryloxy, heteroaryloxy, aliphaticthioxy, heteroaliphaticthioxy, alkylthioxy, heteroalkylthioxy, arylthioxy, heteroarylthioxy, mono- or di- aliphaticamino, mono- or di- heteroaliphaticamino, mono- or di- alkylamino, mono- or di- heteroalkylamino, mono- or di-arylamino, or mono- or di-heteroarylamino; or two R^{X1} groups taken together form a 5- to 6-membered heterocyclic ring. Exemplary acyl groups include aldehydes (-CHO), carboxylic acids (-CO₂H), ketones, acyl halides, esters, amides, imines, carbonates, carbamates, and ureas. Acyl substituents include, but are not limited to, any of the substituents described herein, that result in the formation of a stable moiety (*e*.*g*., aliphatic, alkyl, alkenyl, alkynyl, heteroaliphatic, heterocyclic, aryl, heteroaryl, acyl, oxo, imino, thiooxo, cyano, isocyano, amino, azido, nitro, hydroxyl, thiol, halo, aliphaticamino, heteroaliphaticamino, alkylamino, heteroalkylamino, arylamino, heteroarylamino, alkylaryl, arylalkyl, aliphaticoxy, heteroaliphaticoxy, alkyloxy, heteroalkyloxy, aryloxy, heteroaryloxy, aliphaticthioxy, heteroaliphaticthioxy, alkylthioxy, heteroalkylthioxy, arylthioxy, heteroarylthioxy, acyloxy, and the like, each of which may or may not be further substituted).

"Alkoxy" or "alkoxyl" refers to a radical of the formula: -O-alkyl.

Nitrogen atoms can be substituted or unsubstituted as valency permits, and include primary, secondary, tertiary, and quaternary nitrogen atoms. Exemplary nitrogen atom substituents include, but are not limited to, hydrogen, -OH, -OR^{aa}, -N(R^{cc})₂, -CN, -C(=O)R^{aa}, -C(=O)N(R^{cc})₂, -CO₂R^{aa}, -SO₂R^{aa}, -C(=NR^{bb})R^{aa}, -C(=NR^{cc})OR^{aa}, -C(=NR^{cc})N(R^{cc})₂, -SO₂N(R^{cc})₂, -SO₂R^{cc}, -SO₂OR^{cc}, -SOR^{aa}, -C(=S)N(R^{cc})₂, -C(=O)SR^{cc}, -C(=S)SR^{cc}, -P(=O)(OR^{cc})₂, -P(=O)(R^{aa})₂, -P(=O)(N(R^{cc})₂)₂, C₁₋₁₀ alkyl, C₁₋₁₀ perhaloalkyl, C₂₋₁₀ alkenyl, C₂₋₁₀ alkynyl, heteroC₁₋₁₀alkyl, heteroC₂₋₁₀alkenyl, heteroC₂₋₁₀alkynyl, C₃₋₁₀ carbocyclyl, 3-14 membered heterocyclyl, C₆₋₁₄ aryl, and 5-14 membered heteroaryl, or two R^{cc} groups attached to an N atom are joined to form a 3-14 membered heterocyclyl or 5-14 membered heteroaryl ring, wherein each alkyl, alkenyl, alkynyl, heteroalkyl, heteroalkenyl, heteroalkynyl, carbocyclyl, heterocyclyl, aryl, and heteroaryl is independently substituted with 0, 1, 2, 3, 4, or 5 R^{dd} groups, and wherein R^{aa}, R^{bb}, R^{cc} and R^{dd} are as defined above.

In certain embodiments, the substituent present on a nitrogen atom is a nitrogen protecting group (also referred to as an amino protecting group). Nitrogen protecting groups include, but are not limited to, -OH, -OR^{aa}, -N(R^{cc})₂, -C(=O)R^{aa}, -C(=O)N(R^{cc})₂, -CO₂R^{aa}, - SO₂R^{aa}, -C(=NR^{cc})R^{aa}, -C(=NR^{cc})OR^{aa}, -C(=NR^{cc})N(R^{cc})₂, -SO₁N(R^{cc})₂, -SO₂R^{cc}, -SO₂OR^{cc}, - SOR^{aa}, -C(=S)N(R^{cc})₂, -C(=O)SR^{cc}, -C(=S)SR^{cc}, C₁₋₁₀ alkyl (*e.g*., aralkyl, heteroaralkyl), C₂₋₁₀ alkenyl, C₂₋₁₀ alkynyl, C₃₋₁₀ carbocyclyl, 3-14 membered heterocyclyl, C₆₋₁₄ aryl, and 5-14 membered heteroaryl groups, wherein each alkyl, alkenyl, alkynyl, carbocyclyl, heterocyclyl, aralkyl, aryl, and heteroaryl is independently substituted with 0, 1, 2, 3, 4, or 5 R^{dd} groups, and wherein R^{aa}, R^{bb}, R^{cc} and R^{dd} are as defined herein. Nitrogen protecting groups are well known in the art and include those described in detail in Protecting Groups in Organic Synthesis, T. W. Greene and P. G. M. Wuts, 3rd edition, John Wiley & Sons, 1999.

For example, nitrogen protecting groups such as amide groups (*e.g*., -C(=O)R^{aa}) include, but are not limited to, formamide, acetamide, chloroacetamide, trichloroacetamide, trifluoroacetamide, phenylacetamide, 3-phenylpropanamide, picolinamide, 3-pyridylcarboxamide, *N*-benzoylphenylalanyl derivative, benzamide, *p*-phenylbenzamide, *o-*nitophenylacetamide, *o*-nitrophenoxyacetamide, acetoacetamide, (*N*'-dithiobenzyloxyacylamino)acetamide, 3-(*p*-hydroxyphenyl)propanamide, 3-(*o-*nitrophenyl)propanamide, 2-methyl-2-(*o*-nitrophenoxy)propanamide, 2-methyl-2-(*o-*phenylazophenoxy)propanamide, 4-chlorobutanamide, 3-methyl-3-nitrobutanamide, *o-*nitrocinnamide, *N*-acetylmethionine derivative, *o*-nitrobenzamide, and *o-*(benzoyloxymethyl)benzamide.

Nitrogen protecting groups such as carbamate groups (*e.g.,* -C(=O)OR^{aa}) include, but are not limited to, methyl carbamate, ethyl carbamate, 9-fluorenylmethyl carbamate (Fmoc), 9-(2-sulfo)fluorenylmethyl carbamate, 9-(2,7-dibromo)fluoroenylmethyl carbamate, 2,7-di-*t*-butyl-[9-(10,10-dioxo-10,10,10,10-tetrahydrothioxanthyl)]methyl carbamate (DBD-Tmoc), 4-methoxyphenacyl carbamate (Phenoc), 2,2,2-trichloroethyl carbamate (Troc), 2-trimethylsilylethyl carbamate (Teoc), 2-phenylethyl carbamate (hZ), 1-(1-adamantyl)-1-methylethyl carbamate (Adpoc), 1,1-dimethyl-2-haloethyl carbamate, 1,1-dimethyl-2,2-dibromoethyl carbamate (DB-*t*-BOC), 1,1-dimethyl-2,2,2-trichloroethyl carbamate (TCBOC), 1-methyl-1-(4-biphenylyl)ethyl carbamate (Bpoc), 1-(3,5-di-*t*-butylphenyl)-1-methylethyl carbamate (*t*-Bumeoc), 2-(2'- and 4'-pyridyl)ethyl carbamate (Pyoc), *2-*(*N,N-*dicyclohexylcarboxamido)ethyl carbamate, *t*-butyl carbamate (BOC), 1-adamantyl carbamate (Adoc), vinyl carbamate (Voc), allyl carbamate (Alloc), 1-isopropylallyl carbamate (Ipaoc), cinnamyl carbamate (Coc), 4-nitrocinnamyl carbamate (Noc), 8-quinolyl carbamate, *N-*hydroxypiperidinyl carbamate, alkyldithio carbamate, benzyl carbamate (Cbz), *p*-methoxybenzyl carbamate (Moz), *p*-nitobenzyl carbamate, *p*-bromobenzyl carbamate, *p*-chlorobenzyl carbamate, 2,4-dichlorobenzyl carbamate, 4-methylsulfinylbenzyl carbamate (Msz), 9-anthrylmethyl carbamate, diphenylmethyl carbamate, 2-methylthioethyl carbamate, 2-methylsulfonylethyl carbamate, 2-(*p*-toluenesulfonyl)ethyl carbamate, [2-(1,3-dithianyl)]methyl carbamate (Dmoc), 4-methylthiophenyl carbamate (Mtpc), 2,4-dimethylthiophenyl carbamate (Bmpc), 2-phosphonioethyl carbamate (Peoc), 2-triphenylphosphonioisopropyl carbamate (Ppoc), 1,1-dimethyl-2-cyanoethyl carbamate, *m-*chloro-*p*-acyloxybenzyl carbamate, *p*-(dihydroxyboryl)benzyl carbamate, 5-benzisoxazolylmethyl carbamate, 2-(trifluoromethyl)-6-chromonylmethyl carbamate (Tcroc), *m*-nitrophenyl carbamate, 3,5-dimethoxybenzyl carbamate, *o*-nitrobenzyl carbamate, 3,4-dimethoxy-6-nitrobenzyl carbamate, phenyl(*o*-nitrophenyl)methyl carbamate, *t*-amyl carbamate, *S*-benzyl thiocarbamate, *p*-cyanobenzyl carbamate, cyclobutyl carbamate, cyclohexyl carbamate, cyclopentyl carbamate, cyclopropylmethyl carbamate, *p*-decyloxybenzyl carbamate, 2,2-dimethoxyacylvinyl carbamate, o-(*N,N*-dimethylcarboxamido)benzyl carbamate, 1,1-dimethyl-3-(*N,N*-dimethylcarboxamido)propyl carbamate, 1,1-dimethylpropynyl carbamate, di(2-pyridyl)methyl carbamate, 2-furanylmethyl carbamate, 2-iodoethyl carbamate, isoborynl carbamate, isobutyl carbamate, isonicotinyl carbamate, *p*-(*p*'-methoxyphenylazo)benzyl carbamate, 1-methylcyclobutyl carbamate, 1-methylcyclohexyl carbamate, 1-methyl-1-cyclopropylmethyl carbamate, 1-methyl-1-(3,5-dimethoxyphenyl)ethyl carbamate, 1-methyl-1-(*p*-phenylazophenyl)ethyl carbamate, 1-methyl-1-phenylethyl carbamate, 1-methyl-1-(4-pyridyl)ethyl carbamate, phenyl carbamate, *p*-(phenylazo)benzyl carbamate, 2,4,6-tri-*t*-butylphenyl carbamate, 4-(trimethylammonium)benzyl carbamate, and 2,4,6-trimethylbenzyl carbamate.

Nitrogen protecting groups such as sulfonamide groups (*e.g.,* -S(=O)₂R^{aa}) include, but are not limited to, *p*-toluenesulfonamide (Ts), benzenesulfonamide, 2,3,6,-trimethyl-4-methoxybenzenesulfonamide (Mtr), 2,4,6-trimethoxybenzenesulfonamide (Mtb), 2,6-dimethyl-4-methoxybenzenesulfonamide (Pme), 2,3,5,6-tetramethyl-4-methoxybenzenesulfonamide (Mte), 4-methoxybenzenesulfonamide (Mbs), 2,4,6-trimethylbenzenesulfonamide (Mts), 2,6-dimethoxy-4-methylbenzenesulfonamide (iMds), 2,2,5,7,8-pentamethylchroman-6-sulfonamide (Pmc), methanesulfonamide (Ms), β-trimethylsilylethanesulfonamide (SES), 9-anthracenesulfonamide, 4-(4',8'-dimethoxynaphthylmethyl)benzenesulfonamide (DNMBS), benzylsulfonamide, trifluoromethylsulfonamide, and phenacylsulfonamide.

Other nitrogen protecting groups include, but are not limited to, phenothiazinyl-(10)-acyl derivative, *N'*-*p*-toluenesulfonylaminoacyl derivative, *N*'-phenylaminothioacyl derivative, *N-*benzoylphenylalanyl derivative, *N*-acetylmethionine derivative, 4,5-diphenyl-3-oxazolin-2-one, *N*-phthalimide, *N*-dithiasuccinimide (Dts), *N*-2,3-diphenylmaleimide, *N-*2,5-dimethylpyrrole, *N*-1,1,4,4-tetramethyldisilylazacyclopentane adduct (STABASE), 5-substituted 1,3-dimethyl-1,3,5-triazacyclohexan-2-one, 5-substituted 1,3-dibenzyl-1,3,5-triazacyclohexan-2-one, 1-substituted 3,5-dinitro-4-pyridone, *N*-methylamine, *N*-allylamine, *N*-[2-(trimethylsilyl)ethoxy]methylamine (SEM), *N*-3-acetoxypropylamine, *N*-(1-isopropyl-4-nitro-2-oxo-3-pyroolin-3-yl)amine, quaternary ammonium salts, *N*-benzylamine, N-di(4-methoxyphenyl)methylamine, *N*-5-dibenzosuberylamine, *N*-triphenylmethylamine (Tr), *N-*[(4-methoxyphenyl)diphenylmethyl]amine (MMTr), *N*-9-phenylfluorenylamine (PhF), *N*-2,7-dichloro-9-fluorenylmethyleneamine, *N*-ferrocenylmethylamino (Fcm), *N*-2-picolylamino *N'-*oxide, *N-*1,1-dimethylthiomethyleneamine, *N*-benzylideneamine, *N-p-*methoxybenzylideneamine, *N*-diphenylmethyleneamine, *N-*[(2-pyridyl)mesityl]methyleneamine, *N*-(*N'*,*N'*-dimethylaminomethylene)amine, *N,N-*isopropylidenediamine, *N*-p-nitrobenzylideneamine, *N*-salicylideneamine, *N-*5-chlorosalicylideneamine, *N*-(5-chloro-2-hydroxyphenyl)phenylmethyleneamine, *N-*cyclohexylideneamine, *N*-(5,5-dimethyl-3-oxo-1-cyclohexenyl)amine, *N*-borane derivative, *N*-diphenylborinic acid derivative, *N*-[phenyl(pentaacylchromium- or tungsten)acyl]amine, *N-*copper chelate, *N*-zinc chelate, *N*-nitroamine, *N*-nitrosoamine, amine *N*-oxide, diphenylphosphinamide (Dpp), dimethylthiophosphinamide (Mpt), diphenylthiophosphinamide (Ppt), dialkyl phosphoramidates, dibenzyl phosphoramidate, diphenyl phosphoramidate, benzenesulfenamide, o-nitrobenzenesulfenamide (Nps), 2,4-dinitrobenzenesulfenamide, pentachlorobenzenesulfenamide, 2-nitro-4-methoxybenzenesulfenamide, triphenylmethylsulfenamide, and 3-nitropyridinesulfenamide (Npys).

In certain embodiments, the substituent present on an oxygen atom is an oxygen protecting group (also referred to herein as an "hydroxyl protecting group"). Oxygen protecting groups include, but are not limited to, -R^{aa}, -N(R^{bb})₂, -C(=O)SR^{aa}, -C(=O)R^{aa}, -CO₂R^{aa}, -C(=O)N(R^{bb})₂, -C(=NR^{bb})R^{aa}, -C(=NR^{bb})OR^{aa}, -C(=NR^{bb})N(R^{bb})₂, -S(=O)R^{aa}, -SO₂R^{aa}, -Si(R^{aa})₃, -P(R^{cc})₂, -P(R^{cc})₃⁺X⁻, -P(OR^{cc})₂, -P(OR^{cc})₃⁺X⁻, -P(=O)(R^{aa})₂, -P(=O)(OR^{cc})₂, and -P(=O)(N(R^{bb}) ₂)₂, wherein X⁻, R^{aa}, R^{bb}, and R^{cc} are as defined herein. Oxygen protecting groups are well known in the art and include those described in detail in Protecting Groups in Organic Synthesis, T. W. Greene and P. G. M. Wuts, 3rd edition, John Wiley & Sons, 1999.

Exemplary oxygen protecting groups include, but are not limited to, methyl, methoxylmethyl (MOM), methylthiomethyl (MTM), *t*-butylthiomethyl, (phenyldimethylsilyl)methoxymethyl (SMOM), benzyloxymethyl (BOM), *p-*methoxybenzyloxymethyl (PMBM), (4-methoxyphenoxy)methyl (*p*-AOM), guaiacolmethyl (GUM), t-butoxymethyl, 4-pentenyloxymethyl (POM), siloxymethyl, 2-methoxyethoxymethyl (MEM), 2,2,2-trichloroethoxymethyl, bis(2-chloroethoxy)methyl, 2-(trimethylsilyl)ethoxymethyl (SEMOR), tetrahydropyranyl (THP), 3-bromotetrahydropyranyl, tetrahydrothiopyranyl, 1-methoxycyclohexyl, 4-methoxytetrahydropyranyl (MTHP), 4-methoxytetrahydrothiopyranyl, 4-methoxytetrahydrothiopyranyl S,S-dioxide, 1-[(2-chloro-4-methyl)phenyl]-4-methoxypiperidin-4-yl (CTMP), 1,4-dioxan-2-yl, tetrahydrofuranyl, tetrahydrothiofuranyl, 2,3,3a,4,5,6,7,7a-octahydro-7,8,8-trimethyl-4,7-methanobenzofuran-2-yl, 1-ethoxyethyl, 1-(2-chloroethoxy)ethyl, 1-methyl-1-methoxyethyl, 1-methyl-1-benzyloxyethyl, 1-methyl-1-benzyloxy-2-fluoroethyl, 2,2,2-trichloroethyl, 2-trimethylsilylethyl, 2-(phenylselenyl)ethyl, *t*-butyl, allyl, *p*-chlorophenyl, *p*-methoxyphenyl, 2,4-dinitrophenyl, benzyl (Bn), *p*-methoxybenzyl, 3,4-dimethoxybenzyl, *o-*nitrobenzyl, *p-*nitrobenzyl, *p*-halobenzyl, 2,6-dichlorobenzyl, *p*-cyanobenzyl, *p*-phenylbenzyl, 2-picolyl, 4-picolyl, 3-methyl-2-picolyl *N*-oxido, diphenylmethyl, *p,p*'-dinitrobenzhydryl, 5-dibenzosuberyl, triphenylmethyl, α-naphthyldiphenylmethyl, *p*-methoxyphenyldiphenylmethyl, di(*p*-methoxyphenyl)phenylmethyl, tri(*p*-methoxyphenyl)methyl, 4-(4'-bromophenacyloxyphenyl)diphenylmethyl, 4,4',4"-tris(4,5-dichlorophthalimidophenyl)methyl, 4,4',4"-tris(levulinoyloxyphenyl)methyl, 4,4',4"-tris(benzoyloxyphenyl)methyl, 3-(imidazol-1-yl)bis(4',4"-dimethoxyphenyl)methyl, 1,1-bis(4-methoxyphenyl)-1'-pyrenylmethyl, 9-anthryl, 9-(9-phenyl)xanthenyl, 9-(9-phenyl-10-oxo)anthryl, 1,3-benzodisulfuran-2-yl, benzisothiazolyl S,S-dioxido, trimethylsilyl (TMS), triethylsilyl (TES), triisopropylsilyl (TIPS), dimethylisopropylsilyl (IPDMS), diethylisopropylsilyl (DEIPS), dimethylthexylsilyl, *t-*butyldimethylsilyl (TBDMS), t-butyldiphenylsilyl (TBDPS), tribenzylsilyl, tri-p-xylylsilyl, triphenylsilyl, diphenylmethylsilyl (DPMS), t-butylmethoxyphenylsilyl (TBMPS), formate, benzoylformate, acetate, chloroacetate, dichloroacetate, trichloroacetate, trifluoroacetate, methoxyacetate, triphenylmethoxyacetate, phenoxyacetate, *p*-chlorophenoxyacetate, 3-phenylpropionate, 4-oxopentanoate (levulinate), 4,4-(ethylenedithio)pentanoate (levulinoyldithioacetal), pivaloate, adamantoate, crotonate, 4-methoxycrotonate, benzoate, p-phenylbenzoate, 2,4,6-trimethylbenzoate (mesitoate), alkyl methyl carbonate, 9-fluorenylmethyl carbonate (Fmoc), alkyl ethyl carbonate, alkyl 2,2,2-trichloroethyl carbonate (Troc), 2-(trimethylsilyl)ethyl carbonate (TMSEC), 2-(phenylsulfonyl) ethyl carbonate (Psec), 2-(triphenylphosphonio) ethyl carbonate (Peoc), alkyl isobutyl carbonate, alkyl vinyl carbonate alkyl allyl carbonate, alkyl *p*-nitrophenyl carbonate, alkyl benzyl carbonate, alkyl *p-*methoxybenzyl carbonate, alkyl 3,4-dimethoxybenzyl carbonate, alkyl o-nitrobenzyl carbonate, alkyl *p*-nitrobenzyl carbonate, alkyl S-benzyl thiocarbonate, 4-ethoxy-1-napththyl carbonate, methyl dithiocarbonate, 2-iodobenzoate, 4-azidobutyrate, 4-nitro-4-methylpentanoate, *o-*(dibromomethyl)benzoate, 2-formylbenzenesulfonate, 2-(methylthiomethoxy)ethyl, 4-(methylthiomethoxy)butyrate, 2-(methylthiomethoxymethyl)benzoate, 2,6-dichloro-4-methylphenoxyacetate, 2,6-dichloro-4-(1,1,3,3-tetramethylbutyl)phenoxyacetate, 2,4-bis(1,1-dimethylpropyl)phenoxyacetate, chlorodiphenylacetate, isobutyrate, monosuccinoate, (*E*)-2-methyl-2-butenoate, o-(methoxyacyl)benzoate, α-naphthoate, nitrate, alkyl *N,N,N',N'-*tetramethylphosphorodiamidate, alkyl *N*-phenylcarbamate, borate, dimethylphosphinothioyl, alkyl 2,4-dinitrophenylsulfenate, sulfate, methanesulfonate (mesylate), benzylsulfonate, and tosylate (Ts).

In certain embodiments, the substituent present on a sulfur atom is a sulfur protecting group (also referred to as a "thiol protecting group"). Sulfur protecting groups include, but are not limited to, -R^{aa}, -N(R^{bb})₂, -C(=O)SR^{aa}, -C(=O)R^{aa}, -CO₂R^{aa}, -C(=O)N(R^{bb})₂, -C(=NR^{bb})R^{aa}, -C(=NR^{bb})OR^{aa}, -C(=NR^{bb})N(R^{bb})₂, -S(=O)R^{aa}, -SO₂R^{aa}, -Si(R^{aa})₃, -P(R^{cc})₂, -P(R^{cc})₃⁺X⁻, -P(OR^{cc})₂, -P(OR^{cc})₃⁺X⁻, -P(=O)(R^{aa})₂, -P(=O)(OR^{cc})₂, and -P(=O)(N(R^{bb}) ₂)₂, wherein R^{aa}, R^{bb}, and R^{cc} are as defined herein. Sulfur protecting groups are well known in the art and include those described in detail in Protecting Groups in Organic Synthesis, T. W. Greene and P. G. M. Wuts, 3rd edition, John Wiley & Sons, 1999.

As used herein, a "leaving group" (LG) is an art-understood term referring to a molecular fragment that departs with a pair of electrons in a heterolytic bond cleavage, wherein the molecular fragment is an anion or neutral molecule. As used herein, a leaving group can be an atom or a group capable of being displaced by a nucleophile. *See,* for example, Smith, March Advanced Organic Chemistry 6th ed. (501-502). Exemplary leaving groups include, but are not limited to, halo (*e.g.,* chloro, bromo, iodo) and activated substituted hydroxyl groups (*e.g.,* -OC(=O)SR^{aa}, -OC(=O)R^{aa}, -OCO₂R^{aa}, -OC(=O)N(R^{bb})₂, -OC(=NR^{bb})R^{aa}, -OC(=NR^{bb})OR^{aa}, - OC(=NR^{bb})N(R^{bb})₂, -OS(=O)R^{aa}, -OSO₂R^{aa}, -OP(R^{cc})₂, -OP(R^{cc})₃, -OP(=O)₂R^{aa}, - OP(=O)(R^{aa})₂, -OP(=O)(OR^{cc})₂, -OP(=O)₂N(R^{bb})₂, and -OP(=O)(NR^{bb})₂, wherein R^{aa}, R^{bb}, and R^{cc} are as defined herein). Examples of suitable leaving groups include, but are not limited to, halogen (such as F, Cl, Br, or I (iodine)), alkoxycarbonyloxy, aryloxycarbonyloxy, alkanesulfonyloxy, arenesulfonyloxy, alkyl-carbonyloxy (*e.g*., acetoxy), arylcarbonyloxy, aryloxy, methoxy, *N,O*-dimethylhydroxylamino, pixyl, and haloformates. In some cases, the leaving group is a sulfonic acid ester, such as toluenesulfonate (tosylate, -OTs), methanesulfonate (mesylate, -OMs), *p*-bromobenzenesulfonyloxy (brosylate, -OBs), or trifluoromethanesulfonate (triflate, -OTf). In some cases, the leaving group is a brosylate, such as p-bromobenzenesulfonyloxy. In some cases, the leaving group is a nosylate, such as 2-nitrobenzenesulfonyloxy. In some embodiments, the leaving group is a sulfonate-containing group. In some embodiments, the leaving group is a tosylate group. The leaving group may also be a phosphineoxide (*e.g*., formed during a Mitsunobu reaction) or an internal leaving group such as an epoxide or cyclic sulfate. Other non-limiting examples of leaving groups are water, amines, ammonia, alcohols, ether moieties, sulfur-containing moieties, thioether moieties, zinc halides, magnesium moieties, diazonium salts, and copper moieties.

The term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, Berge et al., describe pharmaceutically acceptable salts in detail in J. Pharmaceutical Sciences, 1977, 66, 1-19. Pharmaceutically acceptable salts of the compounds of the present disclosure include those derived from suitable inorganic and organic acids and bases. Examples of pharmaceutically acceptable, nontoxic acid addition salts are salts of an amino group formed with inorganic acids such as hydrochloric acid, hydrobromic acid, phosphoric acid, sulfuric acid, and perchloric acid or with organic acids such as acetic acid, oxalic acid, maleic acid, tartaric acid, citric acid, succinic acid, or malonic acid or by using other methods known in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Salts derived from appropriate bases include alkali metal, alkaline earth metal, ammonium and N⁺(C₁₋₄ alkyl)₄⁻ salts. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, lower alkyl sulfonate, and aryl sulfonate.

The term "solvate" refers to forms of the compound that are associated with a solvent, usually by a solvolysis reaction. This physical association may include hydrogen bonding. Conventional solvents include water, methanol, ethanol, acetic acid, DMSO, THF, diethyl ether, and the like. The compounds of Formulae **(I)** or **(II)** may be prepared, *e.g.,* in crystalline form, and may be solvated. Suitable solvates include pharmaceutically acceptable solvates and further include both stoichiometric solvates and non-stoichiometric solvates. In certain instances, the solvate will be capable of isolation, for example, when one or more solvent molecules are incorporated in the crystal lattice of a crystalline solid. "Solvate" encompasses both solution-phase and isolable solvates. Representative solvates include hydrates, ethanolates, and methanolates.

The term "hydrate" refers to a compound that is associated with water. Typically, the number of the water molecules contained in a hydrate of a compound is in a definite ratio to the number of the compound molecules in the hydrate. Therefore, a hydrate of a compound may be represented, for example, by the general formula R·x H₂O, wherein R is the compound and wherein x is a number greater than 0. A given compound may form more than one type of hydrates, including, *e.g*., monohydrates (x is 1), lower hydrates (x is a number greater than 0 and smaller than 1, *e.g*., hemihydrates (R·0.5 H₂O)), and polyhydrates (x is a number greater than 1, *e.g*., dihydrates (R·2 H₂O) and hexahydrates (R·6 H₂O)).

The term "tautomers" refer to compounds that are interchangeable forms of a particular compound structure, and that vary in the displacement of hydrogen atoms and electrons. Thus, two structures may be in equilibrium through the movement of π electrons and an atom (usually H). For example, enols and ketones are tautomers because they are rapidly interconverted by treatment with either acid or base. Another example of tautomerism is the aci- and nitro- forms of phenylnitromethane, that are likewise formed by treatment with acid or base.

Tautomeric forms may be relevant to the attainment of the optimal chemical reactivity and biological activity of a compound of interest.

It is also to be understood that compounds that have the same molecular formula but differ in the nature or sequence of bonding of their atoms or the arrangement of their atoms in space are termed "isomers." Isomers that differ in the arrangement of their atoms in space are termed "stereoisomers."

Stereoisomers that are not mirror images of one another are termed "diastereomers" and those that are non-superimposable mirror images of each other are termed "enantiomers." When a compound has an asymmetric center, for example, it is bonded to four different groups, a pair of enantiomers is possible. An enantiomer can be characterized by the absolute configuration of its asymmetric center and is described by the R- and S-sequencing rules of Cahn and Prelog, or by the manner in which the molecule rotates the plane of polarized light and designated as dextrorotatory or levorotatory (*i.e.,* as (+) or (-)-isomers respectively). A chiral compound can exist as either individual enantiomer or as a mixture thereof. A mixture containing equal proportions of the enantiomers is called a "racemic mixture."

The term "polymorphs" refers to a crystalline form of a compound (or a salt, hydrate, or solvate thereof) in a particular crystal packing arrangement. All polymorphs have the same elemental composition. Different crystalline forms usually have different X-ray diffraction patterns, infrared spectra, melting points, density, hardness, crystal shape, optical and electrical properties, stability, and solubility. Recrystallization solvent, rate of crystallization, storage temperature, and other factors may cause one crystal form to dominate. Various polymorphs of a compound can be prepared by crystallization under different conditions.

The term "co-crystal" refers to a crystalline structure comprising at least two different components (*e.g*., a compound of Formula (I) and an acid), wherein each of the components is independently an atom, ion, or molecule. In certain embodiments, none of the components are a solvent. In certain embodiments, at least one of the components is a solvent. A co-crystal of a compound of Formula (I) and an acid is different from a salt formed from a compound of Formula (I) and an acid. In the salt, a compound of Formula (I) is complexed with the acid in a way that proton transfer (*e.g*., a complete proton transfer) from the acid to a compound of Formula (I) easily occurs at room temperature. In the co-crystal, however, a compound of Formula (I) is complexed with the acid in a way that proton transfer from the acid to a compound Formula (I) does not easily occur at room temperature. In certain embodiments, in the co-crystal, there is no proton transfer from the acid to the compound of Formula (I). In certain embodiments, in the co-crystal, there is partial proton transfer from the acid to the compound of Formula (I). Co-crystals may be useful to improve the properties (*e.g*., solubility, stability, and ease of formulation) of a compound of Formula (I).

Unless otherwise stated, structures depicted herein are also meant to include compounds that differ only in the presence of one or more isotopically enriched atoms. For example, compounds having the present structures except for the replacement of hydrogen by deuterium or tritium, replacement of ¹⁹F with ¹⁸F, or the replacement of ¹²C with ¹³C or ¹⁴C are within the scope of the disclosure. Such isotopically labeled derivatives are useful, for example, as analytical tools or probes in biological assays.

A "subject" to which administration is contemplated includes, but is not limited to, humans (*i.e.,* a male or female of any age group, *e.g.,* a pediatric subject (*e.g.,* infant, child, adolescent) or adult subject (*e.g*., young adult, middle-aged adult, or senior adult)) and/or other non-human animals, for example, mammals (*e.g.,* primates (*e.g.,* cynomolgus monkeys, rhesus monkeys); commercially relevant mammals such as cattle, pigs, horses, sheep, goats, cats, and/or dogs) and birds (*e.g*., commercially relevant birds such as chickens, ducks, geese, and/or turkeys). In certain embodiments, the animal is a mammal. The animal may be a male or female and at any stage of development. A non-human animal may be a transgenic animal.

The terms "administer," "administering," or "administration," refers to implanting, absorbing, ingesting, injecting, inhaling, or otherwise introducing a compound according to the present disclosure, or a pharmaceutical composition thereof.

The terms "treatment," "treat," and "treating" refer to reversing, alleviating, delaying the onset of, or inhibiting the progress of a "pathological condition" (*e.g*., a disease, disorder, or condition, or one or more signs or symptoms thereof) described herein. In some embodiments, treatment may be administered after one or more signs or symptoms have developed or have been observed. In other embodiments, treatment may be administered in the absence of signs or symptoms of the disease or condition. For example, treatment may be administered to a susceptible individual prior to the onset of symptoms (*e.g*., in light of a history of symptoms and/or in light of genetic or other susceptibility factors). Treatment may also be continued after symptoms have resolved, for example, to delay or prevent recurrence.

In some embodiments, "treatment," "treat," and "treating" refer to reversing the progress of a pathological condition. In some embodiments, "treatment," "treat," and "treating" refer to reversing the progress of one or more symptoms of a pathological condition.

In some embodiments, "treatment," "treat," and "treating" refer to alleviating a pathological condition. In some embodiments, "treatment," "treat," and "treating" refer to alleviating one or more symptoms of a pathological condition.

In some embodiments, "treatment," "treat," and "treating" refer to delaying the onset of a pathological condition. In some embodiments, "treatment," "treat," and "treating" refer to delaying the onset of one or more symptoms of a pathological condition.

In some embodiments, "treatment," "treat," and "treating" refer to inhibiting the progress of a pathological condition. In some embodiments, "treatment," "treat," and "treating" refer to inhibiting the progress of one or more symptoms of a pathological condition.

The terms "condition," "disease," and "disorder" are used interchangeably.

An "effective amount" of a compound of Formulae **(I)** or **(II)** refers to an amount sufficient to elicit the desired biological response, *i.e.,* treating the condition. As will be appreciated by those of ordinary skill in this art, the effective amount of a compound of Formulae **(I)** or **(II)** may vary depending on such factors as the desired biological endpoint, the pharmacokinetics of the compound, the condition being treated, the mode of administration, and the age and health of the subject. An effective amount encompasses therapeutic and prophylactic treatment. For example, in treating cancer, an effective amount of a compound disclosed herein may reduce the tumor burden or stop the growth or spread of a tumor.

A "therapeutically effective amount" of a compound of Formulae **(I)** or **(II)** is an amount sufficient to provide a therapeutic benefit in the treatment of a condition or to delay or minimize one or more symptoms associated with the condition. A therapeutically effective amount of a compound means an amount of therapeutic agent, alone or in combination with other therapies, which provides a therapeutic benefit in the treatment of the condition. The term "therapeutically effective amount" can encompass an amount that improves overall therapy, reduces, or avoids symptoms or causes of the condition, or enhances the therapeutic efficacy of another therapeutic agent.

A "prophylactically effective amount" of a compound of Formulae **(I)** or **(II)** is an amount sufficient to prevent a condition, or one or more symptoms associated with the condition or prevent its recurrence. A prophylactically effective amount of a compound means an amount of a therapeutic agent, alone or in combination with other agents, which provides a prophylactic benefit in the prevention of the condition. The term "prophylactically effective amount" can encompass an amount that improves overall prophylaxis or enhances the prophylactic efficacy of another prophylactic agent. In some embodiments, the prophylactically effective amount improves overall prophylaxis. In some embodiments, the period of prophylaxis is the life of the subject. In some embodiments, the period of prophylaxis is greater than or equal to 50 years. In some embodiments, the period of prophylaxis is greater than or equal to 40 years. In some embodiments, the period of prophylaxis is greater than or equal to 30 years. In some embodiments, the period of prophylaxis is greater than or equal to 20 years. In some embodiments, the period of prophylaxis is greater than or equal to 10 years. In some embodiments, the period of prophylaxis is greater than or equal to 5 years. In some embodiments, the period of prophylaxis is greater than or equal to 4 years. In some embodiments, the period of prophylaxis is 3 greater than or equal to years. In some embodiments, the period of prophylaxis is greater than or equal to 2 years. In some embodiments, the period of prophylaxis is greater than or equal to 1 year. In some embodiments, the period of prophylaxis is greater than or equal to 11 months. In some embodiments, the period of prophylaxis is greater than or equal to 10 months. In some embodiments, the period of prophylaxis is greater than or equal to 9 months. In some embodiments, the period of prophylaxis is greater than or equal to 8 months. In some embodiments, the period of prophylaxis is greater than or equal to 7 months. In some embodiments, the period of prophylaxis is greater than or equal to 6 months. In some embodiments, the period of prophylaxis is greater than or equal to 5 months. In some embodiments, the period of prophylaxis is greater than or equal to 4 months. In some embodiments, the period of prophylaxis is greater than or equal to 3 months. In some embodiments, the period of prophylaxis is greater than or equal to 2 months. In some embodiments, the period of prophylaxis is greater than or equal to 1 month.

A "proliferative disease" refers to a disease that occurs due to abnormal growth or extension by the multiplication of cells (Walker, Cambridge Dictionary of Biology; Cambridge University Press: Cambridge, UK, 1990). A proliferative disease may be associated with: 1) the pathological proliferation of normally quiescent cells; 2) the pathological migration of cells from their normal location (*e.g*., metastasis of neoplastic cells); 3) the pathological expression of proteolytic enzymes such as the matrix metalloproteinases (*e.g*., collagenases, gelatinases, and elastases); or 4) the pathological angiogenesis as in proliferative retinopathy and tumor metastasis. Exemplary proliferative diseases include cancers (*i.e.,* "malignant neoplasms"), benign neoplasms, angiogenesis, inflammatory diseases, autoinflammatory diseases, and autoimmune diseases.

The terms "neoplasm" and "tumor" are used interchangeably and refer to an abnormal mass of tissue wherein the growth of the mass surpasses and is not coordinated with the growth of a normal tissue. A neoplasm or tumor may be "benign" or "malignant," depending on the following characteristics: degree of cellular differentiation (including morphology and functionality), rate of growth, local invasion, and metastasis. A "benign neoplasm" is generally well differentiated, has characteristically slower growth than a malignant neoplasm, and remains localized to the site of origin. In addition, a benign neoplasm does not have the capacity to infiltrate, invade, or metastasize to distant sites. Exemplary benign neoplasms include, but are not limited to, lipoma, chondroma, adenomas, acrochordon, senile angiomas, seborrheic keratoses, lentigos, and sebaceous hyperplasias. In some cases, certain "benign" tumors may later give rise to malignant neoplasms, which may result from additional genetic changes in a subpopulation of the tumor's neoplastic cells, and these tumors are referred to as "pre-malignant neoplasms." An exemplary pre-malignant neoplasm is a teratoma. In contrast, a "malignant neoplasm" is generally poorly differentiated (anaplasia) and has characteristically rapid growth accompanied by progressive infiltration, invasion, and destruction of the surrounding tissue. Furthermore, a malignant neoplasm generally has the capacity to metastasize to distant sites.

The term "metastasis," "metastatic," or "metastasize" refers to the spread or migration of cancerous cells from a primary or original tumor to another organ or tissue and is typically identifiable by the presence of a "secondary tumor" or "secondary cell mass" of the tissue type of the primary or original tumor and not of that of the organ or tissue in which the secondary (metastatic) tumor is located. For example, a prostate cancer that has migrated to bone is said to be metastasized prostate cancer and includes cancerous prostate cancer cells growing in bone tissue.

The term "cancer" refers to a malignant neoplasm (*S*tedman 's Medical Dictionary, 25th ed.; Hensyl ed.; Williams & Wilkins: Philadelphia, 1990). Exemplary cancers include, but are not limited to, acoustic neuroma; adenocarcinoma; adrenal gland cancer; anal cancer; angiosarcoma (*e.g*., lymphangiosarcoma, lymphangioendotheliosarcoma, hemangiosarcoma); appendix cancer; benign monoclonal gammopathy; biliary cancer (*e.g*., cholangiocarcinoma); bladder cancer; breast cancer (*e.g*., adenocarcinoma of the breast, papillary carcinoma of the breast, mammary cancer, medullary carcinoma of the breast); brain cancer (*e.g*., meningioma, glioblastomas, glioma (*e.g*., astrocytoma, oligodendroglioma), medulloblastoma); bronchus cancer; carcinoid tumor; cervical cancer (*e.g*., cervical adenocarcinoma); choriocarcinoma; chordoma; craniopharyngioma; colorectal cancer (*e.g*., colon cancer, rectal cancer, colorectal adenocarcinoma); connective tissue cancer; epithelial carcinoma; ependymoma; endotheliosarcoma (*e.g*., Kaposi's sarcoma, multiple idiopathic hemorrhagic sarcoma); endometrial cancer (*e.g.,* uterine cancer, uterine sarcoma); esophageal cancer (*e.g.,* adenocarcinoma of the esophagus, Barrett's adenocarcinoma); Ewing's sarcoma; eye cancer (*e.g.*, intraocular melanoma, retinoblastoma); familiar hypereosinophilia; gall bladder cancer; gastric cancer (*e.g*., stomach adenocarcinoma); gastrointestinal stromal tumor (GIST); germ cell cancer; head and neck cancer (*e.g.,* head and neck squamous cell carcinoma, oral cancer (*e.g.,* oral squamous cell carcinoma), throat cancer (*e.g*., laryngeal cancer, pharyngeal cancer, nasopharyngeal cancer, oropharyngeal cancer)); hematopoietic cancers (*e.g*., leukemia such as acute lymphocytic leukemia (ALL) (*e.g.,* B-cell ALL, T-cell ALL), acute myelocytic leukemia (AML) (*e.g.,* B-cell AML, T-cell AML), chronic myelocytic leukemia (CML) (*e.g.,* B-cell CML, T-cell CML), and chronic lymphocytic leukemia (CLL) (*e.g.,* B-cell CLL, T-cell CLL)); lymphoma such as Hodgkin lymphoma (HL) (*e.g.,* B-cell HL, T-cell HL) and non-Hodgkin lymphoma (NHL) (*e.g.,* B-cell NHL such as diffuse large cell lymphoma (DLCL) (*e.g.,* diffuse large B-cell lymphoma), follicular lymphoma, chronic lymphocytic leukemia/small lymphocytic lymphoma (CLL/SLL), mantle cell lymphoma (MCL), marginal zone B-cell lymphomas (*e.g*., mucosa-associated lymphoid tissue (MALT) lymphomas, nodal marginal zone B-cell lymphoma, splenic marginal zone B-cell lymphoma), primary mediastinal B-cell lymphoma, Burkitt lymphoma, lymphoplasmacytic lymphoma (*i.e.,* Waldenström's macroglobulinemia), hairy cell leukemia (HCL), immunoblastic large cell lymphoma, precursor B-lymphoblastic lymphoma and primary central nervous system (CNS) lymphoma; and T-cell NHL such as precursor T-lymphoblastic lymphoma/leukemia, peripheral T-cell lymphoma (PTCL) (*e.g*., cutaneous T-cell lymphoma (CTCL) (*e.g*., mycosis fungoides, Sezary syndrome), angioimmunoblastic T-cell lymphoma, extranodal natural killer T-cell lymphoma, enteropathy type T-cell lymphoma, subcutaneous panniculitis-like T-cell lymphoma, and anaplastic large cell lymphoma); a mixture of one or more leukemia/lymphoma as described above; and multiple myeloma (MM)), heavy chain disease (*e.g*., alpha chain disease, gamma chain disease, mu chain disease); hemangioblastoma; hypopharynx cancer; inflammatory myofibroblastic tumors; immunocytic amyloidosis; kidney cancer (*e.g.,* nephroblastoma *a.k.a.* Wilms' tumor, renal cell carcinoma); liver cancer (*e.g.,* hepatocellular cancer (HCC), malignant hepatoma); lung cancer (*e.g.,* bronchogenic carcinoma, small cell lung cancer (SCLC), non-small cell lung cancer (NSCLC), adenocarcinoma of the lung); leiomyosarcoma (LMS); mastocytosis (*e.g*., systemic mastocytosis); muscle cancer; myelodysplastic syndrome (MDS); mesothelioma; myeloproliferative disorder (MPD) (*e.g.,* polycythemia vera (PV), essential thrombocytosis (ET), agnogenic myeloid metaplasia (AMM) *a.k.a.* myelofibrosis (MF), chronic idiopathic myelofibrosis, chronic myelocytic leukemia (CML), chronic neutrophilic leukemia (CNL), hypereosinophilic syndrome (HES)); neuroblastoma; neurofibroma (*e.g*., neurofibromatosis (NF) type 1 or type 2, schwannomatosis); neuroendocrine cancer (*e.g*., gastroenteropancreatic neuroendocrinetumor (GEP-NET), carcinoid tumor); osteosarcoma (*e.g.*, bone cancer); ovarian cancer (*e.g*., cystadenocarcinoma, ovarian embryonal carcinoma, ovarian adenocarcinoma); papillary adenocarcinoma; pancreatic cancer (*e.g*., pancreatic andenocarcinoma, intraductal papillary mucinous neoplasm (IPMN), Islet cell tumors); penile cancer (*e.g*., Paget's disease of the penis and scrotum); pinealoma; primitive neuroectodermal tumor (PNT); plasma cell neoplasia; paraneoplastic syndromes; intraepithelial neoplasms; prostate cancer (*e.g*., prostate adenocarcinoma); rectal cancer; rhabdomyosarcoma; salivary gland cancer; skin cancer *(e.g.,* squamous cell carcinoma (SCC), keratoacanthoma (KA), melanoma, basal cell carcinoma (BCC)); small bowel cancer (*e.g.,* appendix cancer); soft tissue sarcoma (*e.g.,* malignant fibrous histiocytoma (MFH), liposarcoma, malignant peripheral nerve sheath tumor (MPNST), chondrosarcoma, fibrosarcoma, myxosarcoma); sebaceous gland carcinoma; small intestine cancer; sweat gland carcinoma; synovioma; testicular cancer (*e.g.*, seminoma, testicular embryonal carcinoma); thyroid cancer (*e.g*., papillary carcinoma of the thyroid, papillary thyroid carcinoma (PTC), medullary thyroid cancer); urethral cancer; vaginal cancer; and vulvar cancer (*e.g*., Paget's disease of the vulva).

The term "angiogenesis" refers to the formation and the growth of new blood vessels. Normal angiogenesis occurs in the healthy body of a subject for healing wounds and for restoring blood flow to tissues after injury. The healthy body controls angiogenesis through a number of means, *e.g*., angiogenesis-stimulating growth factors and angiogenesis inhibitors. Many disease states, such as cancer, diabetic blindness, age-related macular degeneration, rheumatoid arthritis, and psoriasis, are characterized by abnormal (*i.e.,* increased or excessive) angiogenesis. Abnormal or pathological angiogenesis refers to angiogenesis greater than that in a normal body, especially angiogenesis in an adult not related to normal angiogenesis (*e.g*., menstruation or wound healing). Abnormal angiogenesis can provide new blood vessels that feed diseased tissues and/or destroy normal tissues, and in the case of cancer, the new vessels can allow tumor cells to escape into the circulation and lodge in other organs (tumor metastases). In certain embodiments, the angiogenesis is pathological angiogenesis.

The term "cardiovascular disease" or "heart disease" refers to diseases associated the heart and/or blood vessels. Exemplary cardiovascular diseases include, but are not limited to, coronary heart disease, stroke or cerebrovascular disease, congenital heart defects, peripheral artery disease, heart disease associated with atherosclerosis, ischemic heart disease, hypertensive heart disease, rheumatic heart disease, cardiac arrhythmias, heart failure, congenital heart disease, inflammatory heart disease, cardiomyopathy, pericardial disease, and valvular heart disease.

The term "biological sample" refers to any sample including tissue samples (such as tissue sections and needle biopsies of a tissue); cell samples (*e.g*., cytological smears (such as Pap or blood smears) or samples of cells obtained by microdissection); samples of whole organisms (such as samples of yeasts or bacteria); or cell fractions, fragments, or organelles (such as obtained by lysing cells and separating the components thereof by centrifugation or otherwise). Other examples of biological samples include blood, serum, urine, semen, fecal matter, cerebrospinal fluid, interstitial fluid, mucus, tears, sweat, pus, biopsied tissue (*e.g*., obtained by a surgical biopsy or needle biopsy), nipple aspirates, milk, vaginal fluid, saliva, swabs (such as buccal swabs), or any material containing biomolecules that is derived from a first biological sample. Biological samples also include those biological samples that are transgenic, such as a transgenic oocyte, sperm cell, blastocyst, embryo, fetus, donor cell, or cell nucleus, or cells or cell lines derived from biological samples.

The term "demethylase" refers to any enzyme that catalyzes the removal of methyl groups from a substrate (*e.g.,* nucleic acids, proteins (*e.g.,* histones), metabolites, natural products and intermediates thereto, and other compounds). A "histone demethylase" catalyzes the removal of a methyl group from a histone protein. For example, a "histone Lys demethylase," or "KDM" catalyzes the removal of a methyl group from the N-methyl lysine residue of a histone protein. KDM's are categorized into two subfamilies: the flavin-dependent KDM1 subfamily, and the 2-oxoglutarate- (2OG) dependent JmjC subfamily (KDM2-7 subfamily). In certain embodiments, the histone demethylase is a KDM. The overexpression of certain KDM's (*e.g*., KDM's in the JmjC KDM2-7 subfamily) has been linked to cancer. Overexpression of JmjC KDM's has been observed in multiple types of cancer cells. Examples of KDM's include, but are not limited to, KDM2/7, KDM3, KDM4, KDMS, and KDM6. Examples of KDM5 include KDMSA, KDMSB, and KDM5C. For KDM5A (*homo sapiens*)*,* exemplary sequences from GenBank are of Genbank ID 5927. For KDM5B (*homo sapiens*)*,* exemplary sequences from GenBank are of Genbank ID 10765. For KDM5C (*homo sapiens*)*,* exemplary sequences from GenBank are of Genbank ID 8242.

### BRIEF DESCRIPTION OF THE DRAWINGS

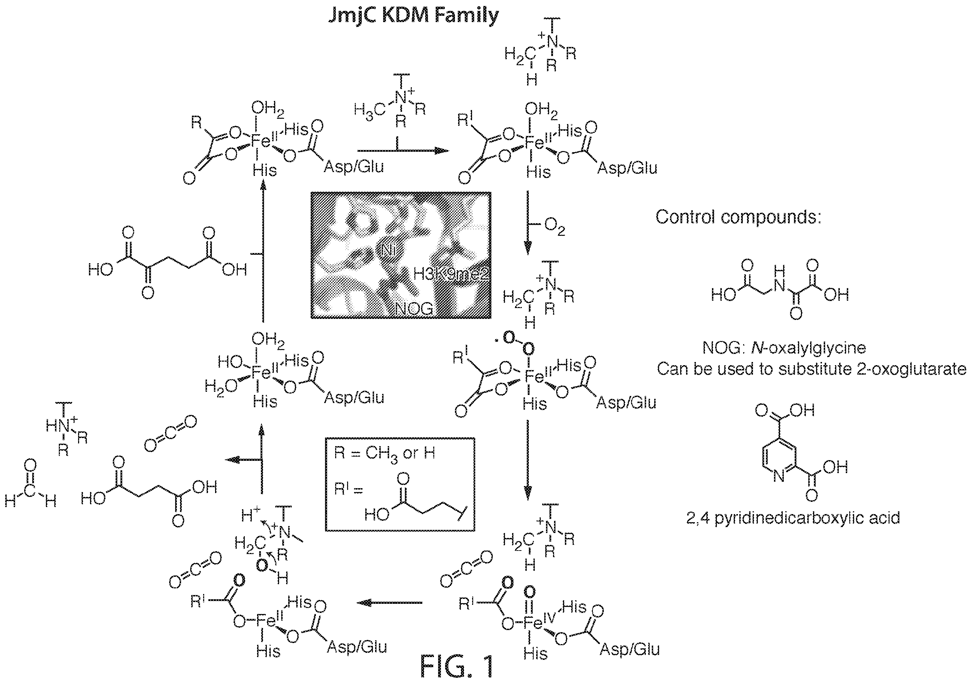

FIG. 1 shows the JmjC KDM family. The positive control compounds for JmjC KDM inhibitors are N-oxalyglycine ("NOG") and 2,4 pyridinedicarboxylic acid. N-oxalyglycine can be used to substitute 2-oxoglutarate.

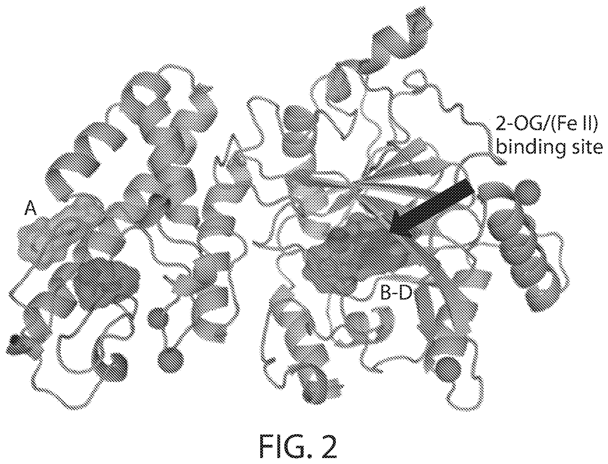

FIG. 2 shows the protein structure of the 2-OG/(Fe (II)) binding site.
FIGs. 3A-3B show data for KDM5 selective inhibitor PCK62 and other exemplary KDM5 inhibitors in a KDM5B_alphascreen activity assay. FIGs. 3A-3B also depict exemplary KDM5 inhibitor PCK62 (JADA62). FIGs. 3A-3B show data for PCK62 against different KDM's (KDM5A, KDM5B, KDM5C; KDM3A, and KDM3B). PCK62 shows reasonable inhibitory activity of KDM5, as compared to other exemplary KDM5 inhibitors.

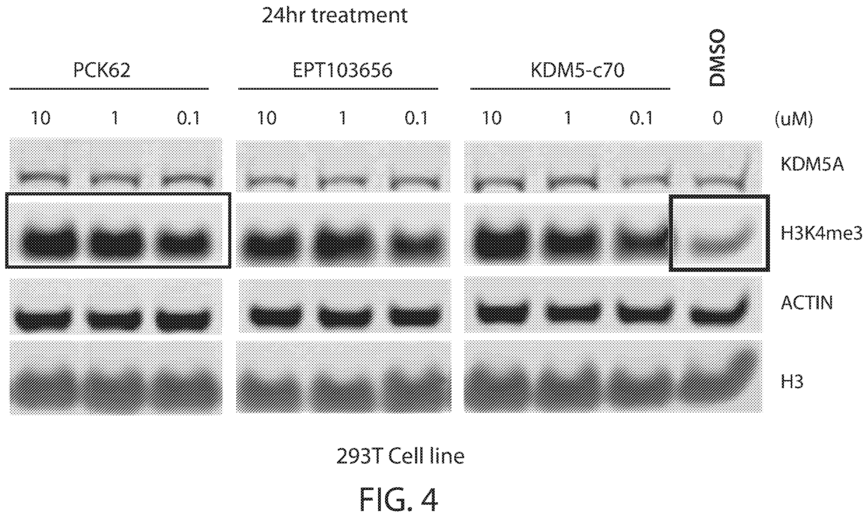

FIG. 4 shows a Western blot showing the cellular activity of PCK62 and other exemplary KDM5 inhibitors at the indicated concentrations (at 10 µM, 1 µM, and 0.1 µM), against KDMSA, H3K4me3 (KDM5 target that is methylated), actin (positive control), and H3 (positive control) upon 24 hour treatment using these exemplary KDM5 inhibitors in the 293T cell line. The blot shows increased inhibition of KDM activity (increased change in methylation level) by the exemplary KDM5 inhibitors (including PCK62) against H3K4me3 (KDM5 target that is methylated).

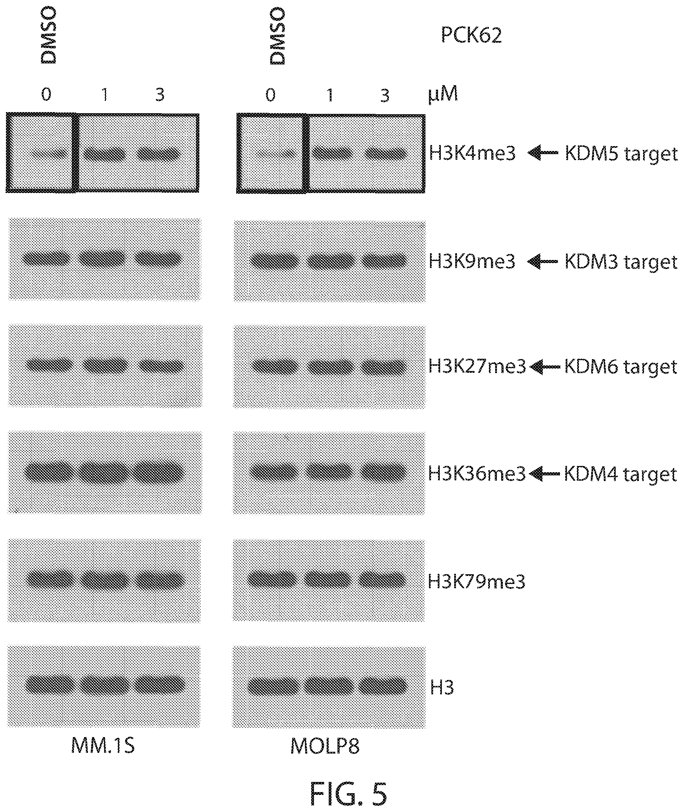

FIG. 5 shows a Western blot showing the selectivity of exemplary KDM5 inhibitor PCK62 for KDM5 in the cell. The KDM inhibitory activity of PCK62 against different KDM targets for KDM3, KDM4, KDM5, and KDM6 (H3K4me3, H3K9me3, H3K27me3, H3K36me3), H3K79me3 (positive control), and H3 (positive control) was assayed after treatment with PCK62 (at 1 µM and 3 µM) in the NM.1S (human multiple myeloma) and MOLP8 (human multiple myeloma) cell lines. H3K4me3 is a KDM5 target, H3K9me3 is a KDM3 target, H3K27me3 is a KDM6 target, and H3K36me3 is a KDM4 target. The blot shows increased selectivity of PCK62 against KDM5 target H3K4me3 in view of the increased inhibition of KDM5 activity against this KDM5 target, in contrast with the other KDM targets.

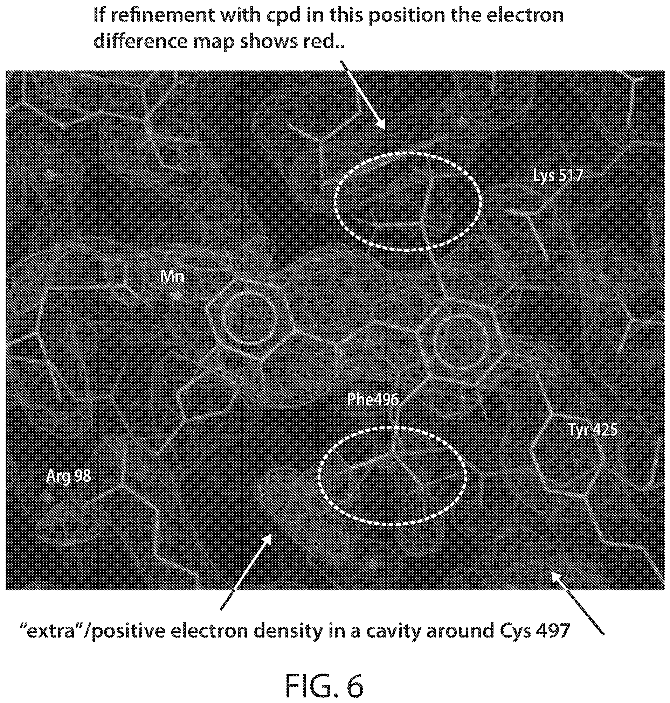

FIG. 6 shows an *in silico* model of KDM5B and its interactions with PCK62 (JADA-62) in docking studies.

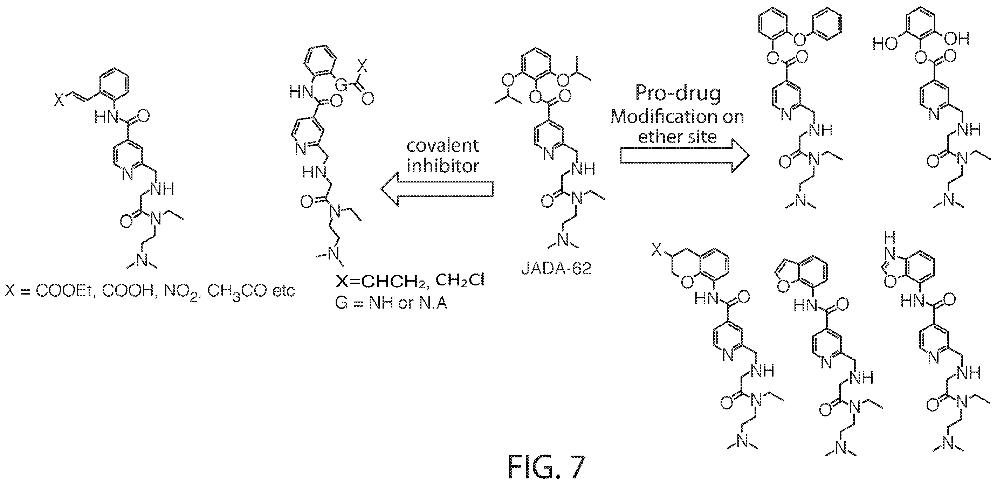

FIG. 7 shows exemplary KDM5 inhibitors.

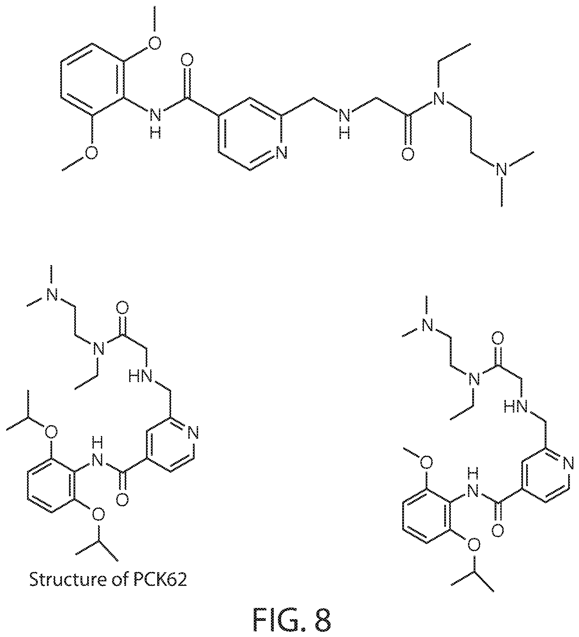

FIG. 8 shows exemplary KDM5 inhibitors.
FIGs. 9A-9B show assay data for exemplary KDM5 inhibitors PCK62 (JADA-62), JADA172, JADA173, JADA174, JADA175, Morgan1, Morgan2, Morgan3, and Morgan4, compounds in a KDM5B_alphascreen activity assay. FIGs. 9A-9B also show the structures of exemplary KDM5 inhibitors PCK62 (JADA-62), JADA172, JADA173, JADA174, JADA175, Morgan1, Morgan2, Morgan3, and Morgan4.

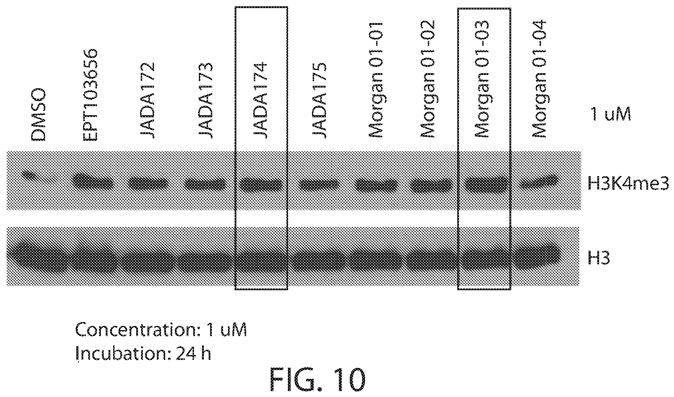

FIG. 10 shows Western Blot assay data for exemplary KDM5 inhibitors EPT103656, JADA172, JADA173, JADA174, JADA175, Morgan1, Morgan2, Morgan3, and Morgan4 (each at a 1 µM concentration) against Anti-H3K4me3 rabbit antibody (KDM5 target) and Anti-H3 rabbit antibody ("H3"; positive control), after a 24 hour incubation. Anti-H3K4me3 Rabbit Ab concentration is 1:2000 and Anti-H3 Rabbit Ab concentration is 1:8000.

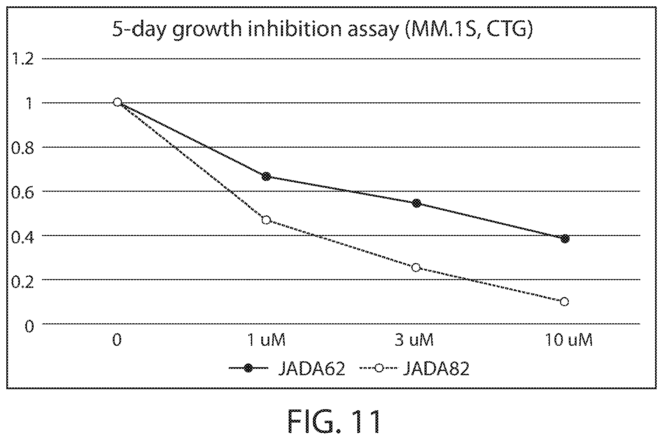

FIG. 11 shows a 5-day growth inhibition CellTiter-Glo Luminescent Cell Viability (CTG) assay upon treatment of MM.1S (human multiple myeloma) cell line, with exemplary KDM5 inhibitors JADA-62 (PCK62), and JADA82 (PCK82; of the structure: 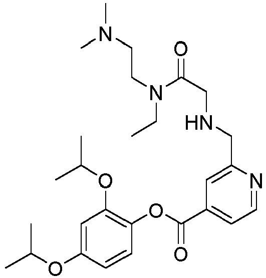 ) at the indicated concentrations.
FIG 12 shows the normalized total flux over 17 days from bioluminescence imaging of mice injected with luciferized molp8 cells. Also shown is the survival proportions of mice treated with PCK82 vs control.

### DETAILED DESCRIPTION OF CERTAIN EMBODIMENTS OF THE INVENTION

The present disclosure provides inhibitors of histone demethylases. In certain embodiments, the inhibitors are selective inhibitors. In certain embodiments, the histone demethylase is a KDM. In certain embodiments, the KDM is a KDM5. In certain embodiments, the compounds disclosed herein inhibit the activity of KDM's. In certain embodiments, the compounds disclosed herein inhibit the activity of KDM5. In certain embodiments, the compounds disclosed herein specifically inhibit the activity of KDM5. In certain embodiments, the compounds disclosed herein inhibit the activity of KDM5. In certain embodiments, the compounds disclosed herein selectively inhibit the activity of KDM5. The present disclosure further provides methods of using the compounds described herein. In certain embodiments, the histone demethylase is a KDM. In certain embodiments, the KDM is a KDM5. In certain embodiments, the use is as a therapeutic. In certain embodiments, the therapeutic use is the treatment and/or prevention of diseases associated with the overexpression and/or aberrant activity of a histone demethylase. In certain embodiments, the histone demethylase is a KDM. In certain embodiments, the KDM is KDM5. In certain embodiments, the compounds covalently inhibit KDM's. In certain embodiments, the diseases treated and/or prevented include, but are not limited to, proliferative diseases. The proliferative diseases include, but are not limited to, cancer. In certain embodiments, the cancer is lung cancer. In certain embodiments, the cancer is breast cancer. In certain embodiments, the cancer is liver cancer. In certain embodiments, the cancer is pancreatic cancer. In certain embodiments, the cancer is gastric cancer. In certain embodiments, the cancer is ovarian cancer. In certain embodiments, the cancer is colon cancer. In certain embodiments, the cancer is colorectal cancer. In certain embodiments, the cancer is a carcinoma. In certain embodiments, the cancer is multiple myeloma. In certain embodiments, the cancer is Ewing's sarcoma. In certain embodiments, the cancer is a cancer that begins in the skin or tissues lining or covering internal organs (a "carcinoma"). In certain embodiments, the carcinoma is a carcinoma of the breast, liver, lung, pancreas, stomach, colon, or prostate. In certain embodiments, the cancer is associated with the overexpression and/or aberrant activity of a histone demethylase. In certain embodiments, the histone demethylase is a KDM. In certain embodiments, the KDM is KDM5. Also provided by the present disclosure are pharmaceutical compositions, and compounds or pharmaceutical compositions as defined herein for use in treating a proliferative disease in which KDM5 plays a role. In certain embodiments, the compounds selectively inhibit a histone demethylase. In certain embodiments, the histone demethylase is a KDM. In certain embodiments, the KDM is KDM5. In certain embodiments, the compounds selectively inhibit KDM5. In certain embodiments, the compounds selectively inhibit KDM5B.

### Compounds

Certain aspects of the present disclosure relate to the compounds described herein. The compounds described herein may be useful in treating and/or preventing diseases or inhibiting the activity of a histone demethylase in a subject or biological sample. In certain embodiments, the disease is a proliferative disease. In certain embodiments, the disease is cancer. In certain embodiments, the disease is a disease associated with the activity of a histone demethylase in a subject. In certain embodiments, the histone demethylase is a KDM. In certain embodiments, the KDM is KDM5.

In one aspect, described herein is a compound of Formula **(I-B):** 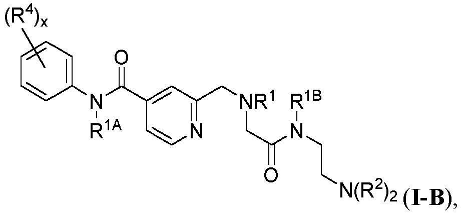 or a pharmaceutically acceptable salt, solvate, hydrate, polymorph, co-crystal, tautomer, stereoisomer, or isotopically labeled derivative thereof, wherein:
R¹ is hydrogen, optionally substituted alkyl, or a nitrogen protecting group;
R^{1A} is hydrogen, optionally substituted acyl, optionally substituted alkyl, optionally substituted carbocyclyl, or a nitrogen protecting group;
R^{1B} is hydrogen, optionally substituted alkyl, optionally substituted carbocyclyl, or a nitrogen protecting group;
each instance of R² is independently optionally substituted alkyl or a nitrogen protecting group;
each R⁴ is independently optionally substituted alkyl, optionally substituted alkenyl, optionally substituted alkynyl, optionally substituted carbocyclyl, optionally substituted heterocyclyl, optionally substituted aryl, optionally substituted heteroaryl, -OR^{A}, -N(R^{B})₂, or -SR^{A}, or optionally two instances of R⁴ are taken together with their intervening atoms to form a substituted or unsubstituted heterocyclic or substituted or unsubstituted heteroaryl ring; and
x is 0, 1, 2, 3, 4, or 5;
wherein the nitrogen protecting group is Bn, BOC, Cbz, Fmoc, trifluoroacetyl, triphenylmethyl, acetyl, or Ts.

In certain embodiments, the compound is a compound of Formula **(II):** 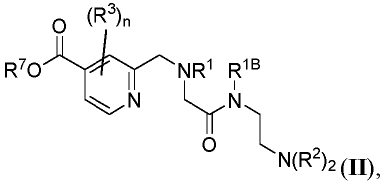 or a pharmaceutically acceptable salt, solvate, hydrate, polymorph, co-crystal, tautomer, stereoisomer, isotopically labeled derivative thereof, wherein:
R¹ is hydrogen, optionally substituted alkyl, or a nitrogen protecting group;
R^{1B} is hydrogen, methyl , optionally substituted carbocyclyl, or a nitrogen protecting group;
each instance of R² is independently optionally substituted alkyl or a nitrogen protecting group;
each instance of R³ is independently halogen, optionally substituted acyl, optionally substituted alkyl, optionally substituted alkenyl, optionally substituted alkynyl, optionally substituted carbocyclyl, optionally substituted heterocyclyl, optionally substituted aryl, optionally substituted heteroaryl, -CN, -SCN, -NO₂, -N₃, -OR^{A}, -N(R^{B})₂, or -SR^{A};
each instance of R^{A} is independently hydrogen, optionally substituted acyl, optionally substituted alkyl, optionally substituted alkenyl, optionally substituted alkynyl, optionally substituted carbocyclyl, optionally substituted heterocyclyl, optionally substituted aryl, optionally substituted heteroaryl;
each instance of R^{B} is independently hydrogen, optionally substituted acyl, optionally substituted alkyl, optionally substituted alkenyl, optionally substituted alkynyl, optionally substituted carbocyclyl, optionally substituted heterocyclyl, optionally substituted aryl, optionally substituted heteroaryl, or a nitrogen protecting group; or optionally two instances of R^{B} are taken together with their intervening atoms to form a substituted or unsubstituted heterocyclic or substituted or unsubstituted heteroaryl ring;
n is 0, 1, 2, or 3; and
R⁷ is ethyl or propyl;
wherein the nitrogen protecting group is Bn, BOC, Cbz, Fmoc, trifluoroacetyl, triphenylmethyl, acetyl, or Ts;
provided that the compound is not of the formula:

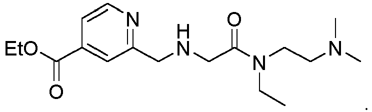

In certain embodiments, R¹ is hydrogen.

In certain embodiments, R¹ is not optionally substituted acyl. In some embodiments, R¹ is not -C(=O)*t*-Bu.

In certain embodiments, R¹ is optionally substituted alkyl.

In certain embodiments, R¹ is substituted or unsubstituted C₁₋₆ alkyl. In certain embodiments, R¹ is substituted or unsubstituted methyl. In certain embodiments, R¹ is unsubstituted methyl. In certain embodiments, R¹ is substituted or unsubstituted ethyl. In certain embodiments, R¹ is substituted or unsubstituted propyl.

In certain embodiments, R¹ is a nitrogen protecting group. In certain embodiments, R¹ is benzyl ("Bn"). In certain embodiments, R¹ is *tert-*butyl carbonate ("BOC" or "Boc"). In certain embodiments, R¹ is benzyl carbamate ("Cbz"). In certain embodiments, R¹ is 9-fluorenylmethyl carbonate ("Fmoc"). In certain embodiments, R¹ is trifluoroacetyl. In certain embodiments, R¹ is triphenylmethyl. In certain embodiments, R¹ is acetyl. In certain embodiments, R¹ is p-toluenesulfonamide ("Ts").

In certain embodiments, R^{1B} is hydrogen.

In certain embodiments, R^{1B} is optionally substituted carbocyclyl. In certain embodiments, R^{1B} is substituted, 3- to 7-membered, monocyclic carbocyclyl comprising zero, one, or two double bonds in the carbocyclic ring system. In certain embodiments, R^{1B} is optionally substituted cyclopropyl. In certain embodiments, R^{1B} is unsubstituted cyclopropyl.

In certain embodiments, R^{1B} is a nitrogen protecting group. In certain embodiments, the nitrogen protecting group is Bn, BOC, Cbz, Fmoc, trifluoroacetyl, triphenylmethyl, acetyl, or Ts.

In certain embodiments, both instances of R² are the same. In certain embodiments, each instance of R² is different.

In certain embodiments, at least one instance of R² is optionally substituted alkyl. In some embodiments, at least one instance of R² is substituted or unsubstituted C₁₋₆ alkyl. In certain embodiments, at least one instance of R² is optionally substituted C₁₋₆ alkyl. In certain embodiments, at least one instance of R² is substituted or unsubstituted methyl. In certain embodiments, at least one instance of R² is unsubstituted methyl. In certain embodiments, both instances of R² are substituted or unsubstituted methyl. In certain embodiments, both instances of R² are unsubstituted methyl. In certain embodiments, at least once instance of R² is substituted or unsubstituted ethyl. In certain embodiments, at least one instance of R² is not substituted or unsubstituted ethyl. In certain embodiments, both instances of R² are not ethyl. In certain embodiments, at least one instance of R² is substituted or unsubstituted propyl. In certain embodiments, at least one instance of R² is unsubstituted n-propyl. In certain embodiments, at least one instance of R² is unsubstituted methyl or isopropyl.

In certain embodiments, each R² is independently optionally substituted alkyl or a nitrogen protecting group. In certain embodiments, each R² is independently optionally substituted alkyl or a nitrogen protecting group. In certain embodiments, R² is Bn, BOC, Cbz, Fmoc, trifluoroacetyl, triphenylmethyl, acetyl, or Ts. In certain embodiments, at least one instance of R² is a nitrogen protecting group. In some embodiments, the nitrogen protecting group is Bn, BOC, Cbz, Fmoc, trifluoroacetyl, triphenylmethyl, acetyl, or Ts.

In certain embodiments, the compound includes no instances of substituent R³. In certain embodiments, the compound includes one or more instances of substituent R³.

In certain embodiments, n is 0. In certain embodiments, n is 1. In certain embodiments, n is 2. In certain embodiments, n is 3.

In certain embodiments, at least one instance of R³ is a halogen. In certain embodiments, at least one instance of R³ is F. In certain embodiments, at least one instance of R³ is Cl. In certain embodiments, at least one instance of R³ is Br. In certain embodiments, at least one instance of R³ is I.

In certain embodiments, at least one instance of R³ is optionally substituted acyl. In certain embodiments, the acyl is -C(=O)Me.

In certain embodiments, at least one instance of R³ is optionally substituted alkyl. In certain embodiments, the alkyl is substituted or unsubstituted C₁₋₆ alkyl. In certain embodiments, at least one instance of R³ is substituted or unsubstituted methyl. In certain embodiments, at least one instance of R³ is substituted or unsubstituted ethyl. In certain embodiments, at least one instance of R³ is substituted or unsubstituted propyl.

In certain embodiments, at least one instance of R³ is optionally substituted alkenyl. In certain embodiments, the alkenyl is substituted or unsubstituted C₂₋₆ alkenyl.

In certain embodiments, at least one instance of R³ is optionally substituted alkynyl. In certain embodiments, the alkynyl is substituted or unsubstituted C₂₋₆ alkynyl.

In certain embodiments, at least one instance of R³ is optionally substituted carbocyclyl. In certain embodiments, the carbocyclyl is substituted or unsubstituted, 3- to 7-membered, monocyclic carbocyclyl comprising zero, one, or two double bonds in the carbocyclic ring system. In certain embodiments, at least one instance of R³ is optionally substituted heterocyclyl. In certain embodiments, the heterocyclyl is substituted or unsubstituted, 5- to 10-membered monocyclic or bicyclic heterocyclic ring, wherein one or two atoms in the heterocyclic ring are independently nitrogen, oxygen, or sulfur.

In certain embodiments, at least one instance of R³ is optionally substituted aryl. In certain embodiments, the aryl is substituted or unsubstituted, 6- to 10-membered aryl. In certain embodiments, at least one instance of R³ is benzyl. In certain embodiments, at least one instance of R³ is substituted or unsubstituted phenyl. In certain embodiments, at least one instance of R³ is optionally substituted heteroaryl. In certain embodiments, at least one instance of R³ is substituted or unsubstituted, 5- to 6-membered, monocyclic heteroaryl, wherein one, two, three, or four atoms in the heteroaryl ring system are independently nitrogen, oxygen, or sulfur; or substituted or unsubstituted, 9- to 10-membered, bicyclic heteroaryl, wherein one, two, three, or four atoms in the heteroaryl ring system are independently nitrogen, oxygen, or sulfur.

In certain embodiments, at least one instance of R³ is -OR^{A}. In certain embodiments, R^{A} is -OH. In certain embodiments, R^{A} is -OMe. In certain embodiments, at least one instance of R³ is -N(R^{B})₂. In certain embodiments, at least one instance of R³ is -NMe₂. In certain embodiments, at least one instance of R³ is -SR^{A}. In certain embodiments, at least one instance of R³ is -SMe.

In certain embodiments, at least one instance of R³ is-CN.

In certain embodiments, at least one instance of R³ is -SCN.

In certain embodiments, at least one instance of R³ is -NO₂.

In certain embodiments, at least one instance of R³ is -N₃.

In certain embodiments, at least one instance of R³ is -OR^{A}, wherein each instance of R^{A} is independently hydrogen, optionally substituted acyl, optionally substituted alkyl, optionally substituted alkenyl, optionally substituted alkynyl, optionally substituted carbocyclyl, optionally substituted heterocyclyl, optionally substituted aryl, optionally substituted heteroaryl, an oxygen protecting group when attached to an oxygen atom, or a sulfur protecting group when attached to a sulfur atom.

In certain embodiments, at least one instance of R³ is N(R^{B})₂, wherein each instance of R^{B} is independently hydrogen, optionally substituted acyl, optionally substituted alkyl, optionally substituted alkenyl, optionally substituted alkynyl, optionally substituted carbocyclyl, optionally substituted heterocyclyl, optionally substituted aryl, optionally substituted heteroaryl, or a nitrogen protecting group; or optionally two instances of R^{B} are taken together with their intervening atoms to form a substituted or unsubstituted heterocyclic or substituted or unsubstituted heteroaryl ring.

In certain embodiments, R^{A} is hydrogen.

In certain embodiments, R^{A} is substituted or unsubstituted acyl In certain embodiments, R^{A} is -C(=O)Me).

In certain embodiments, R^{A} is substituted or unsubstituted alkyl. In certain embodiments, R^{A} is substituted or unsubstituted C₁₋₆ alkyl. In certain embodiments, R^{A} is substituted or unsubstituted methyl. In certain embodiments, R^{A} is substituted or unsubstituted ethyl. In certain embodiments, R^{A} is substituted or unsubstituted propyl.

In certain embodiments, R^{A} is substituted or unsubstituted alkenyl. In certain embodiments, R^{A} is substituted or unsubstituted C₂₋₆ alkenyl.

In certain embodiments, R^{A} is substituted or unsubstituted alkynyl. In certain embodiments, R^{A} is substituted or unsubstituted C₂₋₆ alkynyl.

In certain embodiments, R^{A} is substituted or unsubstituted carbocyclyl. In certain embodiments, R^{A} is substituted or unsubstituted, 3- to 7-membered, monocyclic carbocyclyl comprising zero, one, or two double bonds in the carbocyclic ring system.

In certain embodiments, R^{A} is substituted or unsubstituted heterocyclyl. In certain embodiments, R^{A} is substituted or unsubstituted, 5- to 10-membered monocyclic or bicyclic heterocyclic ring, wherein one or two atoms in the heterocyclic ring are independently nitrogen, oxygen, or sulfur.

In certain embodiments, R^{A} is substituted or unsubstituted aryl. In certain embodiments, R^{A} is substituted or unsubstituted, 6- to 10-membered aryl. In certain embodiments, R^{A} is benzyl. In certain embodiments, R^{A} is substituted or unsubstituted phenyl.

In certain embodiments, R^{A} is substituted or unsubstituted heteroaryl. In certain embodiments, R^{A} is substituted or unsubstituted, 5- to 6-membered, monocyclic heteroaryl, wherein one, two, three, or four atoms in the heteroaryl ring system are independently nitrogen, oxygen, or sulfur; or substituted or unsubstituted, 9- to 10-membered, bicyclic heteroaryl, wherein one, two, three, or four atoms in the heteroaryl ring system are independently nitrogen, oxygen, or sulfur.

In certain embodiments, at least one instance of R^{B} is hydrogen.

In certain embodiments, at least one instance of R^{B} is substituted or unsubstituted acyl In certain embodiments, at least one instance of R^{B} is substituted or unsubstituted -C(=O)Me).

In certain embodiments, at least one R^{B} is substituted or unsubstituted alkyl. In certain embodiments, at least one R^{B} is substituted or unsubstituted C₁₋₆ alkyl. In certain embodiments, at least one instance of R^{B} is substituted or unsubstituted methyl. In certain embodiments, at least one instance of R^{B} is substituted or unsubstituted ethyl. In certain embodiments, at least one instance of R^{B} is substituted or unsubstituted propyl.

In certain embodiments, at least one instance of R^{B} is substituted or unsubstituted alkenyl. In certain embodiments, at least one instance of R^{B} is substituted or unsubstituted C₂₋₆ alkenyl).

In certain embodiments, at least one instance of R^{B} is substituted or unsubstituted alkynyl. In certain embodiments, at least one instance of R^{B} is substituted or unsubstituted C₂₋₆ alkynyl.

In certain embodiments, at least one instance of R^{B} is substituted or unsubstituted carbocyclyl. In certain embodiments, at least one instance of R^{B} is substituted or unsubstituted, 3- to 7-membered. In certain embodiments, at least one instance of R^{B} is monocyclic carbocyclyl comprising zero, one, or two double bonds in the carbocyclic ring system.

In certain embodiments, at least one instance of R^{B} is substituted or unsubstituted heterocyclyl. In certain embodiments, at least one instance of R^{B} is substituted or unsubstituted, 5- to 10-membered monocyclic or bicyclic heterocyclic ring, wherein one or two atoms in the heterocyclic ring are independently nitrogen, oxygen, or sulfur.

In certain embodiments, at least one instance of R^{B} is substituted or unsubstituted aryl. In certain embodiments, at least one instance of R^{B} substituted or unsubstituted, 6- to 10-membered aryl. In certain embodiments, at least one instance of R^{B} is benzyl. In certain embodiments, at least one instance of R^{B} is substituted or unsubstituted phenyl.

In certain embodiments, at least one instance of R^{B} is substituted or unsubstituted heteroaryl. In certain embodiments, at least one instance of R^{B} is substituted or unsubstituted, 5-to 6-membered. In certain embodiments, at least one instance of R^{B} is monocyclic heteroaryl, wherein one, two, three, or four atoms in the heteroaryl ring system are independently nitrogen, oxygen, or sulfur. In certain embodiments, at least one instance of R^{B} is substituted or unsubstituted, 9- to 10-membered, bicyclic heteroaryl, wherein one, two, three, or four atoms in the heteroaryl ring system are independently nitrogen, oxygen, or sulfur.

In certain embodiments, at least one instance of R^{B} is a nitrogen protecting group. In certain embodiments, at least one instance of R^{B} is Bn. In certain embodiments, at least one instance of R^{B} is BOC. In certain embodiments, at least one instance of R^{B} is Cbz. In certain embodiments, at least one instance of R^{B} is Fmoc. In certain embodiments, at least one instance of R^{B} is trifluoroacetyl. In certain embodiments, at least one instance of R^{B} is triphenylmethyl. In certain embodiments, at least one instance of R^{B} is acetyl. In certain embodiments, at least one instance of R^{B} is Ts. In certain embodiments, two instances of R^{B} are taken together with their intervening atoms to form a substituted or unsubstituted heterocyclic ring. In certain embodiments, two instances of R^{B} are substituted or unsubstituted, 5- to 10-membered monocyclic or bicyclic heterocyclic ring, wherein one or two atoms in the heterocyclic ring are independently nitrogen, oxygen, or sulfur.

In certain embodiments, at least one instance of R^{B} is substituted or unsubstituted heteroaryl ring. In certain embodiments, at least one instance of R^{B} is substituted or unsubstituted, 5- to 6-membered. In certain embodiments, at least one instance of R^{B} is monocyclic heteroaryl, wherein one, two, three, or four atoms in the heteroaryl ring system are independently nitrogen, oxygen, or sulfur. In certain embodiments, at least one instance of R^{B} is substituted or unsubstituted, 9- to 10-membered, bicyclic heteroaryl, wherein one, two, three, or four atoms in the heteroaryl ring system are independently nitrogen, oxygen, or sulfur.

In certain embodiments, R^{1A} is hydrogen.

In certain embodiments, R^{1A} is substituted or unsubstituted acyl. In certain embodiments, R^{1A} is -C(=O)Me.

In certain embodiments, R^{1A} is substituted or unsubstituted alkyl. In certain embodiments, R^{1A} is substituted or unsubstituted C₁₋₆ alkyl. In certain embodiments, R^{1A} is substituted or unsubstituted methyl. In certain embodiments, R^{1A} is substituted or unsubstituted ethyl. In certain embodiments, R^{1A} is substituted or unsubstituted propyl.

In certain embodiments, R^{1A} is substituted or unsubstituted alkenyl. In certain embodiments, R^{1A} is substituted or unsubstituted C₂₋₆ alkenyl.

In certain embodiments, R^{1A} is substituted or unsubstituted carbocyclyl. In certain embodiments, R^{1A} is substituted or unsubstituted, 3- to 7-membered, monocyclic carbocyclyl comprising zero, one, or two double bonds in the carbocyclic ring system.

In certain embodiments, R^{1A} is a nitrogen protecting group. In certain embodiments, R^{1A} is Bn. In certain embodiments, R^{1A} is BOC. In certain embodiments, R^{1A} is Cbz. In certain embodiments, R^{1A} is Fmoc. In certain embodiments, R^{1A} is trifluoroacetyl. In certain embodiments, R^{1A} is triphenylmethyl. In certain embodiments, R^{1A} is acetyl. In certain embodiments, R^{1A} is Ts.

In certain embodiments, at least one instance of R⁴ is optionally substituted alkyl. In certain embodiments, at least one instance of R⁴ is substituted or unsubstituted C₁₋₆ alkyl. In certain embodiments, at least one instance of R⁴ is substituted or unsubstituted C₁₋₆ alkyl. In certain embodiments, at least one instance of R⁴ is substituted or unsubstituted methyl. In certain embodiments, at least one instance of R⁴ is substituted or unsubstituted ethyl. In certain embodiments, at least one instance of R⁴ is substituted or unsubstituted propyl. In certain embodiments, R⁴ is not substituted or unsubstituted *tert*-butyl.

In certain embodiments, at least one instance of R⁴ is optionally substituted alkynyl. In certain embodiments, at least one instance of R⁴ is substituted or unsubstituted C₂₋₆ alkynyl.

In certain embodiments, at least one instance of R⁴ is optionally substituted carbocyclyl. In certain embodiments, at least one instance of R⁴ is substituted or unsubstituted, 3- to 7-membered, monocyclic carbocyclyl comprising zero, one, or two double bonds in the carbocyclic ring system.

In certain embodiments, at least one instance of R⁴ is optionally substituted heterocyclyl. In certain embodiments, at least one instance of R⁴ is substituted or unsubstituted, 5- to 10-membered monocyclic or bicyclic heterocyclic ring, wherein one or two atoms in the heterocyclic ring are independently nitrogen, oxygen, or sulfur.

In certain embodiments, at least one instance of R⁴ is optionally substituted aryl. In certain embodiments, at least one instance of R⁴ is substituted or unsubstituted, 6- to 10-membered aryl. In certain embodiments, at least one instance of R⁴ is benzyl. In certain embodiments, at least one instance of R⁴ is substituted or unsubstituted phenyl.

In certain embodiments, at least one instance of R⁴ is optionally substituted heteroaryl. In certain embodiments, at least one instance of R⁴ is substituted or unsubstituted, 5- to 6-membered, monocyclic heteroaryl, wherein one, two, three, or four atoms in the heteroaryl ring system are independently nitrogen, oxygen, or sulfur; or substituted. In certain embodiments, at least one instance of R⁴ is unsubstituted, 9- to 10-membered, bicyclic heteroaryl, wherein one, two, three, or four atoms in the heteroaryl ring system are independently nitrogen, oxygen, or sulfur.

In certain embodiments, at least one instance of R⁴ is -OR^{A}. In certain embodiments, at least one instance of R⁴ is -OH. In certain embodiments, at least one instance of R⁴ is -OMe. In certain embodiments, at least one instance of R⁴ is -OiPr. In certain embodiments, at least one instance of R⁴ is -OR^{A}, and R^{A} is hydrogen, optionally substituted C₁₋₆ alkyl, or optionally substituted aryl. In certain embodiments, at least one instance of R⁴ is -OH. In certain embodiments, two instances of R⁴ are -OH.

In certain embodiments, at least one instance of R⁴ is -O(optionally substituted C₁₋₆ alkyl). In certain embodiments, at least one instance of R⁴ is -OMe. In certain embodiments, two instances of R⁴ are -OMe. In certain embodiments, R⁴ is not -OMe. In certain embodiments, at least one instance of R⁴ is -OEt. In certain embodiments, two instances of R⁴ are -OEt. In certain embodiments, at least one instance of R⁴ is -O(n-propyl). In certain embodiments, two instances of R⁴ are -O(n-propyl). In certain embodiments, at least one instance of R⁴ is -O(isopropyl). In certain embodiments, two instances of R⁴ are -O(isopropyl). In certain embodiments, at least one instance of R⁴ is -O(n-butyl). In certain embodiments, two instances of R⁴ are -O(n-butyl). In certain embodiments, at least one instance of R⁴ is -O(t-butyl). In certain embodiments, two instances of R⁴ are -O(*tert-*butyl)*.* In certain embodiments, at least one instance of R⁴ is *-*O(*n-*pentyl). In certain embodiments, two instances of R⁴ are -O(*n*-pentyl).

In certain embodiments, at least one instance of R⁴ is -O(optionally substituted aryl). In certain embodiments, at least one instance of R⁴ is -O(optionally substituted phenyl). In certain embodiments, at least one instance of R⁴ is -O(unsubstituted phenyl). In certain embodiments, two instances of R⁴ are -O(optionally substituted phenyl). In certain embodiments, two instances of R⁴ are -O(unsubstituted phenyl).

In certain embodiments, at least one instance of R⁴ is -N(R^{B})₂. In certain embodiments, at least one instance of R⁴ is -NMe₂. In certain embodiments, at least one instance of R⁴ is - NH(optionally substituted alkyl). In certain embodiments, at least one instance of R⁴ is - NH(optionally substituted acyl).

In certain embodiments, at least one instance of R⁴ is -NHC(=O)R^{x}, and R^{x} is optionally substituted C₁₋₆ alkyl or optionally substituted alkenyl. In certain embodiments, at least one instance of R⁴ is -NHC(=O)(optionally substituted C₁₋₆ alkyl). In certain embodiments, at least one instance of R⁴ is -NHC(=O)(C₁₋₆ alkyl optionally substituted with halogen). In certain embodiments, at least one instance of R⁴ is -NHC(=O)(CH₂Cl). In certain embodiments, at least one instance of R⁴ is -NHC(=O)(optionally substituted C₂₋₆ alkenyl). In certain embodiments, at least one instance of R⁴ is -NHC(=O)(CHCH₂).

In certain embodiments, at least one instance of R⁴ is -SR^{A}. In certain embodiments, at least one instance of R⁴ is -SMe.

In certain embodiments, two instances of R⁴ are taken together with their intervening atoms to form a substituted or unsubstituted heterocyclic ring. In certain embodiments, two instances of R⁴ are taken together with their intervening atoms to form a substituted or unsubstituted, 5- to 10-membered monocyclic or bicyclic heterocyclic ring, wherein one or two atoms in the heterocyclic ring are independently nitrogen, oxygen, or sulfur.

In certain embodiments, two instances of R⁴ are taken together with their intervening atoms to form a substituted or unsubstituted heteroaryl ring. In certain embodiments, two instances of R⁴ are taken together with their intervening atoms to form a substituted or unsubstituted, 5- to 6-membered, monocyclic heteroaryl, wherein one, two, three, or four atoms in the heteroaryl ring system are independently nitrogen, oxygen, or sulfur. In certain embodiments, two instances of R⁴ are taken together with their intervening atoms to form a substituted or unsubstituted, 9- to 10-membered, bicyclic heteroaryl, wherein one, two, three, or four atoms in the heteroaryl ring system are independently nitrogen, oxygen, or sulfur.

In certain embodiments, y is 0. In certain embodiments, y is 1. In certain embodiments, y is 2. In certain embodiments, y is 3. In certain embodiments, y is 4. In certain embodiments, y is 5. In certain embodiments, y is 6. In certain embodiments, y is 7. In certain embodiments, y is 8. In certain embodiments, y is 9.

In certain embodiments, at least one instance of R^{x} is optionally substituted acyl. In certain embodiments, at least one instance of R^{x} is -C(=O)Me.

In certain embodiments, at least one instance of R^{x} is optionally substituted alkyl. In certain embodiments, at least one instance of R^{x} is substituted or unsubstituted C₁₋₆ alkyl. In certain embodiments, at least one instance of R^{x} is substituted or unsubstituted methyl. In certain embodiments, at least one instance of R^{x} is substituted or unsubstituted ethyl. In certain embodiments, at least one instance of R^{x} is unsubstituted ethyl. In certain embodiments, at least one instance of R^{x} is substituted or unsubstituted propyl. In certain embodiments, at least one instance of R^{x} is unsubstituted n-propyl. In certain embodiments, at least one instance of R^{x} is unsubstituted methyl or isopropyl.

In certain embodiments, at least one instance of R^{x} is -O(optionally substituted alkyl). In certain embodiments, at least one instance of R^{x} is -O(optionally substituted C₁₋₆ alkyl).

In certain embodiments, at least one instance of R^{x} is -NO₂.

In certain embodiments, the compound of Formula **(I-B)** is a compound of the formula: 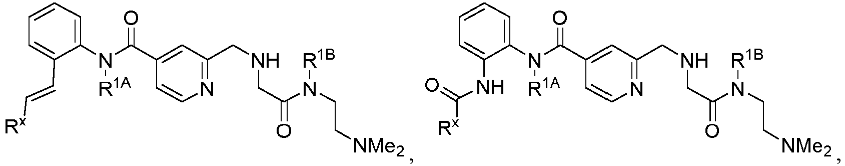 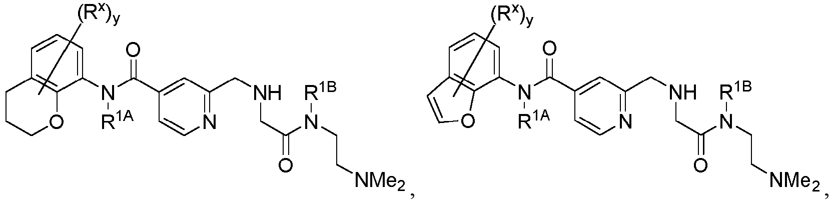 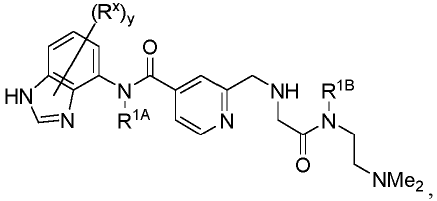 or a pharmaceutically acceptable salt, solvate, hydrate, polymorph, co-crystal, tautomer, stereoisomer, or isotopically labeled derivative thereof.

In certain embodiments, the compound of Formula **(I-B)** is a compound of the formula: 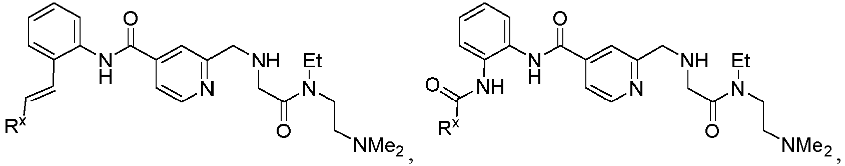 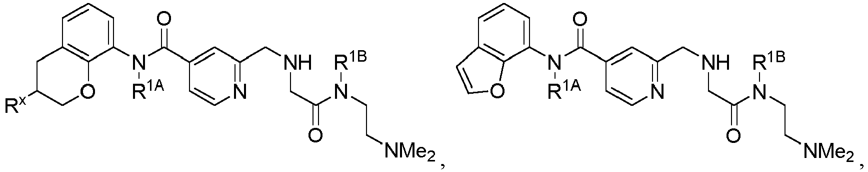 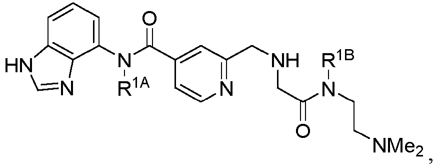 or a pharmaceutically acceptable salt, solvate, hydrate, polymorph, co-crystal, tautomer, stereoisomer, or isotopically labeled derivative thereof.

In certain embodiments, the compound of Formula **(I-B)** is a compound of the formula: 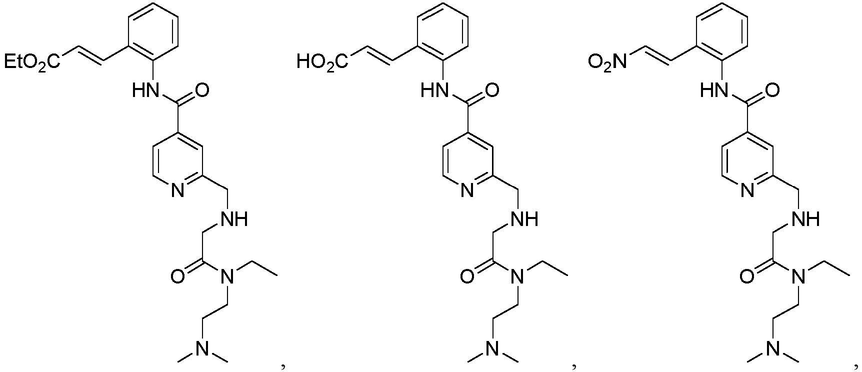 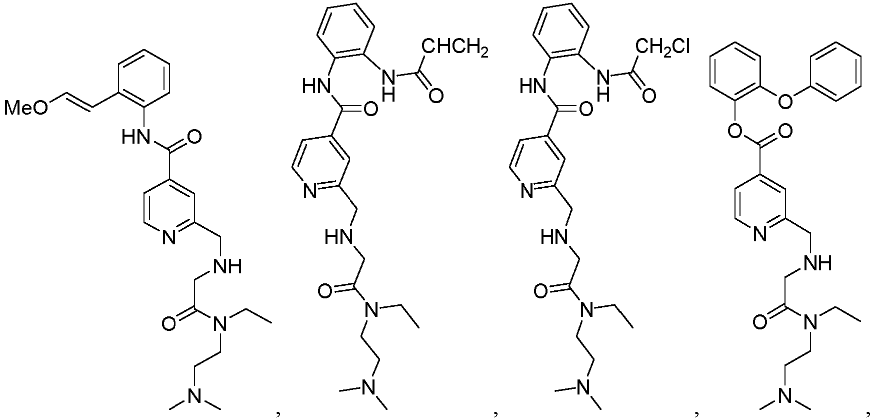 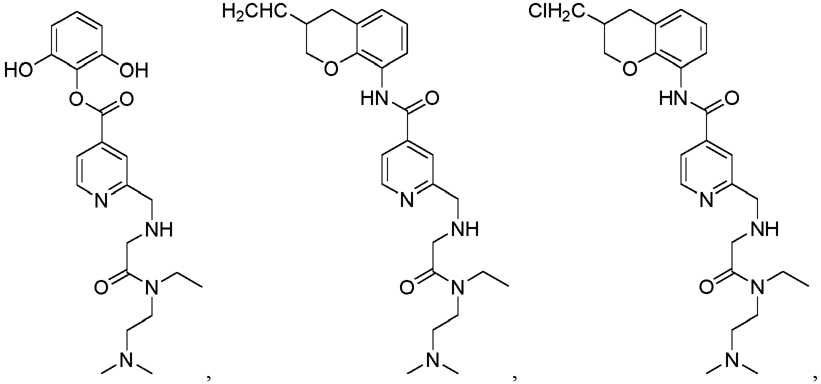 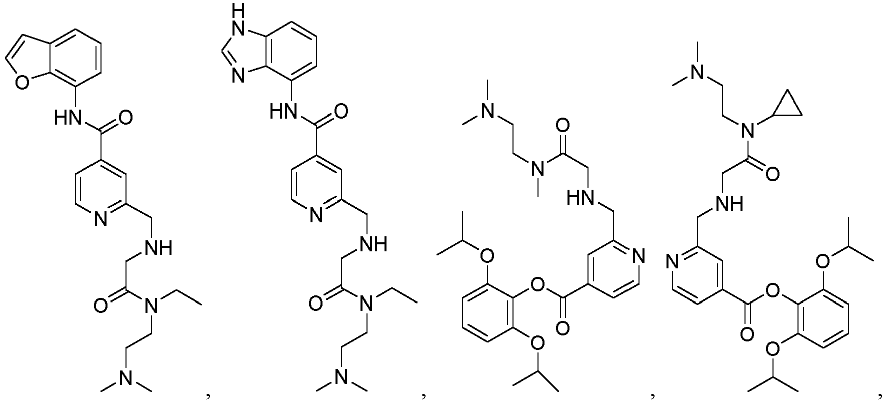 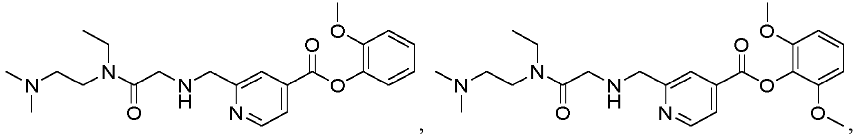 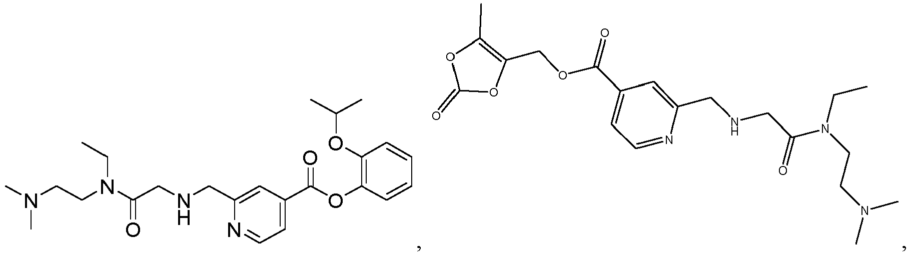 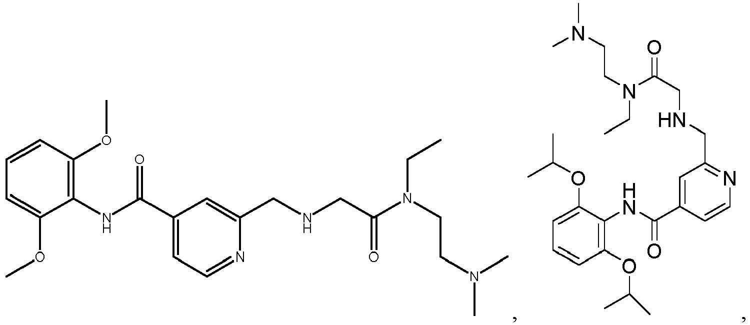 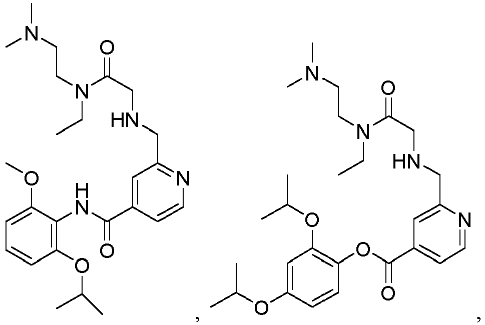 or a pharmaceutically acceptable salt, solvate, hydrate, polymorph, co-crystal, tautomer, stereoisomer, or isotopically labeled derivative thereof.

In certain embodiments, R⁷ is ethyl. In certain embodiments, R⁷ is propyl.

In certain embodiments, the compound of Formula (**II**) is a compound of the formula: 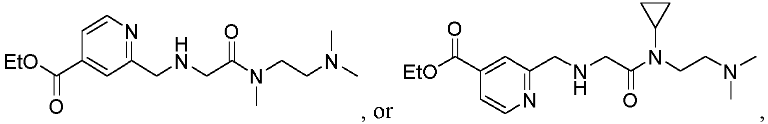 or a pharmaceutically acceptable salt, solvate, hydrate, polymorph, co-crystal, tautomer, stereoisomer, or isotopically labeled derivative thereof.

In certain embodiments, the compound of Formulae **(I-B)** or (**II**) is a compound provided in any one of the Examples below. In certain embodiments, a compound described herein is a compound of Formulae **(I-B), (II),** or a pharmaceutically acceptable salt, solvate, hydrate, polymorph, co-crystal, tautomer, stereoisomer, or isotopically labeled derivative thereof as described herein.

Certain compounds described herein bind, covalently modify, and/or inhibit a histone demethylase. In certain embodiments, the compounds described herein irreversibly inhibit a KDM histone demethylase. In certain embodiments, the compounds described herein reversibly inhibit a KDM histone demethylase. In certain embodiments, the histone demethylase is a KDM. In certain embodiments, the histone demethylase is KDM5. In certain embodiments, the histone demethylase is KDM3. In certain embodiments, the compounds described herein covalently bind to the histone demethylase. In certain embodiments, the compounds described herein reversibly bind to the histone demethylase. In certain embodiments, the compounds described herein non-reversibly bind to the histone demethylase. In certain embodiments, the compounds described herein modulate the activity of a histone demethylase. In certain embodiments, the compounds described herein inhibit the activity of a histone demethylase. In certain embodiments, the compounds described herein specifically inhibit the activity of a histone demethylase. In certain embodiments, the compounds described herein selectively inhibit the activity of a histone demethylase over another histone demethylase for example, but not limited to KDM2/7, KDM3, KDM4, or KDM6. In certain embodiments, the histone demethylase is a KDM. In certain embodiments, the KDM is KDM5. In certain embodiments, the KDM is another KDM. In certain embodiments, the KDM is KDM2/7. In certain embodiments, the KDM is KDM3. In certain embodiments, the KDM is KDM 4. In certain embodiments, the KDM is KDM 6.

The binding affinity of a compound described herein to a histone demethylase may be measured by the dissociation constant (*K*_{d}) value of an adduct of the compound and the histone demethylase using methods known in the art. In certain embodiments the binding affinity is hound with isothermal titration calorimetry (ITC)). In certain embodiments, the histone demethylase is a KDM. In certain embodiments, the KDM is KDM5. In certain embodiments, the *K*_{d} value of the adduct is not more than about 100 µM, not more than about 10 µM, not more than about 1 µM, not more than about 100 nM, not more than about 10 nM, or not more than about 1 nM.

In certain embodiments, the activity of a histone demethylase is inhibited by a compound described herein. The inhibition of the activity of a histone demethylase by a compound described herein may be measured by determining the half maximal inhibitory concentration (IC₅₀) of the compound when the compound, or a pharmaceutical composition thereof, is contacted with the histone demethylase. In certain embodiments, the histone demethylase is a KDM. In certain embodiments, the KDM is KDM5. The IC₅₀ values may be obtained using methods known in the art. in certain embodiments, the IC₅₀ values are obtained using a competition binding assay. In certain embodiments, the IC₅₀ values are obtained using a alphascreen activity assay. In certain embodiments, the IC₅₀ value of a compound described herein is not more than about 1 mM, not more than about 100 µM, not more than about 10 µM, not more than about 1 µM, not more than about 100 nM, not more than about 10 nM, or not more than about 1 nM.

The compounds described herein may selectively modulate the activity of a histone demethylase. In certain embodiments, the compounds selectively inhibit the activity of a histone demethylase. In certain embodiments, the compounds inhibit the activity of two or more histone demethylases to the same extent. In certain embodiments, the histone demethylase is a KDM. In certain embodiments, the KDM is KDM5.

The selectivity of a compound described herein in inhibiting the activity of a first histone demethylase over a second histone demethylase may be measured by the quotient of the IC₅₀ value of the compound in inhibiting the activity of the second histone demethylase over the IC₅₀ value of the compound in inhibiting the activity of the first histone demethylase. The selectivity of a compound described herein in modulating the activity of a first histone demethylase over a second histone demethylase may also be measured by the quotient of the *K*_{d} value of an adduct of the compound and the second histone demethylase over the *K*_{d} value of an adduct of the compound and the first histone demethylase. In certain embodiments, the histone demethylase is a KDM. In certain embodiments, the KDM is KDM5. In certain embodiments, the selectivity is at least about 1-fold, at least about 2-fold, at least about 3-fold, at least about 5-fold, at least about 10-fold, at least about 30-fold, at least about 50-fold, at least about 100-fold, at least about 300-fold, at least about 500-fold, at least about 1,000-fold, at least about 3,000-fold, at least about 5,000-fold, at least about 10,000-fold, at least about 30,000-fold, at least about 50,000-fold, or at least about 100,000-fold.

It is expected that the compounds described herein may be useful in treating and/or preventing diseases associated with aberrant activity of a histone demethylase. In certain embodiments, the aberrant activity is increased activity. In certain embodiments, the aberrant activity is undesired activity. In certain embodiments, the aberrant activity is abnormal activity. In certain embodiments, the histone demethylase is a KDM. In certain embodiments, the KDM is KDM5. It is known in the art that histone demethylases are implicated in a wide range of diseases and conditions, such as proliferative diseases. Therefore, the compounds described herein are expected to be useful in treating and/or preventing diseases. In certain embodiments, the disease are proliferative diseases.

### Pharmaceutical Compositions, and Administration

The present disclosure also provides pharmaceutical compositions comprising a compound described herein and optionally a pharmaceutically acceptable excipient. In certain embodiments, a compound described herein is a compound of Formulae **(I-B),** (**II**), or a pharmaceutically acceptable salt thereof, and a pharmaceutically acceptable excipient, as described herein.

In certain embodiments, the compound described herein is provided in an effective amount in the pharmaceutical composition. In certain embodiments, the effective amount is a therapeutically effective amount. In certain embodiments, the effective amount is a prophylactically effective amount. In certain embodiments, a therapeutically effective amount is an amount effective for inhibiting the aberrant activity of a histone demethylase. In certain embodiments, a therapeutically effective amount is an amount effective for inhibiting the aberrant activity of KDM5 and treating a disease. In certain embodiments, a prophylactically effective amount is an amount effective for inhibiting the aberrant activity of a histone demethylase. In certain embodiments, a prophylactically effective amount is an amount effective for preventing or keeping a subject in need thereof in remission of a disease. In certain embodiments, a prophylactically effective amount is an amount effective for inhibiting the aberrant activity of KDM5, and preventing or keeping a subject in need thereof in remission of a disease. In certain embodiments, the histone demethylase is a KDM. In certain embodiments, the KDM is KDM5. In certain embodiments, a therapeutically effective amount is an amount effective for treating a disease. In certain embodiments, the disease is a disease associated with aberrant activity of KDM5. In certain embodiments, the disease is a proliferative disease. In certain embodiments, the disease is a cardiovascular disease.

In certain embodiments, the effective amount is an amount effective for inhibiting the activity of a histone demethylase by at least 10%, at least 20%, at least 30%, at least 40%, at least 50%, at least 60%, at least 70%, at least 80%, at least 90%, at least 95%, or at least 98%. In certain embodiments, the effective amount is an amount effective for inhibiting the activity of a histone demethylase by not more than 10%, not more than 20%, not more than 30%, not more than 40%, not more than 50%, not more than 60%, not more than 70%, not more than 80%, not more than 90%, not more than 95%, or not more than 98%. In certain embodiments, the effective amount is an amount effective for inhibiting the activity of a KDM by not more than 10%, not more than 20%, not more than 30%, not more than 40%, not more than 50%, not more than 60%, not more than 70%, not more than 80%, not more than 90%, not more than 95%, or not more than 98%. In certain embodiments, the histone demethylase is a KDM. In certain embodiments, the KDM is KDM5.

In certain embodiments, the subject is an animal. The animal may be of either sex and may be at any stage of development. In certain embodiments, the subject described herein is a human. In certain embodiments, the subject is a non-human animal. In certain embodiments, the subject is a mammal. In certain embodiments, the subject is a non-human mammal. In certain embodiments, the subject is a domesticated animal, such as a dog, cat, cow, pig, horse, sheep, or goat. In certain embodiments, the subject is a companion animal, such as a dog or cat. In certain embodiments, the subject is a livestock animal, such as a cow, pig, horse, sheep, or goat. In certain embodiments, the subject is a zoo animal. In another embodiment, the subject is a research animal, such as a rodent, dog, pig, or non-human primate. In certain embodiments, the rodent is a mouse. In certain embodiments, the rodent is a rat. In certain embodiments, the animal is a genetically engineered animal. In certain embodiments, the animal is a transgenic animal. In certain embodiments, the transgenic animal is a transgenic mouse. In certain embodiments, the transgenic animal is a transgenic pig. In certain embodiments, the subject is a fish or reptile.

In certain embodiments, the cell being contacted with a compound or composition described herein is *in vitro.* In certain embodiments, the cell being contacted with a compound or composition described herein is *in vivo.*

Pharmaceutical compositions described herein can be prepared by any method known in the art of pharmacology. In general, such preparatory methods include bringing the compound described herein (*i.e.,* the "active ingredient") into association with a carrier or excipient, and/or one or more other accessory ingredients, and then, if necessary and/or desirable, shaping, and/or packaging the product into a desired single- or multi-dose unit.

Pharmaceutical compositions can be prepared, packaged, and/or sold in bulk, as a single unit dose, and/or as a plurality of single unit doses. A "unit dose" is a discrete amount of the pharmaceutical composition comprising a predetermined amount of the active ingredient. The amount of the active ingredient is generally equal to the dosage of the active ingredient which would be administered to a subject and/or a convenient fraction of such a dosage, such as one-half or one-third of such a dosage.

Relative amounts of the active ingredient, the pharmaceutically acceptable excipient, and/or any additional ingredients in a pharmaceutical composition described herein will vary, depending upon the identity, size, and/or condition of the subject treated and further depending upon the route by which the composition is to be administered. The composition may comprise between 0.1% and 100% (w/w) active ingredient.

Pharmaceutically acceptable excipients used in the manufacture of provided pharmaceutical compositions include inert diluents, dispersing and/or granulating agents, surface active agents and/or emulsifiers, disintegrating agents, binding agents, preservatives, buffering agents, lubricating agents, and/or oils. Excipients such as cocoa butter and suppository waxes, coloring agents, coating agents, sweetening, flavoring, and perfuming agents may also be present in the composition.

Exemplary diluents include calcium carbonate, sodium carbonate, calcium phosphate, dicalcium phosphate, calcium sulfate, calcium hydrogen phosphate, sodium phosphate lactose, sucrose, cellulose, microcrystalline cellulose, kaolin, mannitol, sorbitol, inositol, sodium chloride, dry starch, cornstarch, powdered sugar, and mixtures thereof.

Exemplary granulating and/or dispersing agents include potato starch, corn starch, tapioca starch, sodium starch glycolate, clays, alginic acid, guar gum, citrus pulp, agar, bentonite, cellulose, and wood products, natural sponge, cation-exchange resins, calcium carbonate, silicates, sodium carbonate, cross-linked poly(vinyl-pyrrolidone) (crospovidone), sodium carboxymethyl starch (sodium starch glycolate), carboxymethyl cellulose, cross-linked sodium carboxymethyl cellulose (croscarmellose), methylcellulose, pregelatinized starch (starch 1500), microcrystalline starch, water insoluble starch, calcium carboxymethyl cellulose, magnesium aluminum silicate (Veegum), sodium lauryl sulfate, quaternary ammonium compounds, and mixtures thereof.

Exemplary surface active agents and/or emulsifiers include natural emulsifiers, colloidal clays, long chain amino acid derivatives, high molecular weight alcohols, carbomers, carrageenan, cellulosic derivatives, sorbitan fatty acid esters, poly(vinyl-pyrrolidone), diethylene glycol monolaurate, triethanolamine oleate, sodium oleate, potassium oleate, ethyl oleate, oleic acid, ethyl laurate, sodium lauryl sulfate, Pluronic^{®} F-68, poloxamer P-188, cetrimonium bromide, cetylpyridinium chloride, benzalkonium chloride, docusate sodium, and/or mixtures thereof. In certain embodiments, the natural emulsifier is acacia. In certain embodiments, the natural emulsifier is agar. In certain embodiments, the natural emulsifier is alginic acid. In certain embodiments, the natural emulsifier is sodium alginate. In certain embodiments, the natural emulsifier is tragacanth. In certain embodiments, the natural emulsifier is chondrux. In certain embodiments, the natural emulsifier is cholesterol. In certain embodiments, the natural emulsifier is xanthan. In certain embodiments, the natural emulsifier is pectin. In certain embodiments, the natural emulsifier is gelatin. In certain embodiments, the natural emulsifier is egg yolk. In certain embodiments, the natural emulsifier is casein. In certain embodiments, the natural emulsifier is wool fat. In certain embodiments, the natural emulsifier is cholesterol. In certain embodiments, the natural emulsifier is wax. In certain embodiments, the natural emulsifier is lecithin. In certain embodiments, the colloidal clay is bentonite (*i.e.,* aluminum silicate. In certain embodiments, the colloidal clay is Veegum (*i.e.,* magnesium aluminum silicate). In certain embodiments, the high molecular weight alcohol is stearyl alcohol. In certain embodiments, the high molecular weight alcohol is cetyl alcohol. In certain embodiments, the high molecular weight alcohol is oleyl alcohol. In certain embodiments, the high molecular weight alcohol is triacetin monostearate. In certain embodiments, the high molecular weight alcohol is ethylene glycol distearate. In certain embodiments, the high molecular weight alcohol is glyceryl monostearate. In certain embodiments, the high molecular weight alcohol is propylene glycol monostearate. In certain embodiments, the high molecular weight alcohol is polyvinyl alcohol. In certain embodiments, the carbomer is carboxy polymethylene. In certain embodiments, the carbomer is polyacrylic acid. In certain embodiments, the carbomer is acrylic acid polymer. In certain embodiments, the carbomer is carboxyvinyl polymer. In certain embodiments, the cellulosic derivative is carboxymethylcellulose sodium. In certain embodiments, the cellulosic derivative is powdered cellulose. In certain embodiments, the cellulosic derivative is hydroxymethyl cellulose. In certain embodiments, the cellulosic derivative is hydroxypropyl cellulose. In certain embodiments, the cellulosic derivative is hydroxypropyl methylcellulose. In certain embodiments, the cellulosic derivative is methylcellulose. In certain embodiments, the sorbitan fatty acid ester is polyoxyethylene sorbitan monolaurate (Tween^{®} 20). In certain embodiments, the sorbitan fatty acid ester is polyoxyethylene sorbitan (Tween^{®} 60). In certain embodiments, the sorbitan fatty acid ester is polyoxyethylene sorbitan monooleate (Tween^{®} 80). In certain embodiments, the sorbitan fatty acid ester is sorbitan monopalmitate (Span^{®} 40). In certain embodiments, the sorbitan fatty acid ester is sorbitan monostearate (Span^{®} 60). In certain embodiments, the sorbitan fatty acid ester is sorbitan tristearate (Span^{®} 65). In certain embodiments, the sorbitan fatty acid ester is glyceryl monooleate. In certain embodiments, the sorbitan fatty acid ester is sorbitan monooleate (Span^{®} 80). In certain embodiments, the polyoxyethylene ester is polyoxyethylene monostearate (Myrj^{®} 45). In certain embodiments, the polyoxyethylene ester is polyoxyethylene hydrogenated castor oil. In certain embodiments, the polyoxyethylene ester is polyethoxylated castor oil. In certain embodiments, the polyoxyethylene ester is polyoxymethylene stearate. In certain embodiments, the polyoxyethylene ester is Solutol^{®}. In certain embodiments, the polyethylene glycol fatty acid ester is Cremophor^{®}. In certain embodiments, the polyoxyethylene ether is polyoxyethylene lauryl ether (Brij^{®} 30).

Exemplary binding agents include starch, gelatin, sugars, natural and synthetic gums, magnesium aluminum silicate (Veegum^{®}), and larch arabogalactan), alginates, polyethylene oxide, polyethylene glycol, inorganic calcium salts, silicic acid, polymethacrylates, waxes, water, alcohol, and/or mixtures thereof. In certain embodiments, the starch is cornstarch. In certain embodiments, the starch is starch paste. In certain embodiments, the sugar is sucrose. In certain embodiments, the sugar is glucose. In certain embodiments, the sugar is dextrose. In certain embodiments, the sugar is dextrin. In certain embodiments, the sugar is molasses. In certain embodiments, the sugar is lactose. In certain embodiments, the sugar is lactitol. In certain embodiments, the sugar is mannitol. In certain embodiments, the synthetic gum is acacia. In certain embodiments, the synthetic gum is sodium alginate. In certain embodiments, the synthetic gum is extract of Irish moss. In certain embodiments, the synthetic gum is panwar gum. In certain embodiments, the synthetic gum is ghatti gum. In certain embodiments, the synthetic gum is mucilage of isapol husks. In certain embodiments, the synthetic gum is carboxymethylcellulose. In certain embodiments, the synthetic gum is methylcellulose. In certain embodiments, the synthetic gum is ethylcellulose. In certain embodiments, the synthetic gum is hydroxyethylcellulose. In certain embodiments, the synthetic gum is hydroxypropyl cellulose. In certain embodiments, the synthetic gum is hydroxypropyl methylcellulose. In certain embodiments, the synthetic gum is microcrystalline cellulose. In certain embodiments, the synthetic gum is cellulose acetate. In certain embodiments, the synthetic gum is poly(vinylpyrrolidone).

Exemplary preservatives include antioxidants, chelating agents, antimicrobial preservatives, antifungal preservatives, antiprotozoan preservatives, alcohol preservatives, acidic preservatives, and other preservatives. In certain embodiments, the preservative is an antioxidant. In other embodiments, the preservative is a chelating agent.

Exemplary antioxidants include alpha tocopherol, ascorbic acid, acorbyl palmitate, butylated hydroxyanisole, butylated hydroxytoluene, monothioglycerol, potassium metabisulfite, propionic acid, propyl gallate, sodium ascorbate, sodium bisulfite, sodium metabisulfite, and sodium sulfite.

Exemplary chelating agents include ethylenediaminetetraacetic acid (EDTA) and salts and hydrates thereof, citric acid and salts and hydrates thereof, fumaric acid and salts and hydrates thereof, malic acid and salts and hydrates thereof, phosphoric acid and salts and hydrates thereof, and tartaric acid and salts and hydrates thereof. Exemplary antimicrobial preservatives include benzalkonium chloride, benzethonium chloride, benzyl alcohol, bronopol, cetrimide, cetylpyridinium chloride, chlorhexidine, chlorobutanol, chlorocresol, chloroxylenol, cresol, ethyl alcohol, glycerin, hexetidine, imidurea, phenol, phenoxyethanol, phenylethyl alcohol, phenylmercuric nitrate, propylene glycol, and thimerosal. In certain embodiments, the EDTA salt is sodium edetate. In certain embodiments, the EDTA salt is disodium edetate. In certain embodiments, the EDTA salt is trisodium edetate. In certain embodiments, the EDTA salt is calcium disodium edetate. In certain embodiments, the EDTA salt is dipotassium edetate.

Exemplary antifungal preservatives include butyl paraben, methyl paraben, ethyl paraben, propyl paraben, benzoic acid, hydroxybenzoic acid, potassium benzoate, potassium sorbate, sodium benzoate, sodium propionate, and sorbic acid. In certain embodiments, the citric acid is citric acid monohydrate.

Exemplary alcohol preservatives include ethanol, polyethylene glycol, phenol, phenolic compounds, bisphenol, chlorobutanol, hydroxybenzoate, and phenylethyl alcohol.

Exemplary acidic preservatives include vitamin A, vitamin C, vitamin E, beta-carotene, citric acid, acetic acid, dehydroacetic acid, ascorbic acid, sorbic acid, and phytic acid.

Other preservatives include tocopherol, tocopherol acetate, deteroxime mesylate, cetrimide, butylated hydroxyanisol (BHA), butylated hydroxytoluened (BHT), ethylenediamine, sodium lauryl sulfate (SLS), sodium lauryl ether sulfate (SLES), sodium bisulfite, sodium metabisulfite, potassium sulfite, potassium metabisulfite, Glydant^{®} Plus, Phenonip^{®}, methylparaben, Germall^{®} 115, Germaben^{®} II, Neolone^{®}, Kathon^{®}, and Euxyl^{®}.

Exemplary buffering agents include citrate buffer solutions, acetate buffer solutions, phosphate buffer solutions, ammonium chloride, calcium carbonate, calcium chloride, calcium citrate, calcium glubionate, calcium gluceptate, calcium gluconate, D-gluconic acid, calcium glycerophosphate, calcium lactate, propanoic acid, calcium levulinate, pentanoic acid, dibasic calcium phosphate, phosphoric acid, tribasic calcium phosphate, calcium hydroxide phosphate, potassium acetate, potassium chloride, potassium gluconate, potassium mixtures, dibasic potassium phosphate, monobasic potassium phosphate, potassium phosphate mixtures, sodium acetate, sodium bicarbonate, sodium chloride, sodium citrate, sodium lactate, dibasic sodium phosphate, monobasic sodium phosphate, sodium phosphate mixtures, tromethamine, magnesium hydroxide, aluminum hydroxide, alginic acid, pyrogen-free water, isotonic saline, Ringer's solution, ethyl alcohol, and mixtures thereof.

Exemplary lubricating agents include magnesium stearate, calcium stearate, stearic acid, silica, talc, malt, glyceryl behanate, hydrogenated vegetable oils, polyethylene glycol, sodium benzoate, sodium acetate, sodium chloride, leucine, magnesium lauryl sulfate, sodium lauryl sulfate, and mixtures thereof.

Exemplary natural oils include almond, apricot kernel, avocado, babassu, bergamot, black current seed, borage, cade, camomile, canola, caraway, carnauba, castor, cinnamon, cocoa butter, coconut, cod liver, coffee, corn, cotton seed, emu, eucalyptus, evening primrose, fish, flaxseed, geraniol, gourd, grape seed, hazel nut, hyssop, isopropyl myristate, jojoba, kukui nut, lavandin, lavender, lemon, litsea cubeba, macademia nut, mallow, mango seed, meadowfoam seed, mink, nutmeg, olive, orange, orange roughy, palm, palm kernel, peach kernel, peanut, poppy seed, pumpkin seed, rapeseed, rice bran, rosemary, safflower, sandalwood, sasquana, savoury, sea buckthorn, sesame, shea butter, silicone, soybean, sunflower, tea tree, thistle, tsubaki, vetiver, walnut, and wheat germ oils. Exemplary synthetic oils include, but are not limited to, butyl stearate, caprylic triglyceride, capric triglyceride, cyclomethicone, diethyl sebacate, dimethicone 360, isopropyl myristate, mineral oil, octyldodecanol, oleyl alcohol, silicone oil, and mixtures thereof.

Liquid dosage forms for oral and parenteral administration include pharmaceutically acceptable emulsions, microemulsions, solutions, suspensions, syrups and elixirs. In addition to the active ingredients, the liquid dosage forms may comprise inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils, glycerol, tetrahydrofurfuryl alcohol, polyethylene glycols and fatty acid esters of sorbitan, and mixtures thereof. Besides inert diluents, the oral compositions can include adjuvants such as wetting agents, emulsifying and suspending agents, sweetening, flavoring, and perfuming agents. In certain embodiments for parenteral administration, the conjugates described herein are mixed with solubilizing agents such as Cremophor^{®}, alcohols, oils, modified oils, glycols, polysorbates, cyclodextrins, polymers, and mixtures thereof. In certain embodiments, the oil is cottonseed oil. In certain embodiments, the oil is groundnut oil. In certain embodiments, the oil is corn oil. In certain embodiments, the oil is germ oil. In certain embodiments, the oil is olive oil. In certain embodiments, the oil is castor oil. In certain embodiments, the oil is sesame oil.

Injectable preparations, for example, sterile injectable aqueous or oleaginous suspensions can be formulated according to the known art using suitable dispersing or wetting agents and suspending agents. The sterile injectable preparation can be a sterile injectable solution, suspension, or emulsion in a nontoxic parenterally acceptable diluent or solvent, for example, as a solution in 1,3-butanediol. Among the acceptable vehicles and solvents that can be employed are water, Ringer's solution, U.S.P., and isotonic sodium chloride solution. In addition, sterile, fixed oils are conventionally employed as a solvent or suspending medium. For this purpose any bland fixed oil can be employed including synthetic mono- or di-glycerides. In addition, fatty acids such as oleic acid are used in the preparation of injectables.

The injectable formulations can be sterilized, for example, by filtration through a bacterial-retaining filter, or by incorporating sterilizing agents in the form of sterile solid compositions which can be dissolved or dispersed in sterile water or other sterile injectable medium prior to use.

In order to prolong the effect of a drug, it is often desirable to slow the absorption of the drug from subcutaneous or intramuscular injection. This can be accomplished by the use of a liquid suspension of crystalline or amorphous material with poor water solubility. The rate of absorption of the drug then depends upon its rate of dissolution, which, in turn, may depend upon crystal size and crystalline form. Alternatively, delayed absorption of a parenterally administered drug form may be accomplished by dissolving or suspending the drug in an oil vehicle.

Compositions for rectal or vaginal administration are typically suppositories which can be prepared by mixing the conjugates described herein with suitable non-irritating excipients or carriers such as cocoa butter, polyethylene glycol, or a suppository wax which are solid at ambient temperature but liquid at body temperature and therefore melt in the rectum or vaginal cavity and release the active ingredient.

Solid dosage forms for oral administration include capsules, tablets, pills, powders, and granules. In such solid dosage forms, the active ingredient is mixed with at least one inert, pharmaceutically acceptable excipient or carrier such as sodium citrate or dicalcium phosphate and/or (a) fillers or extenders such as starches, lactose, sucrose, glucose, mannitol, and silicic acid, (b) binders such as, for example, carboxymethylcellulose, alginates, gelatin, polyvinylpyrrolidinone, sucrose, and acacia, (c) humectants such as glycerol, (d) disintegrating agents such as agar, calcium carbonate, potato or tapioca starch, alginic acid, certain silicates, and sodium carbonate, (e) solution retarding agents such as paraffin, (f) absorption accelerators such as quaternary ammonium compounds, (g) wetting agents such as, for example, cetyl alcohol and glycerol monostearate, (h) absorbents such as kaolin and bentonite clay, and (i) lubricants such as talc, calcium stearate, magnesium stearate, solid polyethylene glycols, sodium lauryl sulfate, and mixtures thereof. In the case of capsules, tablets, and pills, the dosage form may include a buffering agent.

Solid compositions of a similar type can be employed as fillers in soft and hard-filled gelatin capsules using such excipients as lactose or milk sugar as well as high molecular weight polyethylene glycols and the like. The solid dosage forms of tablets, dragees, capsules, pills, and granules can be prepared with coatings and shells such as enteric coatings and other coatings well known in the art of pharmacology. They may optionally comprise opacifying agents and can be of a composition that they release the active ingredient(s) only, or in a certain part of the intestinal tract, optionally, in a delayed manner. Examples of encapsulating compositions which can be used include polymeric substances and waxes. Solid compositions of a similar type can be employed as fillers in soft and hard-filled gelatin capsules using such excipients as lactose or milk sugar as well as high molecular weight polyethylene glycols and the like.

The active ingredient can be in a micro-encapsulated form with one or more excipients as noted above. The solid dosage forms of tablets, dragees, capsules, pills, and granules can be prepared with coatings and shells such as enteric coatings, release controlling coatings, and other coatings well known in the pharmaceutical formulating art. In such solid dosage forms the active ingredient can be admixed with at least one inert diluent such as sucrose, lactose, or starch. Such dosage forms may comprise, as is normal practice, additional substances other than inert diluents. In certain embodiments, the formulation comprises tableting lubricants. In certain embodiments, the formulation comprises other tableting aids. In certain embodiments, the formulation comprises magnesium stearate. In certain embodiments, the formulation comprises microcrystalline cellulose. In the case of capsules, tablets and pills, the dosage forms may comprise buffering agents. They may optionally comprise opacifying agents and can be of a composition that they release the active ingredient(s) only, or in a certain part of the intestinal tract, optionally, in a delayed manner. Examples of encapsulating agents which can be used include polymeric substances and waxes.

Dosage forms for topical and/or transdermal administration of a compound described herein may include ointments, pastes, creams, lotions, gels, powders, solutions, sprays, inhalants, and/or patches. Generally, the active ingredient is admixed under sterile conditions with a pharmaceutically acceptable carrier or excipient and/or any needed preservatives and/or buffers as can be required. Additionally, the present disclosure contemplates the use of transdermal patches, which often have the added advantage of providing controlled delivery of an active ingredient to the body. Such dosage forms can be prepared, for example, by dissolving and/or dispensing the active ingredient in the proper medium. Alternatively or additionally, the rate can be controlled by either providing a rate controlling membrane and/or by dispersing the active ingredient in a polymer matrix and/or gel.

Suitable devices for use in delivering intradermal pharmaceutical compositions described herein include short needle devices. Intradermal compositions can be administered by devices which limit the effective penetration length of a needle into the skin. Alternatively or additionally, conventional syringes can be used in the classical mantoux method of intradermal administration. Jet injection devices which deliver liquid formulations to the dermis *via* a liquid jet injector and/or *via* a needle which pierces the stratum corneum and produces a jet which reaches the dermis are suitable. Ballistic powder/particle delivery devices which use compressed gas to accelerate the compound in powder form through the outer layers of the skin to the dermis are suitable.

Formulations suitable for topical administration include, but are not limited to, liquid and/or semi-liquid preparations such as liniments, lotions, oil-in-water and/or water-in-oil emulsions such as creams, ointments, and/or pastes, and/or solutions and/or suspensions. Topically administrable formulations may, for example, comprise from about 1% to about 10% (w/w) active ingredient, although the concentration of the active ingredient can be as high as the solubility limit of the active ingredient in the solvent. Formulations for topical administration may further comprise one or more of the additional ingredients described herein.

A pharmaceutical composition described herein can be prepared, packaged, and/or sold in a formulation suitable for pulmonary administration *via* the buccal cavity. Such a formulation may comprise dry particles which comprise the active ingredient and which have a diameter in the range from about 0.5 to about 7 nanometers, or from about 1 to about 6 nanometers. Such compositions are conveniently in the form of dry powders for administration using a device comprising a dry powder reservoir to which a stream of propellant can be directed to disperse the powder and/or using a self-propelling solvent/powder dispensing container such as a device comprising the active ingredient dissolved and/or suspended in a low-boiling propellant in a sealed container. Such powders comprise particles wherein at least 98% of the particles by weight have a diameter greater than 0.5 nanometers and at least 95% of the particles by number have a diameter less than 7 nanometers. Alternatively, at least 95% of the particles by weight have a diameter greater than 1 nanometer and at least 90% of the particles by number have a diameter less than 6 nanometers. Dry powder compositions may include a solid fine powder diluent such as sugar and are conveniently provided in a unit dose form.

Low boiling propellants generally include liquid propellants having a boiling point of below 65 °F at atmospheric pressure. Generally the propellant may constitute 50 to 99.9% (w/w) of the composition, and the active ingredient may constitute 0.1 to 20% (w/w) of the composition. The propellant may further comprise additional ingredients such as a liquid nonionic and/or solid anionic surfactant and/or a solid diluent (which may have a particle size of the same order as particles comprising the active ingredient).

Pharmaceutical compositions described herein formulated for pulmonary delivery may provide the active ingredient in the form of droplets of a solution and/or suspension. Such formulations can be prepared, packaged, and/or sold as aqueous and/or dilute alcoholic solutions and/or suspensions, optionally sterile, comprising the active ingredient, and may conveniently be administered using any nebulization and/or atomization device. Such formulations may further comprise one or more additional ingredients including, but not limited to, a flavoring agent such as saccharin sodium, a volatile oil, a buffering agent, a surface active agent, and/or a preservative such as methylhydroxybenzoate. The droplets provided by this route of administration may have an average diameter in the range from about 0.1 to about 200 nanometers.

Formulations described herein as being useful for pulmonary delivery are useful for intranasal delivery of a pharmaceutical composition described herein. Another formulation suitable for intranasal administration is a coarse powder comprising the active ingredient and having an average particle from about 0.2 to 500 micrometers. Such a formulation is administered by rapid inhalation through the nasal passage from a container of the powder held close to the nares.

Formulations for nasal administration may, for example, comprise from about as little as 0.1% (w/w) to as much as 100% (w/w) of the active ingredient, and may comprise one or more of the additional ingredients described herein. A pharmaceutical composition described herein can be prepared, packaged, and/or sold in a formulation for buccal administration. Such formulations may, for example, be in the form of tablets and/or lozenges made using conventional methods, and may contain, for example, 0.1 to 20% (w/w) active ingredient, the balance comprising an orally dissolvable and/or degradable composition and, optionally, one or more of the additional ingredients described herein. Alternately, formulations for buccal administration may comprise a powder and/or an aerosolized and/or atomized solution and/or suspension comprising the active ingredient. Such powdered, aerosolized, and/or aerosolized formulations, when dispersed, may have an average particle and/or droplet size in the range from about 0.1 to about 200 nanometers, and may further comprise one or more of the additional ingredients described herein.

A pharmaceutical composition described herein can be prepared, packaged, and/or sold in a formulation for ophthalmic administration. Such formulations may, for example, be in the form of eye drops including, for example, a 0.1-1.0% (w/w) solution and/or suspension of the active ingredient in an aqueous or oily liquid carrier or excipient. Such drops may further comprise buffering agents, salts, and/or one or more other of the additional ingredients described herein. Other opthalmically-administrable formulations which are useful include those which comprise the active ingredient in microcrystalline form and/or in a liposomal preparation. Ear drops and/or eye drops are also contemplated as being within the scope of this disclosure.

Although the descriptions of pharmaceutical compositions provided herein are principally directed to pharmaceutical compositions which are suitable for administration to humans, it will be understood by the skilled artisan that such compositions are generally suitable for administration to animals of all sorts. Modification of pharmaceutical compositions suitable for administration to humans in order to render the compositions suitable for administration to various animals is well understood, and the ordinarily skilled veterinary pharmacologist can design and/or perform such modification with ordinary experimentation.

Compounds provided herein are typically formulated in dosage unit form for ease of administration and uniformity of dosage. It will be understood, however, that the total daily usage of the compositions described herein will be decided by a physician within the scope of sound medical judgment. The specific therapeutically effective dose level for any particular subject or organism will depend upon a variety of factors including the disease being treated and the severity of the disorder; the activity of the specific active ingredient employed; the specific composition employed; the age, body weight, general health, sex, and diet of the subject; the time of administration, route of administration, and rate of excretion of the specific active ingredient employed; the duration of the treatment; drugs used in combination or coincidental with the specific active ingredient employed; and like factors well known in the medical arts.

The compounds and compositions provided herein can be administered by any route, including enteral, parenteral, intravenous, intramuscular, intra-arterial, intramedullary, intrathecal, subcutaneous, intraventricular, transdermal, interdermal, rectal, intravaginal, intraperitoneal, topical (as by powders, ointments, creams, and/or drops), mucosal, nasal, bucal, sublingual; by intratracheal instillation, bronchial instillation, and/or inhalation; and/or as an oral spray, nasal spray, and/or aerosol. In certain embodiments, the enteral administration is oral administration. Specifically contemplated routes are oral administration, intravenous administration, regional administration *via* blood and/or lymph supply, and/or direct administration to an affected site. In general, the most appropriate route of administration will depend upon a variety of factors including the nature of the agent, including but not limited to, stability in the environment of the gastrointestinal tract, and/or the condition of the subject, including but not limited to, whether the subject is able to tolerate oral administration. In certain embodiments, the compound or pharmaceutical composition described herein is suitable for topical administration to the eye of a subject. In certain embodiments, the intravenous administration is systemic intravenous injection.

The exact amount of a compound required to achieve an effective amount will vary from subject to subject, depending, for example, on species, age, and general condition of a subject, severity of the side effects or disorder, identity of the particular compound, mode of administration, and the like. An effective amount may be included in a single dose or multiple doses. In certain embodiments, when multiple doses are administered to a subject or applied to a biological sample, any two doses of the multiple doses include different or substantially the same amounts of a compound described herein. In certain embodiments, when multiple doses are administered to a subject or applied to a biological sample, the frequency of administering the multiple doses to the subject or applying the multiple doses to the biological sample is three doses a day, two doses a day, one dose a day, one dose every other day, one dose every third day, one dose every week, one dose every two weeks, one dose every three weeks, or one dose every four weeks. In certain embodiments, the frequency of administering the multiple doses to the subject or applying the multiple doses to the biological sample is one dose per day. In certain embodiments, the frequency of administering the multiple doses to the subject or applying the multiple doses to the biological sample is two doses per day. In certain embodiments, the frequency of administering the multiple doses to the subject or applying the multiple doses to the biological sample is three doses per day. In certain embodiments, when multiple doses are administered to a subject or applied to a biological sample, the duration between the first dose and last dose of the multiple doses is one day, two days, four days, one week, two weeks, three weeks, one month, two months, three months, four months, six months, nine months, one year, two years, three years, four years, five years, seven years, ten years, fifteen years, twenty years, or the lifetime of the subject, tissue, or cell. In certain embodiments, the duration between the first dose and last dose of the multiple doses is three months, six months, or one year. In certain embodiments, the duration between the first dose and last dose of the multiple doses is the lifetime of the subject, tissue, or cell. In certain embodiments, a dose described herein includes independently between 0.1 µg and 1 µg, between 0.001 mg and 0.01 mg, between 0.01 mg and 0.1 mg, between 0.1 mg and 1 mg, between 1 mg and 3 mg, between 3 mg and 10 mg, between 10 mg and 30 mg, between 30 mg and 100 mg, between 100 mg and 300 mg, between 300 mg and 1,000 mg, or between 1 g and 10 g, inclusive, of a compound described herein. In certain embodiments, a dose described herein includes independently between 1 mg and 3 mg, inclusive, of a compound described herein. In certain embodiments, a dose described herein includes independently between 3 mg and 10 mg, inclusive, of a compound described herein. In certain embodiments, a dose described herein includes independently between 10 mg and 30 mg, inclusive, of a compound described herein. In certain embodiments, a dose described herein includes independently between 30 mg and 100 mg, inclusive, of a compound described herein. In certain embodiments, the biological sample is a cell. In certain embodiments, the biological sample is a tissue. In certain embodiments, the dose is a single dose. In certain embodiments, the dose is a single oral dose. In certain embodiments, the dose is multiple doses.

Dose ranges as described herein provide guidance for the administration of provided pharmaceutical compositions to an adult. The amount to be administered to, for example, a child or an adolescent can be determined by a medical practitioner or person skilled in the art and can be lower or the same as that administered to an adult.

A compound or composition, as described herein, can be administered in combination with one or more additional pharmaceutical agents. In certain embodiments, the additional pharmaceutical agent is a therapeutically active agent. In certain embodiments, the additional pharmaceutical agent is a prophylactically active agent. The compounds or compositions can be administered in combination with additional pharmaceutical agents that improve their activity in treating a disease in a subject in need thereof, in preventing a disease in a subject in need thereof, in inhibiting the activity of a histone demethylase in a subject, or biological sample, improve bioavailability, improve safety, reduce drug resistance, reduce and/or modify metabolism, inhibit excretion, and/or modify distribution in a subject, biological sample. In certain embodiments, activity is efficacy. In certain embodiments, activity is potency. It will also be appreciated that the therapy employed may achieve a desired effect for the same disorder, and/or it may achieve different effects. In certain embodiments, a pharmaceutical composition described herein including a compound described herein and an additional pharmaceutical agent shows a synergistic effect that is absent in a pharmaceutical composition including one of the compound and the additional pharmaceutical agent, but not both. In certain embodiments, the histone demethylase is a KDM. In certain embodiments, the KDM is KDM5. In certain embodiments, the biological sample is a cell. In certain embodiments, the biological sample is a tissue.

The compound or composition can be administered concurrently with, prior to, or subsequent to one or more additional pharmaceutical agents, which may be useful as. In certain embodiments, the compound or composition can be administered in a combination therapy. Pharmaceutical agents include therapeutically active agents. Pharmaceutical agents also include prophylactically active agents. Pharmaceutical agents include small organic molecules such as drug compounds, peptides, proteins, carbohydrates, monosaccharides, oligosaccharides, polysaccharides, nucleoproteins, mucoproteins, lipoproteins, synthetic polypeptides or proteins, small molecules linked to proteins, glycoproteins, steroids, nucleic acids, DNAs, RNAs, nucleotides, nucleosides, oligonucleotides, antisense oligonucleotides, lipids, hormones, vitamins, and cells. In certain embodiments, the additional pharmaceutical agent is a pharmaceutical agent useful for treating and/or preventing a disease. In certain embodiments, the disease is a proliferative disease. Each additional pharmaceutical agent may be administered at a dose and/or on a time schedule determined for that pharmaceutical agent. In certain embodiments, the drug compounds is a compound approved for human or veterinary use by the U.S. Food and Drug Administration as provided in the Code of Federal Regulations (CFR). The additional pharmaceutical agents may also be administered together with each other and/or with the compound or composition described herein in a single dose or administered separately in different doses. The particular combination to employ in a regimen will take into account compatibility of the compound described herein with the additional pharmaceutical agent(s) and/or the desired therapeutic and/or prophylactic effect to be achieved. In general, it is expected that the additional pharmaceutical agent(s) in combination be utilized at levels that do not exceed the levels at which they are utilized individually. In some embodiments, the levels utilized in combination will be lower than those utilized individually.

The additional pharmaceutical agents include, but are not limited to, anti-proliferative agents, anti-cancer agents, anti-angiogenesis agents, anti-inflammatory agents, immunosuppressants, anti-bacterial agents, anti-viral agents, cardiovascular agents, cholesterol-lowering agents, anti-diabetic agents, anti-allergic agents, contraceptive agents, pain-relieving agents, and a combination thereof. In certain embodiments, the additional pharmaceutical agent is an anti-proliferative agent. In certain embodiments, the anti-proliferative agent is an anti-cancer agent. In certain embodiments, the additional pharmaceutical agent is an anti-leukemia agent. In certain embodiments, the additional pharmaceutical agent is ABITREXATE (methotrexate), ADE, Adriamycin RDF (doxorubicin hydrochloride), Ambochlorin (chlorambucil), ARRANON (nelarabine), ARZERRA (ofatumumab), BOSULIF (bosutinib), BUSULFEX (busulfan), CAMPATH (alemtuzumab), CERUBIDINE (daunorubicin hydrochloride), CLAFEN (cyclophosphamide), CLOFAREX (clofarabine), CLOLAR (clofarabine), CVP, CYTOSAR-U (cytarabine), CYTOXAN (cyclophosphamide), ERWINAZE (Asparaginase Erwinia Chrysanthemi), FLUDARA (fludarabine phosphate), FOLEX (methotrexate), FOLEX PFS (methotrexate), GAZYVA (obinutuzumab), GLEEVEC (imatinib mesylate), Hyper-CVAD, ICLUSIG (ponatinib hydrochloride), IMBRUVICA (ibrutinib), LEUKERAN (chlorambucil), LINFOLIZIN (chlorambucil), MARQIBO (vincristine sulfate liposome), METHOTREXATE LPF (methorexate), MEXATE (methotrexate), MEXATE-AQ (methotrexate), mitoxantrone hydrochloride, MUSTARGEN (mechlorethamine hydrochloride), MYLERAN (busulfan), NEOSAR (cyclophosphamide), ONCASPAR (Pegaspargase), PURINETHOL (mercaptopurine), PURIXAN (mercaptopurine), Rubidomycin (daunorubicin hydrochloride), SPRYCEL (dasatinib), SYNRIBO (omacetaxine mepesuccinate), TARABINE PFS (cytarabine), TASIGNA (nilotinib), TREANDA (bendamustine hydrochloride), TRISENOX (arsenic trioxide), VINCASAR PFS (vincristine sulfate), ZYDELIG (idelalisib), or a combination thereof. In certain embodiments, the additional pharmaceutical agent is an anti-lymphoma agent. In certain embodiments, the additional pharmaceutical agent is ABITREXATE (methotrexate), ABVD, ABVE, ABVE-PC, ADCETRIS (brentuximab vedotin), ADRIAMYCIN PFS (doxorubicin hydrochloride), ADRIAMYCIN RDF (doxorubicin hydrochloride), AMBOCHLORIN (chlorambucil), AMBOCLORIN (chlorambucil), ARRANON (nelarabine), BEACOPP, BECENUM (carmustine), BELEODAQ (belinostat), BEXXAR (tositumomab and iodine I 131 tositumomab), BICNU (carmustine), BLENOXANE (bleomycin), CARMUBRIS (carmustine), CHOP, CLAFEN (cyclophosphamide), COPP, COPP-ABV, CVP, CYTOXAN (cyclophosphamide), DEPOCYT (liposomal cytarabine), DTIC-DOME (dacarbazine), EPOCH, FOLEX (methotrexate), FOLEX PFS (methotrexate), FOLOTYN (pralatrexate), HYPER-CVAD, ICE, IMBRUVICA (ibrutinib), INTRON A (recombinant interferon alfa-2b), ISTODAX (romidepsin), LEUKERAN (chlorambucil), LINFOLIZIN (chlorambucil), Lomustine, MATULANE (procarbazine hydrochloride), METHOTREXATE LPF (methotrexate), MEXATE (methotrexate), MEXATE-AQ (methotrexate), MOPP, MOZOBIL (plerixafor), MUSTARGEN (mechlorethamine hydrochloride), NEOSAR (cyclophosphamide), OEPA, ONTAK (denileukin diftitox), OPPA, R-CHOP, REVLIMID (lenalidomide), RITUXAN (rituximab), STANFORD V, TREANDA (bendamustine hydrochloride), VAMP, VELBAN (vinblastine sulfate), VELCADE (bortezomib), VELSAR (vinblastine sulfate), VINCASAR PFS (vincristine sulfate), ZEVALIN (ibritumomab tiuxetan), ZOLINZA (vorinostat), ZYDELIG (idelalisib), or a combination thereof. In certain embodiments, the additional pharmaceutical agent is REVLIMID (lenalidomide), DACOGEN (decitabine), VIDAZA (azacitidine), CYTOSAR-U (cytarabine), IDAMYCIN (idarubicin), CERUBIDINE (daunorubicin), LEUKERAN (chlorambucil), NEOSAR (cyclophosphamide), FLUDARA (fludarabine), LEUSTATIN (cladribine), or a combination thereof. In certain embodiments, the additional pharmaceutical agent is ABITREXATE (methotrexate), ABRAXANE (paclitaxel albumin-stabilized nanoparticle formulation), AC, AC-T, ADE, ADRIAMYCIN PFS (doxorubicin hydrochloride), ADRUCIL (fluorouracil), AFINITOR (everolimus), AFINITOR DISPERZ (everolimus), ALDARA (imiquimod), ALIMTA (pemetrexed disodium), AREDIA (pamidronate disodium), ARIMIDEX (anastrozole), AROMASIN (exemestane), AVASTIN (bevacizumab), BECENUM (carmustine), BEP, BICNU (carmustine), BLENOXANE (bleomycin), CAF, CAMPTOSAR (irinotecan hydrochloride), CAPOX, CAPRELSA (vandetanib), CARBOPLATIN-TAXOL, CARMUBRIS (carmustine), CASODEX (bicalutamide), CEENU (lomustine), CERUBIDINE (daunorubicin hydrochloride), CERVARIX (recombinant HPV bivalent vaccine), CLAFEN (cyclophosphamide), CMF, COMETRIQ (cabozantinib-s-malate), COSMEGEN (dactinomycin), CYFOS (ifosfamide), CYRAMZA (ramucirumab), CYTOSAR-U (cytarabine), CYTOXAN (cyclophosphamide), DACOGEN (decitabine), DEGARELIX, DOXIL (doxorubicin hydrochloride liposome), DOXORUBICIN HYDROCHLORIDE, DOX-SL (doxorubicin hydrochloride liposome), DTIC-DOME (dacarbazine), EFUDEX (fluorouracil), ELLENCE (epirubicin hydrochloride), ELOXATIN (oxaliplatin), ERBITUX (cetuximab), ERIVEDGE (vismodegib), ETOPOPHOS (etoposide phosphate), EVACET (doxorubicin hydrochloride liposome), FARESTON (toremifene), FASLODEX (fulvestrant), FEC, FEMARA (letrozole), FLUOROPLEX (fluorouracil), FOLEX (methotrexate), FOLEX PFS (methotrexate), FOLFIRI , FOLFIRI-BEVACIZUMAB, FOLFIRI-CETUXIMAB, FOLFIRINOX, FOLFOX, FU-LV, GARDASIL (recombinant human papillomavirus (HPV) quadrivalent vaccine), GEMCITABINE-CISPLATIN, GEMCITABINE-OXALIPLATIN, GEMZAR (gemcitabine hydrochloride), GILOTRIF (afatinib dimaleate), GLEEVEC (imatinib mesylate), GLIADEL (carmustine implant), GLIADEL WAFER (carmustine implant), HERCEPTIN (trastuzumab), HYCAMTIN (topotecan hydrochloride), IFEX (ifosfamide), IFOSFAMIDUM (ifosfamide), INLYTA (axitinib), INTRON A (recombinant interferon alfa-2b), IRESSA (gefitinib), IXEMPRA (ixabepilone), JAKAFI (ruxolitinib phosphate), JEVTANA (cabazitaxel), KADCYLA (ado-trastuzumab emtansine), KEYTRUDA (pembrolizumab), KYPROLIS (carfilzomib), LIPODOX (doxorubicin hydrochloride liposome), LUPRON (leuprolide acetate), LUPRON DEPOT (leuprolide acetate), LUPRON DEPOT-3 MONTH (leuprolide acetate), LUPRON DEPOT-4 MONTH (leuprolide acetate), LUPRON DEPOT-PED (leuprolide acetate), MEGACE (megestrol acetate), MEKINIST (trametinib), METHAZOLASTONE (temozolomide), METHOTREXATE LPF (methotrexate), MEXATE (methotrexate), MEXATE-AQ (methotrexate), MITOXANTRONE HYDROCHLORIDE, MITOZYTREX (mitomycin c), MOZOBIL (plerixafor), MUSTARGEN (mechlorethamine hydrochloride), MUTAMYCIN (mitomycin c), MYLOSAR (azacitidine), NAVELBINE (vinorelbine tartrate), NEOSAR (cyclophosphamide), NEXAVAR (sorafenib tosylate), NOLVADEX (tamoxifen citrate), NOVALDEX (tamoxifen citrate), OFF, PAD, PARAPLAT (carboplatin), PARAPLATIN (carboplatin), PEG-INTRON (peginterferon alfa-2b), PEMETREXED DISODIUM, PERJETA (pertuzumab), PLATINOL (cisplatin), PLATINOL-AQ (cisplatin), POMALYST (pomalidomide), prednisone, PROLEUKIN (aldesleukin), PROLIA (denosumab), PROVENGE (sipuleucel-t), REVLIMID (lenalidomide), RUBIDOMYCIN (daunorubicin hydrochloride), SPRYCEL (dasatinib), STIVARGA (regorafenib), SUTENT (sunitinib malate), SYLATRON (peginterferon alfa-2b), SYLVANT (siltuximab), SYNOVIR (thalidomide), TAC, TAFINLAR (dabrafenib), TARABINE PFS (cytarabine), TARCEVA (erlotinib hydrochloride), TASIGNA (nilotinib), TAXOL (paclitaxel), TAXOTERE (docetaxel), TEMODAR (temozolomide), THALOMID (thalidomide), TOPOSAR (etoposide), TORISEL (temsirolimus), TPF, TRISENOX (arsenic trioxide), TYKERB (lapatinib ditosylate), VECTIBIX (panitumumab), VEIP, VELBAN (vinblastine sulfate), VELCADE (bortezomib), VELSAR (vinblastine sulfate), VEPESID (etoposide), VIADUR (leuprolide acetate), VIDAZA (azacitidine), VINCASAR PFS (vincristine sulfate), VOTRIENT (pazopanib hydrochloride), WELLCOVORIN (leucovorin calcium), XALKORI (crizotinib), XELODA (capecitabine), XELOX, XGEVA (denosumab), XOFIGO (radium 223 dichloride), XTANDI (enzalutamide), YERVOY (ipilimumab), ZALTRAP (ziv-aflibercept), ZELBORAF (vemurafenib), ZOLADEX (goserelin acetate), ZOMETA (zoledronic acid), ZYKADIA (ceritinib), ZYTIGA (abiraterone acetate), ENMD-2076, PCI-32765, AC220, dovitinib lactate (TKI258, CHIR-258), BIBW 2992 (TOVOK^{™}), SGX523, PF-04217903, PF-02341066, PF-299804, BMS-777607, ABT-869, MP470, BIBF 1120 (VARGATEF^{®}), AP24534, JNJ-26483327, MGCD265, DCC-2036, BMS-690154, CEP-11981, tivozanib (AV-951), OSI-930, MM-121, XL-184, XL-647, and/or XL228), proteasome inhibitors, mTOR inhibitors, oblimersen, gemcitabine, carminomycin, leucovorin, pemetrexed, cyclophosphamide, dacarbazine, procarbizine, prednisolone, dexamethasone, campathecin, plicamycin, asparaginase, aminopterin, methopterin, porfiromycin, melphalan, leurosidine, leurosine, chlorambucil, trabectedin, procarbazine, discodermolide, carminomycin, aminopterin, and hexamethyl melamine, or a combination thereof. In certain embodiments, the proteasome inhibitor is bortezomib (Velcade). In certain embodiments, the mTOR inhibitor is rapamycin. In certain embodiments, the mTOR inhibitor is temsirolimus (CCI-779). In certain embodiments, the mTOR inhibitor is everolimus (RAD-001). In certain embodiments, the mTOR inhibitor is ridaforolimus. In certain embodiments, the mTOR inhibitor is AP23573 (Ariad). In certain embodiments, the mTOR inhibitor is AZD8055 (AstraZeneca). In certain embodiments, the mTOR inhibitor is BEZ235 (Novartis). In certain embodiments, the mTOR inhibitor is BGT226 (Norvartis). In certain embodiments, the mTOR inhibitor is XL765 (Sanofi Aventis). In certain embodiments, the mTOR inhibitor is PF-4691502 (Pfizer). In certain embodiments, the mTOR inhibitor is GDC0980 (Genetech). In certain embodiments, the mTOR inhibitor is SF1126 (Semafoe). In certain embodiments, the mTOR inhibitor is OSI-027 (OSI). In certain embodiments, the additional pharmaceutical agent is ibrutinib. In certain embodiments, the additional pharmaceutical agent is a protein kinase inhibitor. In certain embodiments, the additional protein kinase inhibitor is a tyrosine protein kinase inhibitor. In certain embodiments, the additional pharmaceutical agent is a binder or inhibitor of a histone demethylase. In certain embodiments, the histone demethylase is a KDM. In certain embodiments, the KDM is KDM5. In certain embodiments, the additional pharmaceutical agent is a binder or inhibitor of a KDM. In certain embodiments, the additional pharmaceutical agent is a binder or inhibitor of KDM5. In certain embodiments, the additional pharmaceutical agent is a binder or inhibitor of Bruton's tyrosine kinase (BTK). In certain embodiments, the additional pharmaceutical agent is selected from the group consisting of epigenetic or transcriptional modulators, antimitotic drugs, hormone receptor modulators, Hsp90 inhibitors, glucocorticoids, all*-trans* retinoic acids, and other agents that promote differentiation. In certain embodiments, the compounds described herein or pharmaceutical compositions can be administered in combination with an anti-cancer therapy including, but not limited to, surgery, radiation therapy, transplantation, immunotherapy, and chemotherapy. In certain embodiments, the transplantation is stem cell transplantation. In certain embodiments, the transplantation is bone marrow transplantation. In certain embodiments, the transcriptional modulator is a DNA methyltransferase inhibitor. In certain embodiments, the transcriptional modulator is histone deacetylase inhibitors (HDAC inhibitors). In certain embodiments, the transcriptional modulator is a lysine methyltransferase inhibitors. In certain embodiments, the antimitotic drug is a taxanes. In certain embodiments, the antimitotic drug is a vinca alkaloids. In certain embodiments, the hormone receptor modulator is an estrogen receptor modulators. In certain embodiments, the hormone receptor modulator is an androgen receptor modulators. In certain embodiments, the cell signaling pathway inhibitor is a tyrosine protein kinase inhibitors. In certain embodiments, the modulator of protein stability is a proteasome inhibitors.

### Uses

The present disclosure also provides the pharmaceutical composition according to the present invention for use in treating a proliferative disease in which KDM5 plays a role. In certain embodiments, the proliferative disease is cancer. In certain embodiments, the cancer is carcinoma. In certain embodiments, the cancer is lung cancer. In certain embodiments, the cancer is breast cancer. In certain embodiments, the cancer is liver cancer. In certain embodiments, the cancer is pancreatic cancer. In certain embodiments, the cancer is gastric cancer. In certain embodiments, the cancer is ovarian cancer. In certain embodiments, the cancer is colon cancer. In certain embodiments, the cancer is colorectal cancer.

The present disclosure also provides a compound of Formulae (I-B), (II), or a pharmaceutically acceptable salt, co-crystal, tautomer, stereoisomer, solvate, hydrate, polymorph, isotopically enriched derivative, or composition thereof, as defined herein, for use in the treatment of a proliferative diseases in which KDM5 plays a role, in a subject in need thereof.

Without wishing to be bound by any particular theory, the compounds described herein are able to bind the histone demethylase being inhibited. In certain embodiments, a compound described herein is able to bind the histone demethylase. In certain embodiments, the compound described herein is able to covalently bind a KDM. In certain embodiments, the compound described herein is able to covalently bind KDM5. In certain embodiments, the compounds as described herein covalently modify the histone demethylase.

In certain embodiments, the subject being treated is a mammal. In certain embodiments, the subject is a human. In certain embodiments, the subject is a domesticated animal, such as a dog, cat, cow, pig, horse, sheep, or goat. In certain embodiments, the subject is a companion animal, such as a dog or cat. In certain embodiments, the subject is a livestock animal, such as a cow, pig, horse, sheep, or goat. In certain embodiments, the subject is a zoo animal. In another embodiment, the subject is a research animal such as a rodent, dog, or non-human primate. In certain embodiments, the subject is a non-human transgenic animal such as a transgenic mouse or transgenic pig.

The disease to be treated or prevented using the compounds described herein may be associated with increased activity of a histone demethylase, such as a KDM. The disease to be treated or prevented using the compounds described herein may be associated with the overexpression of a histone demethylase, such as a KDM. In certain embodiments, the disease is a proliferative disease. In certain embodiments, the disease is a cardiovascular disease. In certain embodiments, the histone demethylase is a KDM. In certain embodiments, the KDM is KDM5.

In certain embodiments, the disease to be treated or prevented using the compounds described herein may be associated with the overexpression of a histone demethylase, such as a KDM. A disease may be associated with the aberrant activity of a histone demethylase, such as a KDM. Aberrant activity of a histone demethylase, such as a KDM, may be elevated and/or inappropriate or undesired activity of the histone demethylase. The compounds described herein, and pharmaceutically acceptable salts, solvates, hydrates, polymorphs, co-crystals, tautomers, stereoisomers, isotopically labeled derivatives, and compositions thereof, may inhibit the activity of a histone demethylase and be useful in treating and/or preventing diseases. The compounds described herein, and pharmaceutically acceptable salts, solvates, hydrates, polymorphs, co-crystals, tautomers, stereoisomers, isotopically labeled derivatives, and compositions thereof, may inhibit the activity of a CDK and be useful in treating and/or preventing diseases. The compounds described herein, and pharmaceutically acceptable salts, solvates, hydrates, polymorphs, co-crystals, tautomers, stereoisomers, isotopically labeled derivatives, and compositions thereof, may inhibit the activity of a histone demethylase and be useful in treating and/or preventing diseases. In certain embodiments, the disease is a proliferative disease.

In certain embodiments, the proliferative disease is a hematological malignancy. In certain embodiments, the proliferative disease is a blood cancer. In certain embodiments, the proliferative disease is leukemia. In certain embodiments, the proliferative disease is a carcinoma. In certain embodiments, the proliferative disease is lymphoma. In certain embodiments, the proliferative disease is T-cell lymphoma. In some embodiments, the proliferative disease is Burkitt's lymphoma. In certain embodiments, the proliferative disease is a Hodgkin's lymphoma. In certain embodiments, the proliferative disease is a non-Hodgkin's lymphoma. In certain embodiments, the proliferative disease is multiple myeloma. In certain embodiments, the proliferative disease is melanoma. In certain embodiments, the proliferative disease is colorectal cancer. In certain embodiments, the proliferative disease is colon cancer. In certain embodiments, the proliferative disease is breast cancer. In certain embodiments, the proliferative disease is recurring breast cancer. In certain embodiments, the proliferative disease is mutant breast cancer. In certain embodiments, the proliferative disease is HER2+ breast cancer. In certain embodiments, the proliferative disease is HER2- breast cancer. In certain embodiments, the proliferative disease is triple-negative breast cancer (TNBC). In certain embodiments, the proliferative disease is a bone cancer. In certain embodiments, the proliferative disease is osteosarcoma. In certain embodiments, the proliferative disease is Ewing's sarcoma. In some embodiments, the proliferative disease is a brain cancer. In some embodiments, the proliferative disease is neuroblastoma. In some embodiments, the proliferative disease is a lung cancer. In some embodiments, the proliferative disease is small cell lung cancer (SCLC). In some embodiments, the proliferative disease is non-small cell lung cancer (NSCLC). In some embodiments, the proliferative disease is liver cancer. In some embodiments, the proliferative disease is pancreatic cancer. In some embodiments, the proliferative disease is gastric cancer. In some embodiments, the proliferative disease is bladder cancer. In some embodiments, the proliferative disease is prostate cancer. In some embodiments, the proliferative disease is ovarian cancer. In some embodiments, the proliferative disease is ovarian cancer. In some embodiments, the proliferative disease is a benign neoplasm. In some embodiments, the proliferative disease is lung cancer, breast cancer, liver cancer, pancreatic cancer, gastric cancer, ovarian cancer, colon cancer, colorectal cancer, bladder cancer, or prostate cancer.

All types of benign neoplasms disclosed herein or known in the art are contemplated as being within the scope of the present disclosure. In some embodiments, the proliferative disease is associated with angiogenesis. All types of angiogenesis disclosed herein or known in the art are contemplated as being within the scope of the present disclosure.

In certain embodiments, the methods described herein include administering to a subject an effective amount of a compound described herein, or a pharmaceutically acceptable salt, solvate, hydrate, polymorph, co-crystal, tautomer, stereoisomer, or isotopically labeled derivative, thereof, or a pharmaceutical composition thereof. In certain embodiments, the methods described herein include administering to a subject an effective amount of a compound described herein, or a pharmaceutically acceptable salt thereof, or a pharmaceutical composition thereof. In certain embodiments, the compound is administered to a subject. In certain embodiments, the compound is administered in combination with one or more additional pharmaceutical agents described herein. The additional pharmaceutical agent may be an anti-proliferative agent. In certain embodiments, the additional pharmaceutical agent is an anti-cancer agent. The additional pharmaceutical agent may also be a kinase inhibitor. In certain embodiments, the additional pharmaceutical agent is an inhibitor of histone demethylase. In certain embodiments, the additional pharmaceutical agent is an inhibitor of a KDM. In certain embodiments, the additional pharmaceutical agent includes an anti-cancer agent, anti-inflammatory agent, steroids, immunosuppressant, radiation therapy, or other agents. In certain embodiments, the additional pharmaceutical agent is an anti-proliferative agent. In certain embodiments, the additional pharmaceutical agent is a non-selective inhibitor of a histone demethylase. In certain embodiments, the additional pharmaceutical agent is an immunotherapy agent. In certain embodiments, the additional pharmaceutical agent is an immune checkpoint inhibitor. In certain embodiments, the anti-cancer agent is a chemotherapeutic. In certain embodiments, the immunotherapy agent is a PD1 inhibitor. In certain embodiments, the immunotherapy agent is a PDL1 inhibitor.

In some embodiments, the additional pharmaceutical agent is a topoisomerase inhibitor, a MCL1 inhibitor, a BCL-2 inhibitor, a BCL-xL inhibitor, a BRD4 inhibitor, a BRCA1 inhibitor, BRCA2 inhibitor, HER1 inhibitor, HER2 inhibitor, a CDK9 inhibitor, a Jumonji histone demethylase inhibitor, or a DNA damage inducer. In some embodiments, the additional pharmaceutical agent is etoposide, obatoclax, navitoclax, JQ1, 4-(((5'-chloro-2'-(((1*R*,4*R*)-4-(((*R*)-1-methoxypropan-2-yl)amino)cyclohexyl)amino)-[2,4'-bipyridin]-6-yl)amino)methyl)tetrahydro-2H-pyran-4-carbonitrile, JIB04, or cisplatin. Exemplary chemotherapeutic agents include alkylating agents such as nitrogen mustards, ethylenimines, methylmelamines, alkyl sulfonates, nitrosuoureas, and triazenes; antimetabolites such as folic acid analogs, pyrimidine analogs, in particular fluorouracil and cytosine arabinoside, and purine analogs; natural products such as vinca alkaloids epi-podophyllotoxins, antibiotics, enzymes, and biological response modifiers; and miscellaneous products such as platinum coordination complexes, anthracenedione, substituted urea such as hydroxyurea, methyl hydrazine derivatives, and adrenocorticoid suppressant. Exemplary chemotherapeutic agents also include anthracycline antibiotics, actinomycin D, plicamycin, puromycin, gramicidin D, paclitaxel, colchicine, cytochalasin B, emetine, maytansine, amsacrine, cisplatin, carboplatin, mitomycin, altretamine, cyclophosphamide, lomustine, and carmustine. In certain embodiments, a pharmaceutical composition described herein further comprises a combination of the additional pharmaceutical agents described herein.

The compounds or compositions disclosed herein may synergistically augment inhibition of histone demethylase induced by the additional pharmaceutical agent(s) in the biological sample or subject. Thus, the combination of the compounds or compositions disclosed herein and the additional pharmaceutical agent(s) may be useful in treating proliferative diseases and/or cardiovascular diseases resistant to a treatment using the additional pharmaceutical agent(s) without the compounds or compositions.

In some embodiments, the activity of a histone demethylase is non-selectively inhibited by the compounds or pharmaceutical compositions described herein. In some embodiments, the activity of the histone demethylase being inhibited is selectively inhibited by the compounds or pharmaceutical compositions described herein, compared to the activity of a different protein. In certain embodiments, the activity of a histone demethylase is selectively inhibited by a compound or pharmaceutical composition described herein, compared to the activity of a different protein. In certain embodiments, the activity of KDM5 is selectively inhibited by a compound or pharmaceutical composition described herein, compared to the activity of another KDM. In certain embodiments, the KDM is KDM2/7. In certain embodiments, the KDM is KDM3. In certain embodiments, the KDM is KDM4. In certain embodiments, the KDM is KDM6. In certain embodiments, the histone demethylase is a KDM. In certain embodiments, the KDM is KDM5.

The selectivity of a compound or pharmaceutical composition described herein in inhibiting the activity of a histone demethylase over a different histone demethylase may be measured by the quotient of the IC₅₀ value of the compound or pharmaceutical composition in inhibiting the activity of the different histone demethylase over the IC₅₀ value of the compound or pharmaceutical composition in inhibiting the activity of the histone demethylase. The selectivity of a compound or pharmaceutical composition described herein for a histone demethylase over a different histone demethylase may also be measured by the quotient of the *K*_{d} value of an adduct of the compound or pharmaceutical composition and the different protein over the *K*_{d} value of an adduct of the compound or pharmaceutical composition and the histone demethylase. In certain embodiments, the selectivity is at least 2-fold, at least 3-fold, at least 5-fold, at least 10-fold, at least 30-fold, at least 50-fold, at least 100-fold, at least 300-fold, at least 500-fold, at least 1,000-fold, at least 3,000-fold, at least 5,000-fold, at least 10,000-fold, at least 30,000-fold, at least 50,000-fold, or at least 100,000-fold. In certain embodiments, the selectivity is not more than 100,000-fold, not more than 10,000-fold, not more than 1,000-fold, not more than 100-fold, not more than 10-fold, or not more than 2-fold. Combinations of the above-referenced range are also within the scope of the disclosure. In certain embodiments, the histone demethylase is a KDM. In certain embodiments, the KDM is KDM5.

### EXAMPLES

In order that the present disclosure may be more fully understood, the following examples are set forth. The synthetic and biological examples described in this application are offered to illustrate the compounds, pharmaceutical compositions, and methods provided herein and are not to be construed in any way as limiting their scope.

### Example 1. IC₅₀ for exemplary compounds.

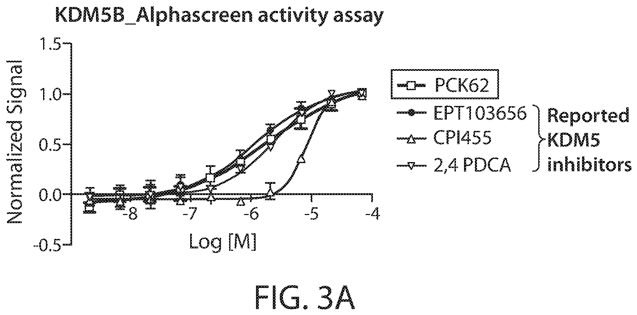

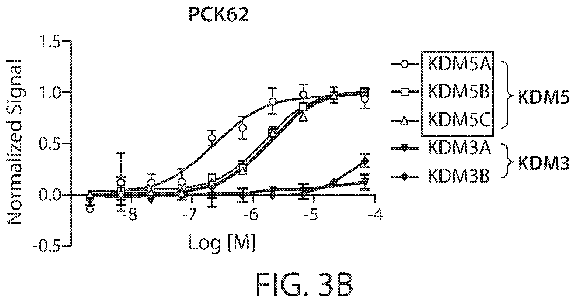

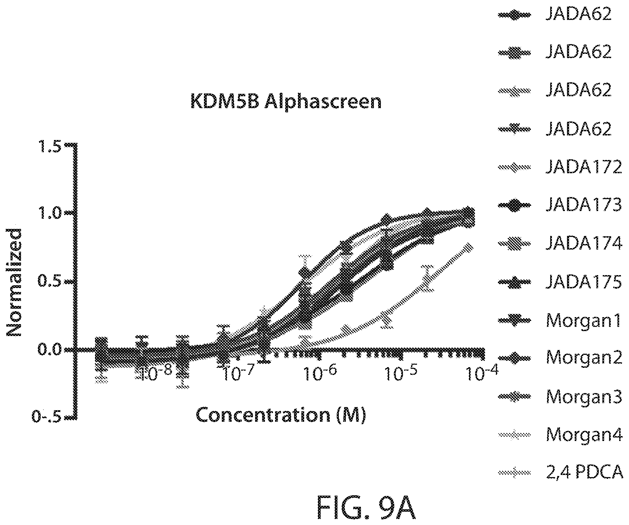

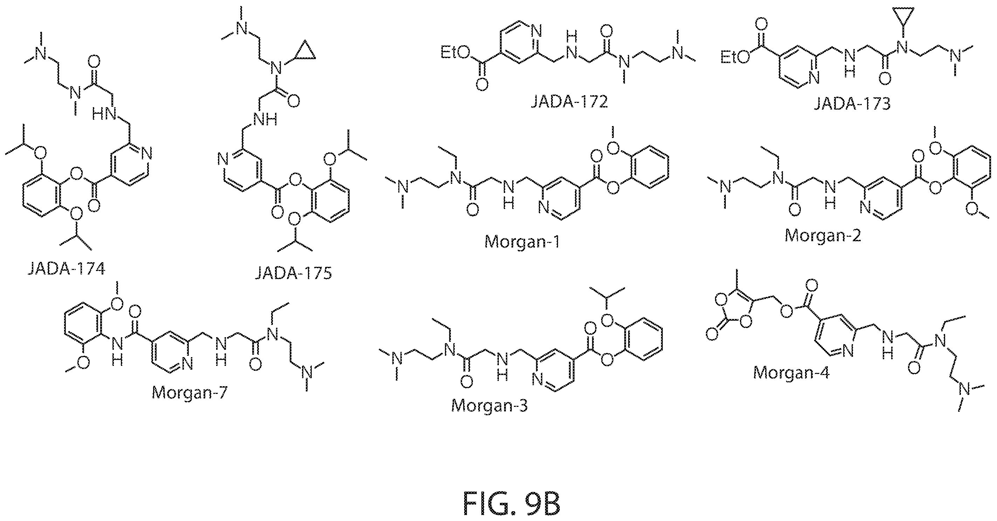

IC₅₀ inhibitory activity for KDM5 selective inhibitor PCK62 and other exemplary KDM5 inhibitors against KDM5B was determined in an alphascreen activity assay (FIG. 3; *Table 1*). Exemplary KDM5 inhibitor PCK62 (JADA62) was also screened against different KDMs (KDM5A, KDM5B, KDM5C; KDM3A, and KDM3B). (FIG. 3; *Table* 2)*.* PCK62 shows reasonable inhibitory activity of KDM5, as compared to other exemplary KDM5 inhibitors. Inhibitory activity for exemplary KDM5 inhibitors PCK62 (JADA-62), JADA172, JADA173, JADA174, JADA175, Morgan1, Morgan2, Morgan3, and Morgan4 against KDM5B was determined in an alphascreen activity assay (FIG. 9A and FIG. 9B; *Table 3*)*.*

**Table 1. IC₅₀ data for exemplary KDM5 inhibitors in a KDM5B alphascreen activity assay.**

| **compounds** | **IC50 (uM)** |
|---|---|
| **PCK62** | **1.54** |
| **EPT103656** | **2.39** |
| **CPI455** | **7.92** |
| **2,4 PDCA** | **3.19** |

**Table 2. IC₅₀ data for PCK62 against different KDM's.**

| **Protein** | **IC50 (uM)** |
|---|---|
| **KDM5A** | **0.24** |
| **KDM5B** | **1.54** |
| **KDM5C** | **1.74** |
| **KDM3A** | **N/A** |
| **KDM3B** | **N/A** |

**Table 3. IC₅₀ data for Exemplary KDM5B Inhibitors Against KDM5B.**

| **compounds** | **IC50 (uM)** |
|---|---|
| **JADA62** | **0.61** |
| **JADA172** | **6.75** |
| **JADA173** | **1.53** |
| **JADA174** | **1.04** |
| **JADA175** | **0.89** |
| **Morgan1** | **0.94** |
| **Morgan2** | **0.39** |
| **Morgan3** | **0.52** |
| **Morpan4** | **0.29** |
| **Morgan7** | **0.46** |
| **2,4 PDCA** | **0.85** |

### Example 2. Alphascreen Assays.

The AlphaScreen assay was performed in 384-well plate format using white AlphaPlate (PerkinElmer, USA), and transfer of pre-diluted compound (100 nl) was performed using a Janus Workstation (PerkinElmer, USA). All subsequent steps were carried out in assay buffer (50 mM HEPES, pH 7.5, 0.1% (wt/vol) BSA and 0.01% (vol/vol) Tween-20). In brief, 10 µl of assay buffer containing demethylase enzyme (2 nM Final) was preincubated for 15 min with dilutions of compound. The enzyme reaction was initiated by the addition of substrate (5 µl) consisting of L-ascorbic acid (100 µM Final), 2-OG (5 uM Final), FAS (10 uM Final) and histone H3(1-21)K4Me3-GGK Biotin (100nM Final). The enzyme reaction was allowed to proceed for 30 minutes and was stopped by the addition of 5 µl of assay buffer containing EDTA (40 mM) and NaCl (1200 mM). The final concentration of DMSO was 1%. Streptavidin donor beads (0.08 mg/ml) and protein-A-conjugated acceptor beads (0.08 mg/ml) were preincubated for 1 h with antibody to methyl mark (300 ng/ml Final), and the presence of histone H3 product methyl mark was detected using the preincubated AlphaScreen beads (5 µl). Detection was allowed to proceed for 2 h at room temperature, and the assay plates were read on the Envision 2104 plate reader. Data were normalized to the (no-enzyme) control, and the IC₅₀ values were determined via nonlinear regression curve fit using GraphPad Prism 7. The results of the Alphascreen assays are depicted in FIG. 3A-3B and FIG. 9A-9B.

| | peptide | Supplier | Code |
|---|---|---|---|
| KDM5 | H3(1-21)K4Me3-GGK Biotin | Anaspec | 64192 |
| KDM6 | H3(21-44)K27Me3-GGK Biotin | Anaspec | 64367 |
| KDM4 | H3(1-21)K9Me3-GGK Biotin | Anaspec | 64360 |
| KDM3 | H3(1-21)K9Me2-GGK Biotin | Anaspec | 64359 |
| KDM2 | H3(21-44)K36Me2-GGK Biotin | Anaspec | 64442 |

| | antibody | Supplier | catalog# |
|---|---|---|---|
| KDM5 | Anti-H3K4me2 | Cell Signaling Technology | 9725S |
| KDM6 | Anti-H3K27me2 | Millipore | 07-452 |
| KDM4 | Anti-H3K9me2 | Abcam | ab1220 |
| KDM3 | Anti-H3K9me1 | Abcam | ab8896 |
| KDM2 | Anti-H3K36me1 | Abcam | ab9048 |

### Example 3. CTG assay for exemplary compounds.

Cell Titer glo assays were performed by treating MM.1S cells with compounds at concentrations indicated. Anti-proliferative effects of compounds were assessed using Cell Titer Glo assay kit (Promega). IC₅₀ values were determined using Graphpad Prism nonlinear regression curve fit.

The CellTiter-Glo^{®} Luminescent Cell Viability Assay is a homogeneous method of determining the number of viable cells in culture based on quantitation of the ATP present, an indicator of metabolically active cells. The CellTiter-Glo^{®} Assay is designed for use with multiwell formats, making it ideal for automated high-throughput screening (HTS), cell proliferation and cytotoxicity assays. The homogeneous assay procedure involves adding the single reagent (CellTiter-Glo^{®} Reagent) directly to cells cultured in serum-supplemented medium. Cell washing, removal of medium and multiple pipetting steps are not required. The system detects as few as 15 cells/well in a 384-well format in 10 minutes after adding reagent and mixing.

The homogeneous "add-mix-measure" format results in cell lysis and generation of a luminescent signal proportional to the amount of ATP present. The amount of ATP is directly proportional to the number of cells present in culture. The CellTiter-Glo^{®} Assay generates a "glow-type" luminescent signal, which has a half-life generally greater than five hours, depending on cell type and medium used. The extended half-life eliminates the need to use reagent injectors and provides flexibility for continuous or batch mode processing of multiple plates. The unique homogeneous format avoids errors that may be introduced by other ATP measurement methods that require multiple steps.

### Example 4. Biological function of KDM5A in Multiple Myeloma by shRNA-mediated knockdown

Knockdown or pharmacologic inhibition of KDM5A induced G0/G1 cell cycle arrest and impaired the growth of MM cell lines. In contrast, JADA62 did not affect the growth of normal peripheral blood mononuclear cells (PBMNCs). KDM5A inhibitor modestly induced apoptosis in MM cell lines after the cell cycle arrest, indicating that KDM5A is primarily required for MM cell proliferation rather than survival.

Deregulation of MYC is implicated in the pathogenesis of MM, and recent studies show that MYC selectively activates or represses its target genes, cooperating with other factors such as WDR5 and MIZ1 in a cancer-specific manner. Hence, the factors mediating cancer-specific MYC program could be ideal therapeutic targets in MYC-driven cancers.

To clarify the molecular mechanism whereby KDM5A mediates MM cell growth, the gene expression profile using RNA-seq after the treatment with pck82 in MM.1S cells was examined. Gene set enrichment analysis showed that pck82 decreased expression of MYC target genes. ChIP-seq analysis revealed that KDM5A not only co-localized with H3K4me3 mark, but also with MYC across the genome. The treatment with pck82 globally increased H3K4me3 mark but did not increase this mark at the loci of MYC and its targets whose expression is downregulated after pck82 treatment, suggesting that KDM5A may activate MYC targets in an enzymatically independent manner, and the pck82 may abrogate this transcriptional activation by binding KDM5A and interfering with KDM5A related-transcriptional complex.

### Lentiviral shRNA infection

pLKO.1-based plasmids for shRNAs were obtained from the RNAi Consortium (Broad Institute). The RNAi Consortium clone ID and target sequence of each vector are provided in *Table 4* below. The human *KDM5A* and *KDM5C* cDNA was amplified using PCR and ligated into the HpaI and XhoI sites of pMSCV retroviral expression vector (Clontech). Recombinant lentivirus was produced by transient transfection of 293T cells following a standard protocol. After 48 hours, MM cells were incubated with culture supernatants from 293T cells containing crude virus for 6 hours and washed with media. After 24h of infection, cells expressing shRNA were selected with puromycin dihydrochloride (Sigma-Aldrich) at 1-2 µg/ml for 48 h, and then examined for proliferation and/or subjected to immunoblotting analysis.

### Cell cycle assay

Cells were harvested and fixed with 70% ethanol for 20 min on ice. After washing with PBS twice, cells were incubated with 5 µg ml⁻¹ RNase (Roche) in PBS for 20 min at room temperature, and then resuspended in PBS containing 10 µg ml⁻¹ propidium iodide (Sigma-Aldrich). The stained cells were analysed with flow cytometry (BD FACSCanto II, BD Biosciences), and the percentage of cells in G1, S and G2/M phases was determined using the ModFit LT software (Verity Software House).

**Table 4. RNAi Consortium clone ID and target sequence of each vector.**

| Target Genes | Vectors | Clone IDs | Target Sequences |
|---|---|---|---|
| KDM5A | shKDM5A #1 | TRCN0000014629 | CCAGACTTACAGGGACACTTA |
| | shKDM5A #2 | TRCN0000014630 | CGGACCGACATTGGTGTATAT |
| | shKDM5A #3 | TRCN0000014631 | CCCATGCAGAAGAAATGTCTT |
| | shKDM5A #4 | TRCN0000014632 | CCTTGAAAGAAGCCTTACAAA |
| KDM5C | shKDM5C #1 | TRCN0000097856 | GCATTGTTTATCCCTATGAAA |
| | shKDM5C #2 | TRCN0000097857 | CGCATTGTTTATCCCTATGAA |
| | shKDM5C #3 | TRCN0000022084 | GCAGTGTAACACACGTCCATT |
| | shKDM5C #4 | TRCN0000022085 | CCCACTACGAACGCATTGTTT |

### Example 5. Exemplary Synthesis of KDM5 inhibitors

The compounds provided herein can be prepared from readily available starting materials using the following general methods and procedures or methods known in the art. It will be appreciated that where typical process conditions *(i.e.,* reaction temperatures, times, mole ratios of reactants, solvents, pressures, *etc.*) are given, other process conditions can also be used unless otherwise stated. Optimum reaction conditions may vary with the particular reactants or solvents used, but such conditions can be determined by those skilled in the art by routine optimization procedures.

Compounds of Formulae (**I**) or (**II**) may be prepared using the synthetic schemes and procedures described in detail below. In an exemplary synthesis, an exemplary KDM5 inhibitor, PCK62 (JADA62), was synthesized using the steps shown in *Scheme 1.*

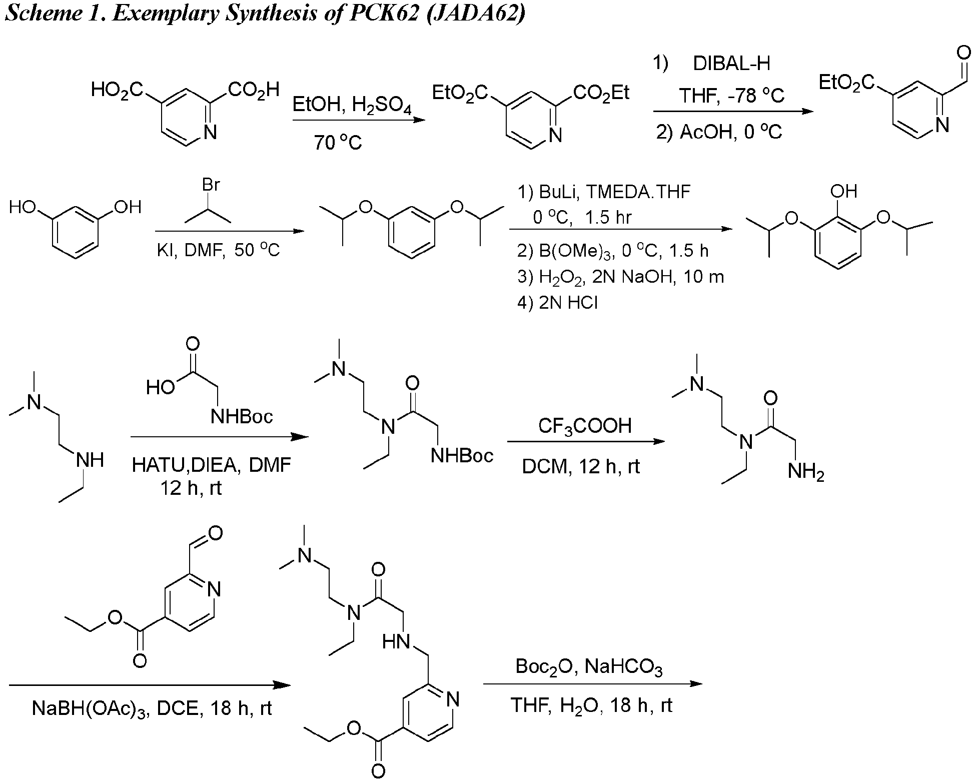

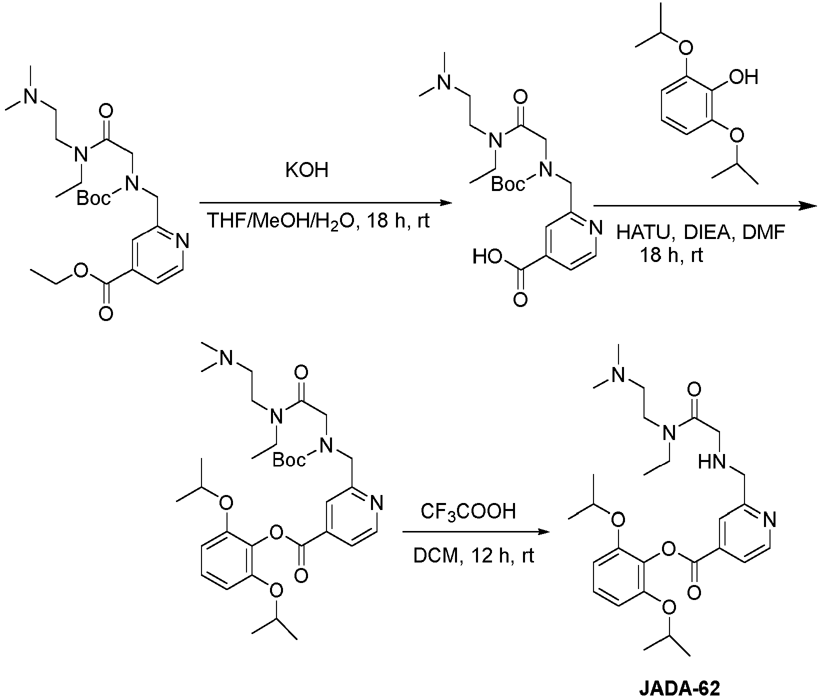

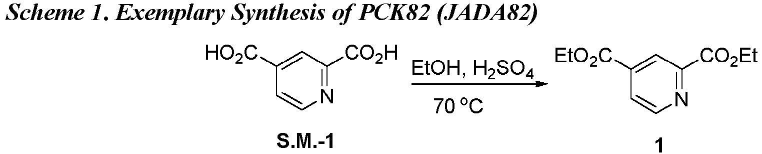

To a solution of S.M.-1 (5 g) in Ethanol (50 ml), Conc. H₂SO₄ (98%, 4 ml) was added dropwise, and the mixture was kept stirring for 14 hrs at 70 °C. After that, the reaction was cooled down to room temperature and poured into 100 ml 10% NaHCO₃ aqueous solution at 0 °C slowly. Then the mixture was extracted with ethyl acetate 100 ml x 2), the organic phase was washed with 10% NaHCO₃( 60 ml) , Brine (60 ml) , dried over Na₂SO₄. After evaporation of the solvent, the crude was triturated with Et₂O/hexane (20 ml/120 ml) at 0 °C for 4 hrs. Then the mixture was filtered and the filter cake was washed by cold Hexane, collected the solid and dried to give **1** (6 g as white solid). ¹H NMR (500 MHz CD₃OD) *δ* = 8.89 (d, *J =* 5.0 Hz, 1H), 8.62 (s, 1H), 8.02 (d, *J =* 5.0 Hz, 1H), 4.42-4.52 (m, 4H), 1.38-1.46 (m, 6H).

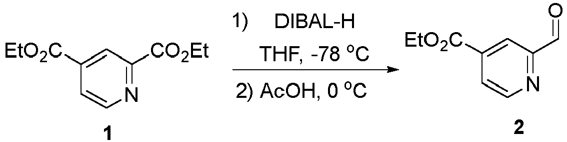

To a solution of **1** (3 g) in dry THF (60 ml), DIBAL-H (1M in THF, 16 ml, 1.2 equiv.) was added dropwise at -78 °C and kept the reaction stirring at 78 °C for 3 hrs, then the reaction was poured into ice-cold AcOH (10 ml) in water (10 ml) slowly and stirred for another 1 hr at r.t. The mixture was treated using 10% NaHCO₃ aqueous solution to pH=8, extracted with ethyl acetate (80 ml x 2), washed with 10% NaHCO₃ ( 60 ml), Brine (60 ml), dried over Na₂SO₄. After evaporation of the solvent, the crude was triturated hexane (50 ml) at r.t. for 2 hrs. Then the mixture was filtered and the filter cake was washed by cold Hexane, collected the solid and dried to give **2** 1.6 g as yellow-white solid. ¹H NMR (500 MHz CD₃OD) *δ* = 10.10 (s, 1H), 8.91 (d, *J=* 5.0 Hz, 1H), 8.43 (s, 1H), 8.06 (d, *J =* 5.0 Hz, 1H), 4.42 (q, *J =* 7.0 Hz, 2H), 1.38-1.46 (m, 6H).

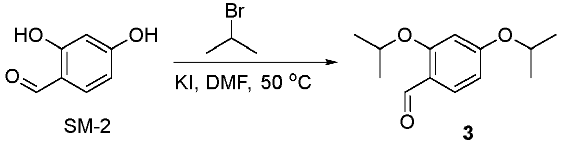

To a solution of SM-2 (4.2 g, 30.2 mmol) ,K₂CO₃ (16.7 g, 121 mmol) and KI (21.8 g, 121 mmol) in DMF (30 ml, 2-bromopropane was added dropwise, and the reaction was stirred at 50 °C for 24 hrs. Then the reaction was cooled down to rt and filtered, the filtrate was diluted with ethyl acetate (50 ml), washed with water (25 ml), brine (25 ml), dried over Na₂SO₄. After evaporation of the solvent, the crude was purified using silica gel chromatography (hexane/ethyl acetate=10/1~4/1) to yield the yellow oil **3** (2.1 g). ¹H NMR (500 MHz CD₃OD) *δ* = 10.21 (s, 1H), 7.82 (d, *J =* 5.0 Hz, 1H), 6.52 (m, 2H), 4.70-4.81 (m, 2H), 1.34 (d, *J =* 7.0 Hz, 3H), 1.28 (d, *J =* 7.0 Hz , 3H).

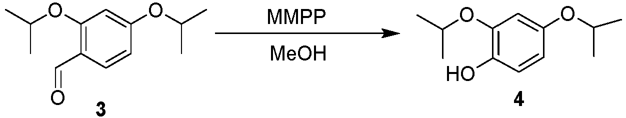

To a solution of **3** (1.95 g, 10 mmol) in dry MeOH (10 ml), Magnesium monoperoxyphthalate hexahydrate (MMPP) (3.5 g) was added and kept stirring for overnight at rt. After evaporation of the solvent, the crude was purified using silica gel chromatography (hexane/ethyl acetate=95/5) to yield the yellow oil **4** (400 mg). ¹H NMR (500 MHz, CD₃OD) *δ =* 6.81 (d, *J =* 5.0 Hz, 1H), 6.60 (s, 1H), 6.35 (d, *J =* 5.0 Hz, 1H), 4.50-4.56 (m, 1H), 4.45-4.52 (m, 1H), 1.34 (d, *J =* 7.0 Hz, 3H), 1.28 (d, *J =* 7.0 Hz , 3H).

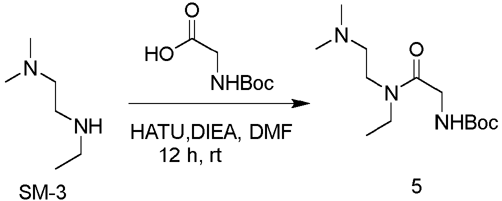

To a solution of **SM-3** (175 mg, 1 mmol) in dry DMF (0.5 ml), DIPEA (645 mg, 5 mmol) and HATU (466 mg, 1.2 mmol) were added, then (tert-butoxycarbonyl)glycine (116 mg, 1mmol) was added and the reaction was stirred for 16 hrs, then diluted with CH₂Cl₂ (50 ml) and washed with 1 N NaOH (20 ml). The aqueous phase was extracted with additional CH₂Cl₂ (2 x 30 mL).The organic phase was dried over Na₂SO4, filtered and concentrated to dryness. After evaporation of the solvent, the crude was purified on silica gel chromatography CH₂Cl₂/MeOH =100/0 ~ 80/20) to give oil **5** (150 mg).

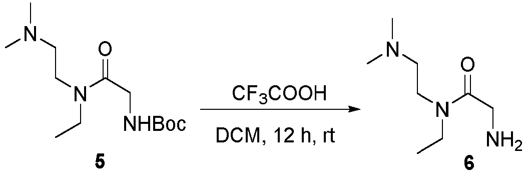

To a solution of **5** (150 mg) in DCM (4 ml), CF₃COOH (1 ml) was added and the reaction was stirred overnight. After that, the solvent was evaporated and the crude was purified by silica gel chromatography (CH₂Cl₂/MeOH =100/0 ~ 80/20) to give oil **6** (120 mg). ¹H NMR (500 MHz , *d₆*-DMSO) *δ* = 5.06 (bs, 4H), 3.52-3.20 (m, 6H), 2.45-2.28 (m, 2H), 2.22-2.12 (m, 6H).

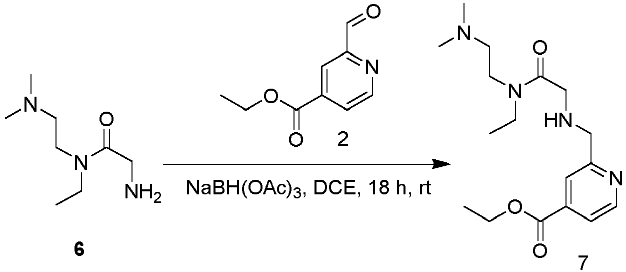

A solution of ethyl 2-formylisonicotinate **(2)** (0.26 g) and 2-amino-N-(2-(dimethylamino)ethyl)-*N*-ethylacetamide **(6)** (0.2 g) in 1,2-dichloroethane (DCE) (18 mL) was stirred at room temperature for 15 min, then NaBH(AcO)₃ (400 mg) was added in one portion. The solution was stirred at room temperature for 16 h, then 10 ml NH₄Cl aqueous solution was added, the layers separated, and the organic phase washed. After that, the solvent was evaporated and the crude was purified by silica gel chromatography (CH₂Cl₂/MeOH =100/0 ~ 80/20) to give oil **7** (120 mg). ¹H NMR (500 MHz, CD₃OD) *δ* = 8.71 (dd, *J =* 5.0, 1.0 Hz, 1H), 8.01 (dd, *J* = 2.0, 1.0 Hz, 1H), 7.82 (dd, *J =* 5.0, 1.0 Hz, 1H), 4.44 (q, *J =* 7.0 Hz, 2H), 4.02 (s, 2H), 3.58 (s, 2H), 3.56 - 3.34 (m, 2H), 2.63 - 2.45 (m, 4H), 2.42 (s, 3H), 2.32 (s, 3H), 1.42 (t, *J* = 7.0 Hz, 3H), 1.16 (t, *J =* 7.2 Hz, 3H).

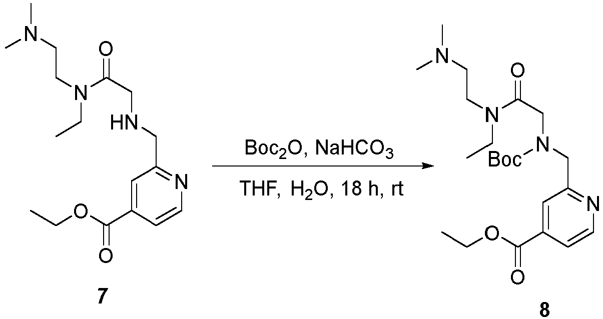

To the solution of **7** ( 120 mg) , THF(20 ml) and NaHCO₃ aq.(10 ml) were added, then Boc₂O (300 mg) was added and the reaction was stirred overnight. Then extracted with ethyl acetate (100 ml x 2) and the solvent was evaporated and the crude was purified by silica gel chromatography (CH₂Cl₂/MeOH =100/0 ~ 90/10) to give oil **8** (60 mg). ¹H NMR (500 MHz , *d6-DMSO): δ* = 8.50-8.30 (m, 1H), 7.71-7.43 (m, 2H), 4.44 (q, *J =* 7.0 Hz, 2H), 4.40-4.38 (m, 2H), 4.22-4.03 (m, 2H), 3.63-3.55 (m, 2H), 3.39-3.25 (m, 2H), 2.97-2.86 (m, 2H), 2.62-2.54 (m, 6H), 1.42 (t, *J =* 7.0 Hz, 3H),1.18-0.99 (m, 3H).

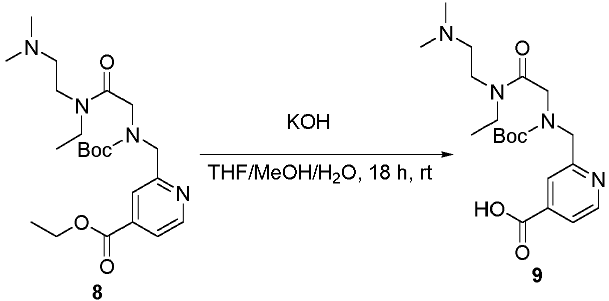

A solution of **8** (60 mg,) in a 1:1:1 mixture of THF/MeOH/H₂O (3 mL) was treated with KOH (142 mg). The solution was stirred at room temperature for 16 h, then the solution was adjusted to pH 3 with 1N HCl, extracted with ethyl acetate (100 ml x 2) and the crude product was purified by silica gel chromatography (CH₂Cl₂/MeOH =100/0 ~ 90/10), affording pure product **9** (30 mg). ¹H NMR (500 MHz, *d6*-DMSO): *δ* = 8.56-8.31 (m, 1H), 7.73-7.41 (m, 2H), 4.50-4.38 (m, 2H), 4.22-4.03 (m, 2H), 3.63-3.55 (m, 2H), 3.39-3.25 (m, 2H), 2.97-2.86 (m, 2H), 2.62-2.54 (m, 6H), 1.41-1.19 (m, 9H), 1.18-0.99 (m, 3H).

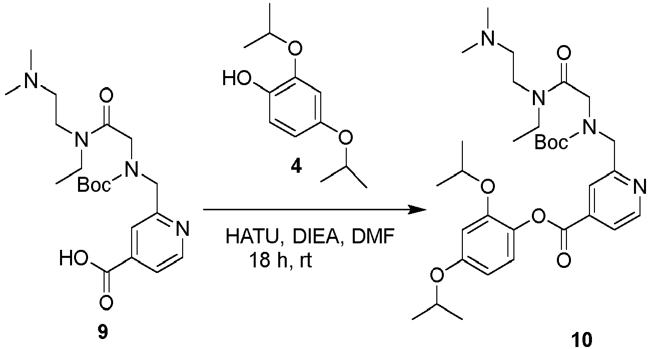

To a solution of **9** (30 mg, 0.075 mmol) in DMF (0.5 ml), DIPEA (96 mg, 0.75 mmol), and HATU (57 mg, 0.15 mmol) were added, then **4** was added and the reaction was stirred for 16 hrs at 50 °C, the crude product was purified by silica gel chromatography (CH₂Cl₂/MeOH =100/0 ~ 90/10), affording pure product **10** (18 mg). ¹H NMR (500 MHz , CD3OD): *δ* = 8.89 (d, *J =* 5.0 Hz 1H), 8.12 (s, 1H), 8.00 (d, *J =* 5.0 Hz 1H), 7.00 (d, *J =* 9.0 Hz, 1H), 6.58 (s, 1H), 6.35 (d, *J =* 9.0 Hz, 1H), 4.50-4.28 (m, 6H), 3.81-3.78 (m, 2H), 3.42-3.38 (m, 4H), 2.92 (s, 6H), 1.49 (s, 9H), 1.31 (d, *J =* 6.0 Hz, 3H), 1.24 (m, 3H), 1.20 (d, *J =* 6.0 Hz, 3H).

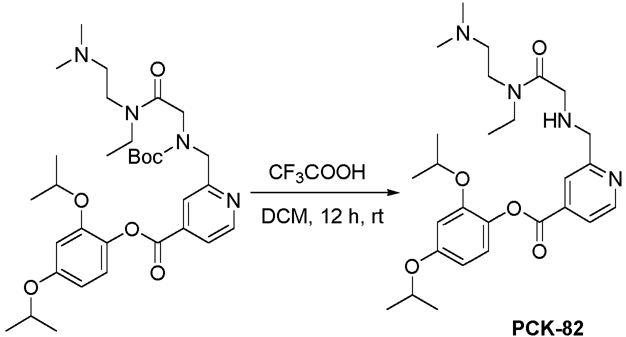

To a solution of **10** (18 mg) in CH₂Cl₂ (2 ml), CF₃COOH (0.5 ml) was added and the reaction was stirred overnight. NaHCO₃ aq. was added to adjust to pH=9, and the mixture extracted with ethyl acetate (100 ml x 2 and the solvent was evaporated and the crude was purified by silica gel chromatography (CH₂Cl₂/MeOH =100/0 ~ 90/10) to give oil **PCK-82**(10 mg). ¹H NMR (500 MHz , CD3OD): *δ* = 8.92 (d, *J =* 5.0 Hz 1H), 8.12 (s, 1H), 8.02 (d, *J =* 5.0 Hz 1H), 7.02 (d, *J =* 9.0 Hz, 1H), 6.60 (s, 1H), 6.35 (d, *J =* 9.0 Hz, 1H), 4.50-4.38 (m, 4H), 4.23 (s, 2H), 3.81-3.78 (m, 2H), 3.42-3.38 (m, 4H), 2.92 (s, 6H), 1.31 (d, *J =* 6.0 Hz, 3H), 1.24 (t, *J* = 7.5 Hz, 3H), 1.20 (d, *J =* 6.0 Hz, 3H).

### Example 6. In vivo mouse study

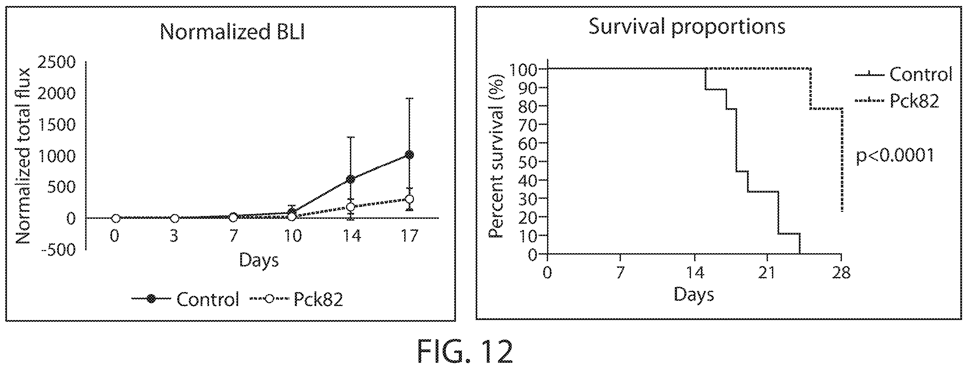

Mice were injected intravenously with luciferized molp8 cells. Half million cells per mice. After 1 week, the tumor was observed using bioluminescence imaging (BLI). The normalized total flux was measured over 17 days (FIG. 12; *Table 5*) Mice were randomized into control and treatment. Treatment lasted for 21 days. Animals with 15% weight loss was terminated.

**Table 5. Normalized total flux from BLI imaging**

| | Day | | | | | | |
|---|---|---|---|---|---|---|---|
| Cage, EP | 0 | 3 | 7 | 10 | 14 | 17 | 21 |
| Control-1 EP#0 | 1 | 0.04431 | 23.965517 | 3.2413793 | 140.689655 | 1032.75862 | dead |
| Control-1 EP#3 | 1 | 0.055709 | 0.2529412 | 139.44637 | 889.273356 | 2138.4083 | dead |
| Control-1 EP#10 | 1 | 0.239466 | 31.454006 | 4.1543027 | 146.884273 | 1551.92878 | dead |
| Control-1 EP#30 | 1 | 0.067811 | 46.781116 | 209.87124 | 1038.62661 | 2321.88841 | dead |
| Control-2 EP#0 | 1 | 5.958549 | 0.6062176 | 223.31606 | 1606.21762 | 311.917098 | 2535.49223 |
| Control-2 EP#1 | 1 | 4.917582 | 29.010989 | 228.02198 | 1615.38462 | 11.3186813 | 65.8708791 |
| Control-2 EP#3 | 1 | 8.057325 | 0.2314225 | 33.014862 | 82.4416136 | dead | dead |
| Control-2 EP#10 | 1 | 3.573446 | 30.225989 | 67.79661 | 126.186441 | 605.579096 | dead |
| Control-2 EP#30 | 1 | 3.039683 | 19.603175 | 49.761905 | 18.6388889 | 163.293651 | 8.68690476 |
| Pck82-1 EP#0 | 1 | 3.763676 | 0.5645514 | 6.6301969 | 191.684902 | 306.345733 | 44.9956236 |
| Pck82-1 EP#1 | 1 | 4.5 | 9.7222222 | 19.333333 | 165.555556 | 4.86111111 | 3.40416667 |
| Pck82-1 EP#3 | 1 | 4.255319 | 8.5460993 | 0.4219858 | 135.106383 | 358.156028 | 435.531915 |
| Pck82-1 EP#10 | 1 | 3.625 | 8.65625 | 44.375 | 93.75 | 465.625 | 58.65625 |
| Pck82-1 EP#30 | 1 | 2.035088 | 13.508772 | 23.377193 | 380.263158 | 258.77193 | 1309.21053 |
| Pck82-2 EP#0 | 1 | 2.29646 | 1.6769912 | 25.973451 | 314.159292 | 133.185841 | 1223.89381 |
| Pck82-2 EP# 1 | 1 | 3.238095 | 8.1693122 | 24.973545 | 275.132275 | 277.248677 | 847.354497 |
| Pck82-2 EP#10 | 1 | 0.214868 | 0.2301426 | 1.0285132 | 75.6619145 | 358.961303 | 121.639511 |
| Pck82-2 EP#30 | 1 | 0.333858 | 39.370079 | 6.2204724 | 5.65354331 | 565.551181 | 1078.14961 |

### EQUIVALENTS AND SCOPE

In the claims articles such as "a," "an," and "the" may mean one or more than one unless indicated to the contrary or otherwise evident from the context. Claims or descriptions that include "or" between one or more members of a group are considered satisfied if one, more than one, or all of the group members are present in, employed in, or otherwise relevant to a given product or process unless indicated to the contrary or otherwise evident from the context. The disclosure includes embodiments in which exactly one member of the group is present in, employed in, or otherwise relevant to a given product or process. The disclosure includes embodiments in which more than one, or all of the group members are present in, employed in, or otherwise relevant to a given product or process.

Furthermore, the disclosure encompasses all variations, combinations, and permutations in which one or more limitations, elements, clauses, and descriptive terms from one or more of the listed claims is introduced into another claim. For example, any claim that is dependent on another claim can be modified to include one or more limitations found in any other claim that is dependent on the same base claim. Where elements are presented as lists, *e.g.,* in Markush group format, each subgroup of the elements is also disclosed, and any element(s) can be removed from the group. It should it be understood that, in general, where the disclosure, or aspects described herein, is/are referred to as comprising particular elements and/or features, certain embodiments described herein or aspects described herein consist, or consist essentially of, such elements and/or features. For purposes of simplicity, those embodiments have not been specifically set forth *in haec verba* herein. It is also noted that the terms "comprising" and "containing" are intended to be open and permits the inclusion of additional elements or steps. Where ranges are given, endpoints are included. Furthermore, unless otherwise indicated or otherwise evident from the context and understanding of one of ordinary skill in the art, values that are expressed as ranges can assume any specific value or sub-range within the stated ranges in different embodiments described herein, to the tenth of the unit of the lower limit of the range, unless the context clearly dictates otherwise.

This application refers to various issued patents, published patent applications, journal articles, and other publications. In addition, any particular embodiment of the present disclosure that falls within the prior art may be explicitly excluded from any one or more of the claims. Because such embodiments are deemed to be known to one of ordinary skill in the art, they may be excluded even if the exclusion is not set forth explicitly herein. Any particular embodiment described herein can be excluded from any claim, for any reason, whether or not related to the existence of prior art.

Those skilled in the art will recognize or be able to ascertain using no more than routine experimentation many equivalents to the specific embodiments described herein. The scope of the present embodiments described herein is not intended to be limited to the above Description, but rather is as set forth in the appended claims.

## Claims

1. A compound of Formula (I-B): 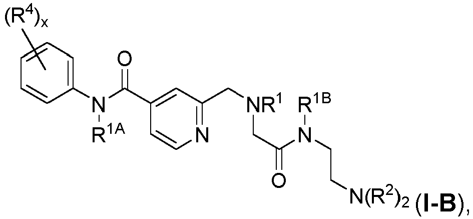 or a pharmaceutically acceptable salt, solvate, hydrate, polymorph, co-crystal, tautomer, stereoisomer, or isotopically labeled derivative thereof, wherein:
R¹ is hydrogen, optionally substituted alkyl, or a nitrogen protecting group;
R^{1A} is hydrogen, optionally substituted acyl, optionally substituted alkyl, optionally substituted carbocyclyl, or a nitrogen protecting group;
R^{1B} is hydrogen, optionally substituted alkyl, optionally substituted carbocyclyl, or a nitrogen protecting group;
each instance of R² is independently optionally substituted alkyl or a nitrogen protecting group;
each R⁴ is independently optionally substituted alkyl, optionally substituted alkenyl, optionally substituted alkynyl, optionally substituted carbocyclyl, optionally substituted heterocyclyl, optionally substituted aryl, optionally substituted heteroaryl, -OR^{A}, -N(R^{B})₂, or -SR^{A}, or optionally two instances of R⁴ are taken together with their intervening atoms to form a substituted or unsubstituted heterocyclic or substituted or unsubstituted heteroaryl ring; and
x is 0, 1, 2, 3, 4, or 5;
wherein the nitrogen protecting group is Bn, BOC, Cbz, Fmoc, trifluoroacetyl, triphenylmethyl, acetyl, or Ts.

2. The compound of claim 1, or a pharmaceutically acceptable salt, solvate, hydrate, polymorph, co-crystal, tautomer, stereoisomer, or isotopically labeled derivative, thereof, which is 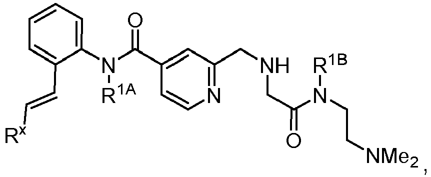 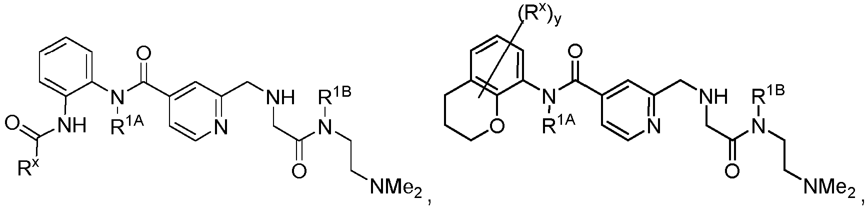 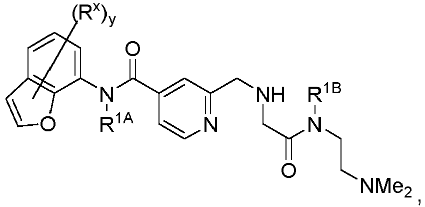 or 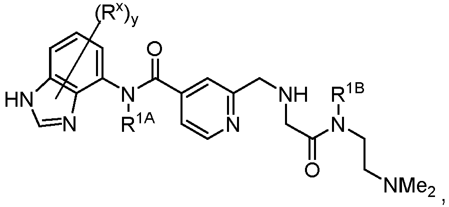 wherein:
R^{x} is optionally substituted acyl, optionally substituted alkyl, -O(optionally substituted alkyl), or -NO₂; and
y is 0, 1, 2, 3, 4, 5, 6, 7, 8, or 9, as valency permits.

3. The compound of claim 1, or a pharmaceutically acceptable salt, solvate, hydrate, polymorph, co-crystal, tautomer, stereoisomer, or isotopically labeled derivative thereof, wherein at least one instance of R⁴ is optionally substituted alkenyl, wherein at least one instance of R⁴ is -OR^{A}, and R^{A} is hydrogen, optionally substituted C₁₋₆ alkyl, or optionally substituted aryl, or wherein at least one instance of R⁴ is -NHC(=O)R^{x}, and R^{x} is optionally substituted C₁₋₆ alkyl or optionally substituted alkenyl.

4. The compound of any one of claims 1 to 3, or a pharmaceutically acceptable salt, solvate, hydrate, polymorph, co-crystal, tautomer, stereoisomer, or isotopically labeled derivative, thereof, wherein x is 0, 1 or 2.

5. The compound of any one of claims 1 to 4, or a pharmaceutically acceptable salt, solvate, hydrate, polymorph, co-crystal, tautomer, stereoisomer, or isotopically labeled derivative thereof, wherein R¹ is hydrogen, or
R^{1B} is optionally substituted C₁₋₆ alkyl or optionally substituted carbocyclyl; or
at least one instance of R² is optionally substituted C₁₋₆ alkyl; or
at least one instance of R² is unsubstituted methyl; or
both instances of R² are unsubstituted methyl.

6. The compound of any one of claims 1 to 4, or a pharmaceutically acceptable salt, solvate, hydrate, polymorph, co-crystal, tautomer, stereoisomer, or isotopically labeled derivative thereof, wherein
R' is hydrogen and R^{1B} is optionally substituted C₁₋₆ alkyl or optionally substituted carbocyclyl; or
R¹ is hydrogen, R^{1B} is optionally substituted C₁₋₆ alkyl or optionally substituted carbocyclyl and at least one instance of R² is optionally substituted C₁₋₆ alkyl.

7. The compound of any one of claims 1 to 4, or a pharmaceutically acceptable salt, solvate, hydrate, polymorph, co-crystal, tautomer, stereoisomer, or isotopically labeled derivative thereof, wherein
R¹ is hydrogen, R^{1B} is optionally substituted C₁₋₆ alkyl or optionally substituted carbocyclyl and at least one instance of R² is unsubstituted methyl; or
R¹ is hydrogen, R^{1B} is optionally substituted C₁₋₆ alkyl or optionally substituted carbocyclyl and both instances of R² are unsubstituted methyl.

8. A compound which is: 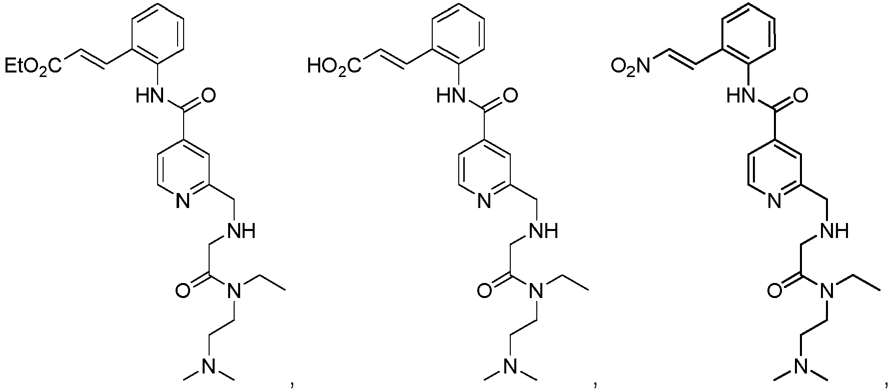 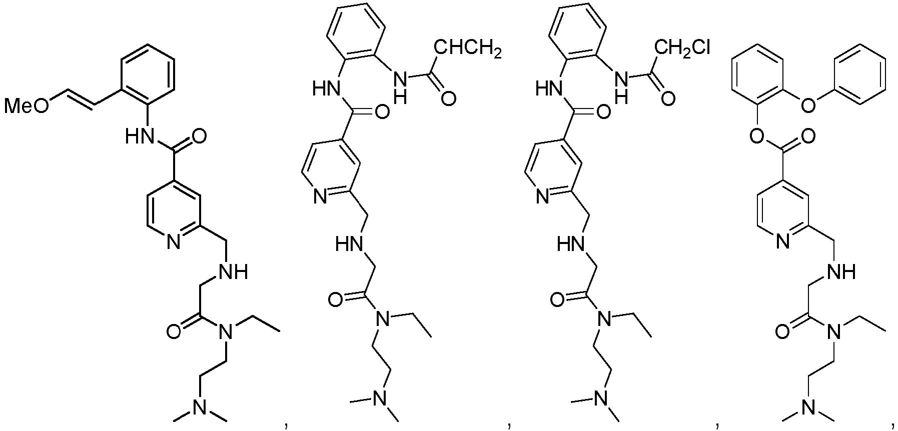 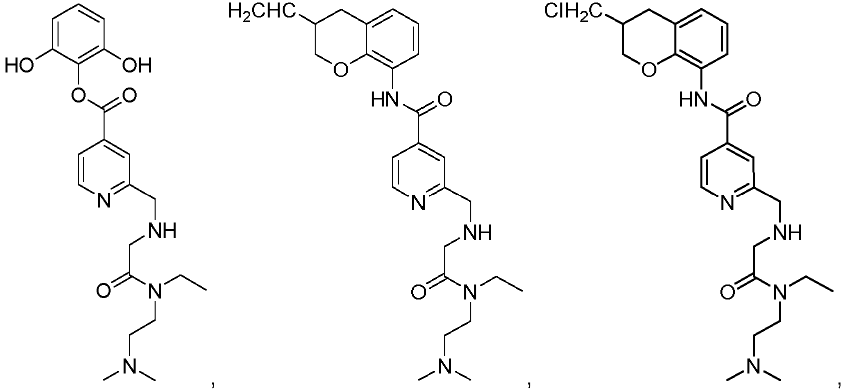 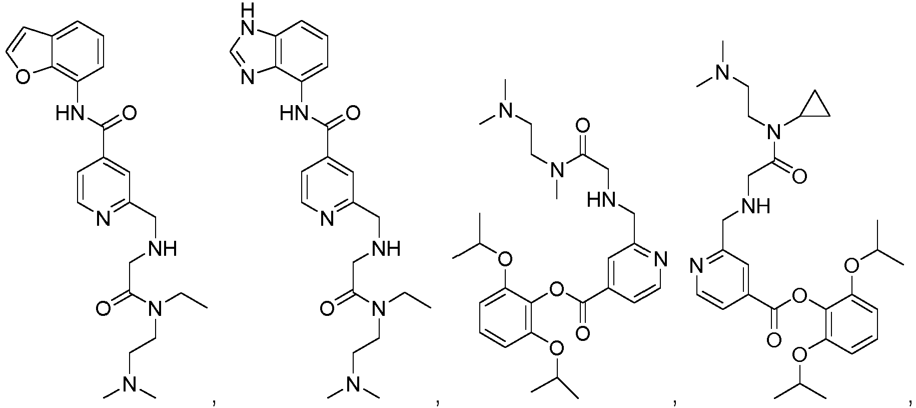 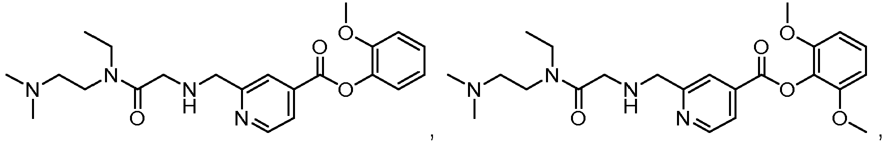 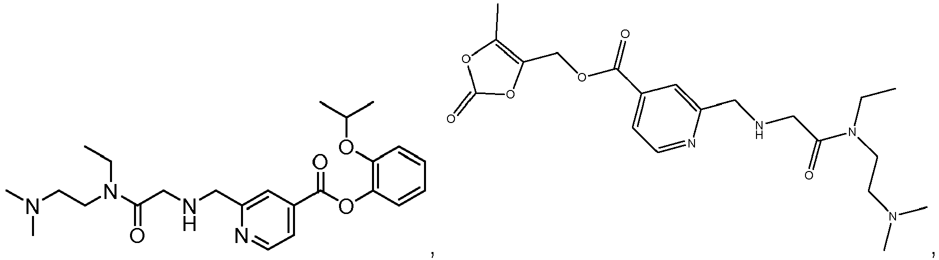 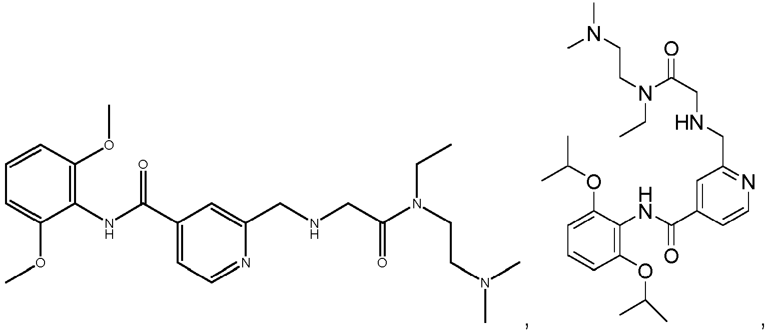 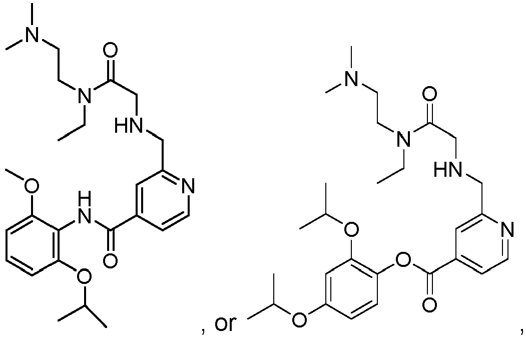 or a pharmaceutically acceptable salt, solvate, hydrate, polymorph, co-crystal, tautomer, stereoisomer, or isotopically labeled derivative thereof.

9. The compound of claim 8, which is: 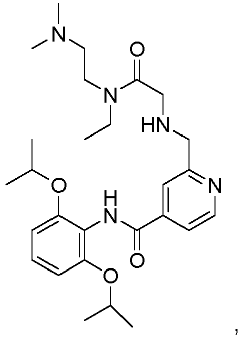 or a pharmaceutically acceptable salt thereof.

10. The compound of claim 8, which is: 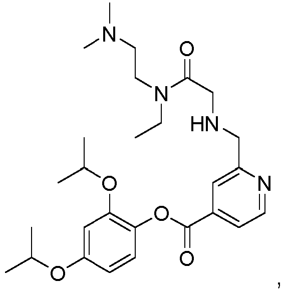 or a pharmaceutically acceptable salt thereof.

11. A compound of Formula (**II**): 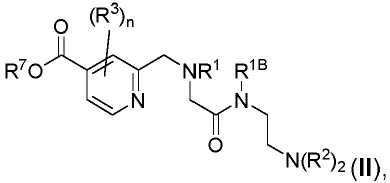 or a pharmaceutically acceptable salt, solvate, hydrate, polymorph, co-crystal, tautomer, stereoisomer, or isotopically labeled derivative, wherein:
R¹ is hydrogen, optionally substituted alkyl, or a nitrogen protecting group;
R^{1B} is hydrogen, methyl , optionally substituted carbocyclyl, or a nitrogen protecting group;
each instance of R² is independently optionally substituted alkyl or a nitrogen protecting group;
each instance of R³ is independently halogen, optionally substituted acyl, optionally substituted alkyl, optionally substituted alkenyl, optionally substituted alkynyl, optionally substituted carbocyclyl, optionally substituted heterocyclyl,
optionally substituted aryl, optionally substituted heteroaryl, -CN, -SCN, - NO₂, -N₃, -OR^{A}, -N(R^{B})₂, or -SR^{A};
each instance of R^{A} is independently hydrogen, optionally substituted acyl, optionally substituted alkyl, optionally substituted alkenyl, optionally substituted alkynyl, optionally substituted carbocyclyl, optionally substituted heterocyclyl, optionally substituted aryl, optionally substituted heteroaryl;
each instance of R^{B} is independently hydrogen, optionally substituted acyl, optionally substituted alkyl, optionally substituted alkenyl, optionally substituted alkynyl, optionally substituted carbocyclyl, optionally substituted heterocyclyl, optionally substituted aryl, optionally substituted heteroaryl, or a nitrogen protecting group; or optionally two instances of R^{B} are taken together with their intervening atoms to form a substituted or unsubstituted heterocyclic or substituted or unsubstituted heteroaryl ring;
n is 0, 1, 2, or 3; and
R⁷ is ethyl or propyl;
wherein the nitrogen protecting group is Bn, BOC, Cbz, Fmoc, trifluoroacetyl, triphenylmethyl, acetyl, or Ts;
provided that the compound is not of the formula:

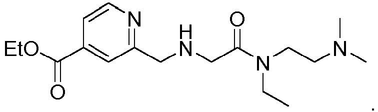

12. The compound of claim 11, wherein the compound is of the formula: 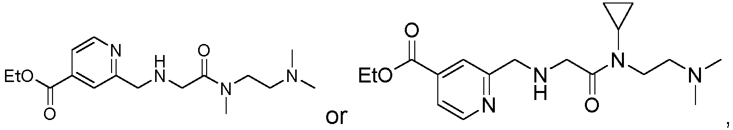 or a pharmaceutically acceptable salt, solvate, hydrate, polymorph, co-crystal, tautomer, stereoisomer, or isotopically labeled derivative thereof.

13. A pharmaceutical composition comprising a compound of any one of claims 1-12, or a pharmaceutically acceptable salt, solvate, hydrate, polymorph, co-crystal, tautomer, stereoisomer, or isotopically labeled derivative, and optionally a pharmaceutically acceptable excipient.

14. The compound of any one of claims 1 to 12, or the pharmaceutical composition of claim 13 for use in treating a proliferative disease in which KDM5 plays a role, wherein a therapeutically effective amount of said compound or pharmaceutical composition is to be administered to a subject.

15. The compound for use or the pharmaceutical composition for use of claim 14, wherein the proliferative disease is a cancer selected from a carcinoma, a sarcoma, lung cancer, breast cancer, liver cancer, pancreatic cancer, gastric cancer, ovarian cancer, colon cancer, leukemia, and multiple myeloma, wherein the lung cancer is non-small cell lung cancer, and the sarcoma is Ewing sarcoma.

## Patentansprüche

1. Verbindung der Formel **(I-B):** 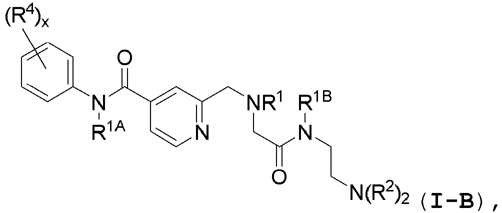 oder ein pharmazeutisch verträgliches/r Salz, Solvat, Hydrat, Polymorph, Co-Kristall, Tautomer, Stereoisomer oder isotopenmarkiertes Derivat davon, wobei:
R¹ Wasserstoff, gegebenenfalls substituiertes Alkyl oder eine Stickstoffschutzgruppe ist;
R^{1A} Wasserstoff, gegebenenfalls substituiertes Acyl, gegebenenfalls substituiertes Alkyl, gegebenenfalls substituiertes Carbocyclyl oder eine Stickstoffschutzgruppe ist;
R^{1B} Wasserstoff, gegebenenfalls substituiertes Alkyl, gegebenenfalls substituiertes Carbocyclyl oder eine Stickstoffschutzgruppe ist;
jedes Vorkommen von R² unabhängig gegebenenfalls substituiertes Alkyl oder eine Stickstoffschutzgruppe ist;
jedes R⁴ unabhängig gegebenenfalls substituiertes Alkyl, gegebenenfalls substituiertes Alkenyl, gegebenenfalls substituiertes Alkinyl, gegebenenfalls substituiertes Carbocyclyl, gegebenenfalls substituiertes Heterocyclyl, gegebenenfalls substituiertes Aryl, gegebenenfalls substituiertes Heteroaryl, -OR^{A}, -N(R^{B})₂ oder -SR^{A} ist, oder gegebenenfalls zwei Vorkommen von R⁴ mit den dazwischenliegenden Atomen zusammengenommen werden, um einen substituierten oder unsubstituierten heterocyclischen Ring oder einen substituierten oder unsubstituierten Heteroarylring zu bilden; und
x 0, 1, 2, 3, 4 oder 5 ist;
wobei die Stickstoffschutzgruppe Bn, BOC, Cbz, Fmoc, Trifluoracetyl, Triphenylmethyl, Acetyl oder Ts ist.

2. Verbindung gemäß Anspruch 1 oder ein pharmazeutisch verträgliches/r Salz, Solvat, Hydrat, Polymorph, Co-Kristall, Tautomer, Stereoisomer oder isotopenmarkiertes Derivat davon, welche: 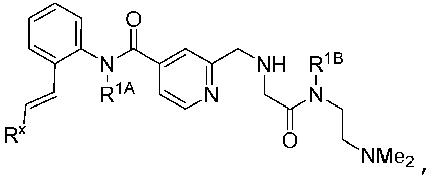 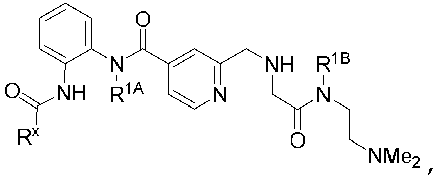 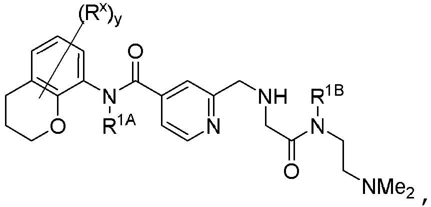 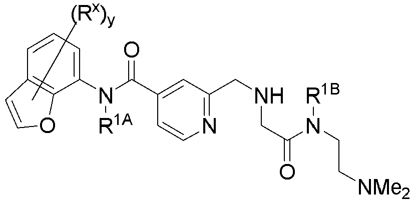 oder 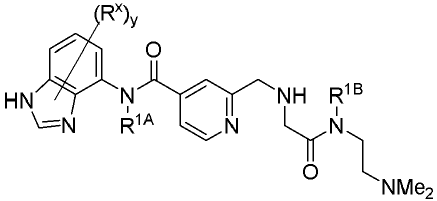 ist, wobei:
R^{x} gegebenenfalls substituiertes Acyl, gegebenenfalls substituiertes Alkyl, -O (gegebenenfalls substituiertes Alkyl) oder -NO₂ ist; und
y 0, 1, 2, 3, 4, 5, 6, 7, 8 oder 9 ist, soweit die Valenz dies zulässt.

3. Verbindung gemäß Anspruch 1 oder ein pharmazeutisch verträgliches/r Salz, Solvat, Hydrat, Polymorph, Co-Kristall, Tautomer, Stereoisomer oder isotopenmarkiertes Derivat davon, wobei mindestens ein Vorkommen von R⁴ gegebenenfalls substituiertes Alkenyl ist, wobei mindestens ein Vorkommen von R⁴ -OR^{A} ist, und R^{A} Wasserstoff, gegebenenfalls substituiertes C₁₋₆-Alkyl oder gegebenenfalls substituiertes Aryl ist, oder wobei mindestens ein Vorkommen von R⁴ -NHC(=O)R^{x} ist, und R^{x} gegebenenfalls substituiertes C₁₋₆-Alkyl oder gegebenenfalls substituiertes Alkenyl ist.

4. Verbindung gemäß einem der Ansprüche 1 bis 3 oder ein pharmazeutisch verträgliches/r Salz, Solvat, Hydrat, Polymorph, Co-Kristall, Tautomer, Stereoisomer oder isotopenmarkiertes Derivat davon, wobei x 0, 1 oder 2 ist.

5. Verbindung gemäß einem der Ansprüche 1 bis 4 oder ein pharmazeutisch verträgliches/r Salz, Solvat, Hydrat, Polymorph, Co-Kristall, Tautomer, Stereoisomer oder isotopenmarkiertes Derivat davon, wobei R¹ Wasserstoff ist, oder
R^{1B} gegebenenfalls substituiertes C₁₋₆-Alkyl oder gegebenenfalls substituiertes Carbocyclyl ist; oder
mindestens ein Vorkommen von R² gegebenenfalls substituiertes C₁₋₆-Alkyl ist; oder
mindestens ein Vorkommen von R² unsubstituiertes Methyl ist; oder
beide Vorkommen von R² unsubstituiertes Methyl sind.

6. Verbindung gemäß einem der Ansprüche 1 bis 4 oder ein pharmazeutisch verträgliches/r Salz, Solvat, Hydrat, Polymorph, Co-Kristall, Tautomer, Stereoisomer oder isotopenmarkiertes Derivat davon, wobei
R¹ Wasserstoff ist und R^{1B} gegebenenfalls substituiertes C₁₋₆-Alkyl oder gegebenenfalls substituiertes Carbocyclyl ist; oder
R¹ Wasserstoff ist, R^{1B} gegebenenfalls substituiertes C₁₋₆-Alkyl oder gegebenenfalls substituiertes Carbocyclyl ist und mindestens ein Vorkommen von R² gegebenenfalls substituiertes C₁₋₆-Alkyl ist.

7. Verbindung gemäß einem der Ansprüche 1 bis 4 oder ein pharmazeutisch verträgliches/r Salz, Solvat, Hydrat, Polymorph, Co-Kristall, Tautomer, Stereoisomer oder isotopenmarkiertes Derivat davon, wobei
R¹ Wasserstoff ist, R^{1B} gegebenenfalls substituiertes C₁₋₆-Alkyl oder gegebenenfalls substituiertes Carbocyclyl ist und mindestens ein Vorkommen von R² unsubstituiertes Methyl ist; oder
R¹ Wasserstoff ist, R^{1B} gegebenenfalls substituiertes C₁₋₆-Alkyl oder gegebenenfalls substituiertes Carbocyclyl ist und beide Vorkommen von R² unsubstituiertes Methyl sind.

8. Verbindung, welche: 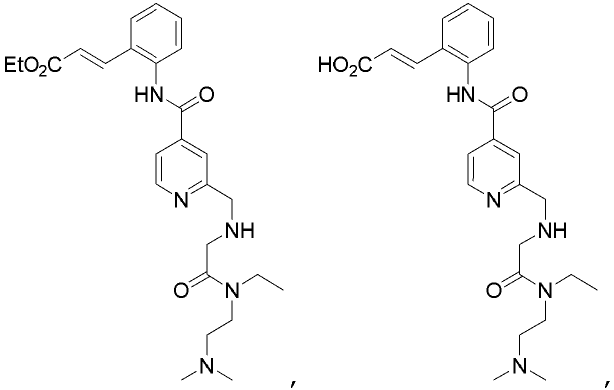 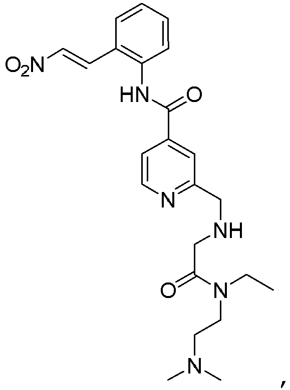 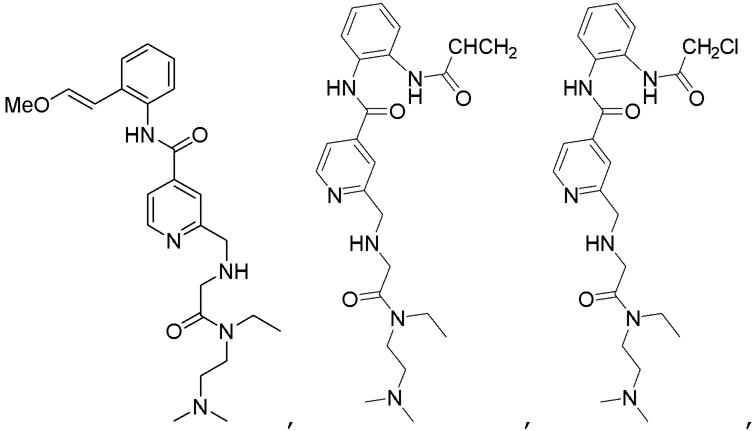 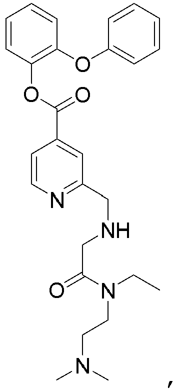 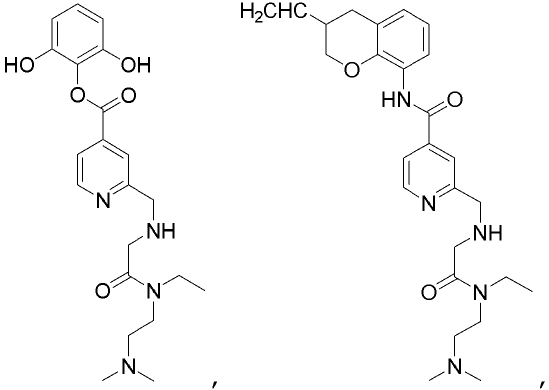 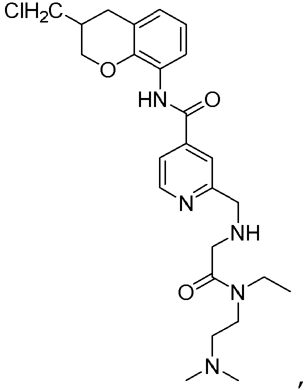 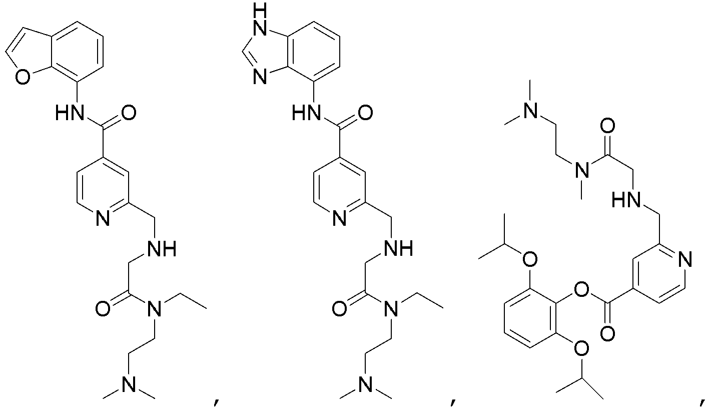 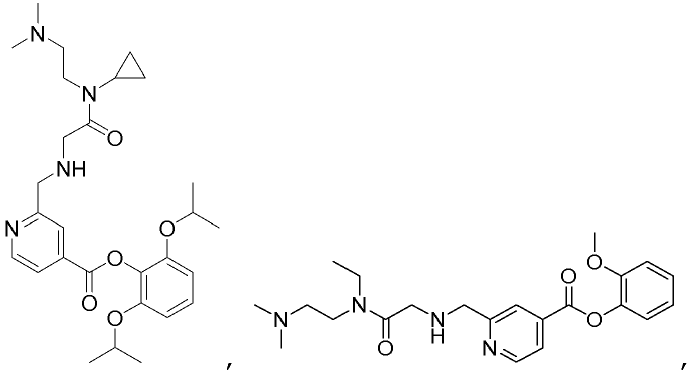 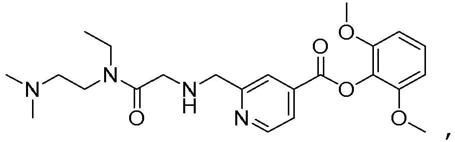 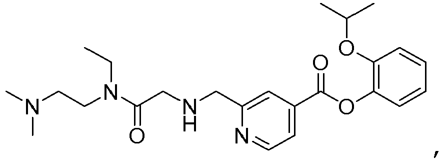 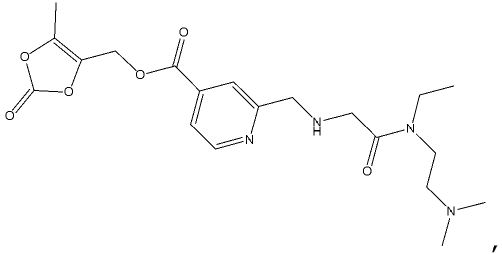 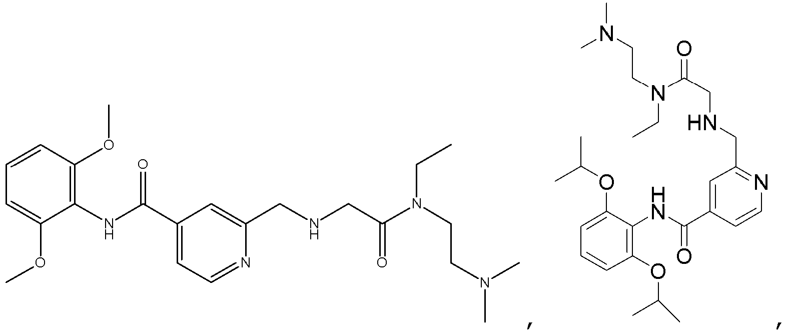 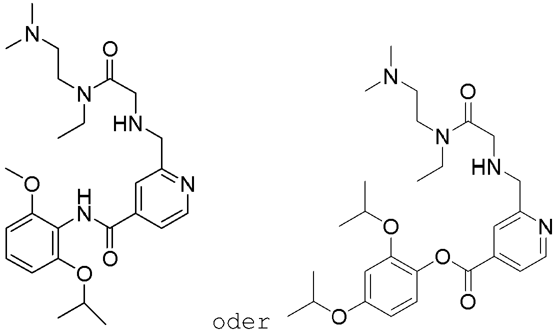 ist, oder ein pharmazeutisch verträgliches/r Salz, Solvat, Hydrat, Polymorph, Co-Kristall, Tautomer, Stereoisomer oder isotopenmarkiertes Derivat davon.

9. Verbindung gemäß Anspruch 8, welche: 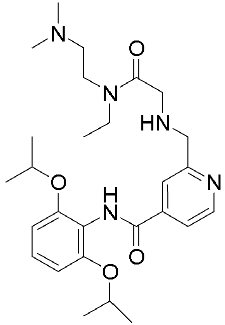 ist, oder ein pharmazeutisch verträgliches Salz davon.

10. Verbindung gemäß Anspruch 8, welche: 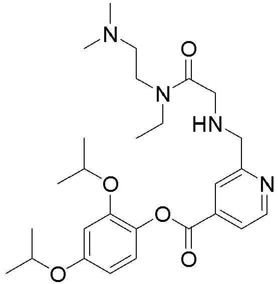 ist, oder ein pharmazeutisch verträgliches Salz davon.

11. Verbindung der Formel **(II):** 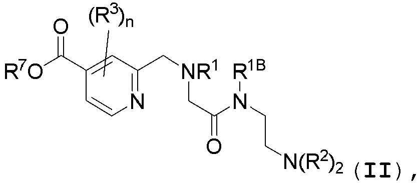 oder ein pharmazeutisch verträgliches/r Salz, Solvat, Hydrat, Polymorph, Co-Kristall, Tautomer, Stereoisomer oder isotopenmarkiertes Derivat, wobei:
R¹ Wasserstoff, gegebenenfalls substituiertes Alkyl oder eine Stickstoffschutzgruppe ist;
R^{1B} Wasserstoff, Methyl, gegebenenfalls substituiertes Carbocyclyl oder eine Stickstoffschutzgruppe ist;
jedes Vorkommen von R² unabhängig gegebenenfalls substituiertes Alkyl oder eine Stickstoffschutzgruppe ist;
jedes Vorkommen von R³ unabhängig Halogen, gegebenenfalls substituiertes Acyl, gegebenenfalls substituiertes Alkyl, gegebenenfalls substituiertes Alkenyl, gegebenenfalls substituiertes Alkinyl, gegebenenfalls substituiertes Carbocyclyl, gegebenenfalls substituiertes Heterocyclyl, gegebenenfalls substituiertes Aryl, gegebenenfalls substituiertes Heteroaryl, -CN, -SCN, -NO₂, -N₃, -OR^{A}, - N(R^{B})₂ oder -SR^{A} ist;
jedes Vorkommen von R^{A} unabhängig Wasserstoff, gegebenenfalls substituiertes Acyl, gegebenenfalls substituiertes Alkyl, gegebenenfalls substituiertes Alkenyl, gegebenenfalls substituiertes Alkinyl, gegebenenfalls substituiertes Carbocyclyl, gegebenenfalls substituiertes Heterocyclyl, gegebenenfalls substituiertes Aryl, gegebenenfalls substituiertes Heteroaryl ist;
jedes Vorkommen von R^{B} unabhängig Wasserstoff, gegebenenfalls substituiertes Acyl, gegebenenfalls substituiertes Alkyl, gegebenenfalls substituiertes Alkenyl, gegebenenfalls substituiertes Alkinyl, gegebenenfalls substituiertes Carbocyclyl, gegebenenfalls substituiertes Heterocyclyl, gegebenenfalls substituiertes Aryl, gegebenenfalls substituiertes Heteroaryl oder eine Stickstoffschutzgruppe ist; oder gegebenenfalls zwei Vorkommen von R^{B} mit den dazwischenliegenden Atomen zusammengenommen werden, um einen substituierten oder unsubstituierten heterocyclischen Ring oder einen substituierten oder unsubstituierten Heteroarylring zu bilden;
n 0, 1, 2 oder 3 ist; und
R⁷ Ethyl oder Propyl ist;
wobei die Stickstoffschutzgruppe Bn, BOC, Cbz, Fmoc, Trifluoracetyl, Triphenylmethyl, Acetyl oder Ts ist;
mit der Maßgabe, dass die Verbindung nicht die folgende Formel aufweist:

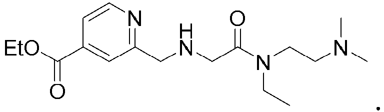

12. Verbindung gemäß Anspruch 11, wobei die Verbindung die folgende Formel aufweist: 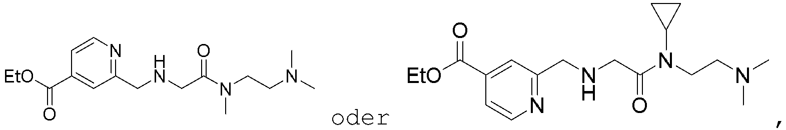 oder ein pharmazeutisch verträgliches/r Salz, Solvat, Hydrat, Polymorph, Co-Kristall, Tautomer, Stereoisomer oder isotopenmarkiertes Derivat davon.

13. Pharmazeutische Zusammensetzung, umfassend eine Verbindung gemäß einem der Ansprüche 1-12 oder ein(en) pharmazeutisch verträgliches/n Salz, Solvat, Hydrat, Polymorph, Co-Kristall, Tautomer, Stereoisomer oder isotopenmarkiertes Derivat und gegebenenfalls einen pharmazeutisch verträglichen Hilfsstoff.

14. Verbindung gemäß einem der Ansprüche 1 bis 12 oder pharmazeutische Zusammensetzung gemäß Anspruch 13 zur Verwendung bei der Behandlung einer proliferativen Erkrankung, bei der KDM5 eine Rolle spielt, wobei eine therapeutisch wirksame Menge der Verbindung oder der pharmazeutischen Zusammensetzung einem Subjekt zu verabreichen ist.

15. Verbindung zur Verwendung oder pharmazeutische Zusammensetzung zur Verwendung gemäß Anspruch 14, wobei die proliferative Erkrankung ein Krebs ist, ausgewählt aus einem Karzinom, einem Sarkom, Lungenkrebs, Brustkrebs, Leberkrebs, Bauchspeicheldrüsenkrebs, Magenkrebs, Eierstockkrebs, Dickdarmkrebs, Leukämie und multiplem Myelom, wobei der Lungenkrebs ein nichtkleinzelliger Lungenkrebs ist und das Sarkom ein Ewing-Sarkom ist.

## Revendications

1. Composé de formule (I-B) : 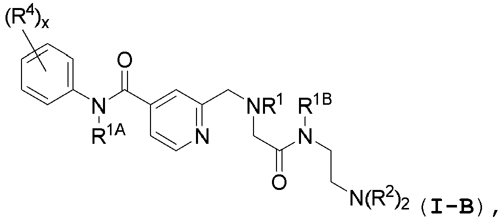 ou un sel pharmaceutiquement acceptable, un solvate, un hydrate, un polymorphe, un co-cristal, un tautomère, un stéréoisomère ou un dérivé marqué isotopiquement de celui-ci, dans lequel :
R¹ est un atome d'hydrogène, un alkyle éventuellement substitué ou un groupe protecteur d'azote ;
R^{1A} est un atome d'hydrogène, un acyle éventuellement substitué, un alkyle éventuellement substitué, un carbocyclyle éventuellement substitué ou un groupe protecteur d'azote ;
R^{1B} est un atome d'hydrogène, un alkyle éventuellement substitué, un carbocyclyle éventuellement substitué ou un groupe protecteur d'azote ;
chaque occurrence de R² est indépendamment un alkyle éventuellement substitué ou un groupe protecteur d'azote ;
chaque R⁴ est indépendamment un alkyle éventuellement substitué, un alcényle éventuellement substitué, un alcynyle éventuellement substitué, un carbocyclyle éventuellement substitué, un hétérocyclyle éventuellement substitué, un aryle éventuellement substitué, un hétéroaryle éventuellement substitué, - OR^{A}, -N(R^{B})₂ ou -SR^{A}, ou éventuellement deux occurrences de R⁴ sont prises ensemble avec leurs atomes intercalaires pour former un cycle hétérocyclique substitué ou non substitué ou un cycle hétéroaryle substitué ou non substitué ; et
x vaut 0, 1, 2, 3, 4 ou 5 ;
où le groupe protecteur d'azote est Bn, BOC, Cbz, Fmoc, trifluoroacétyle, triphénylméthyle, acétyle ou Ts.

2. Composé selon la revendication 1, ou un sel pharmaceutiquement acceptable, un solvate, un hydrate, un polymorphe, un co-cristal, un tautomère, un stéréoisomère ou un dérivé marqué isotopiquement de celui-ci, qui est 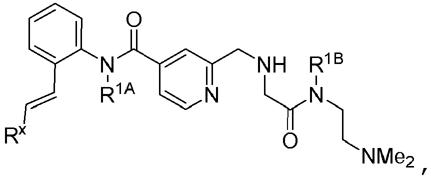 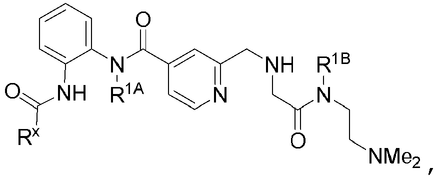 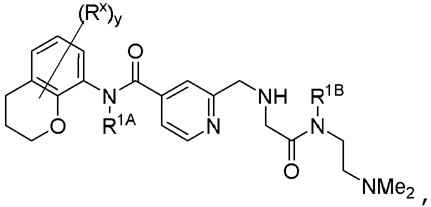 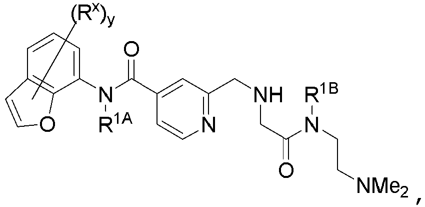 ou 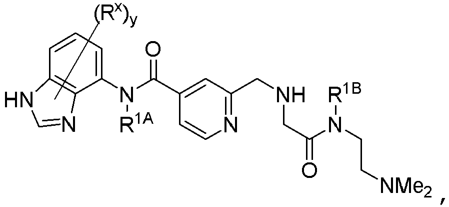 dans lequel :
R^{x} est un acyle éventuellement substitué, un alkyle éventuellement substitué, -O(alkyle éventuellement substitué) ou -NO₂ ; et
y vaut 0, 1, 2, 3, 4, 5, 6, 7, 8 ou 9, selon ce que la valence permet.

3. Composé selon la revendication 1, ou un sel pharmaceutiquement acceptable, un solvate, un hydrate, un polymorphe, un co-cristal, un tautomère, un stéréoisomère ou un dérivé marqué isotopiquement de celui-ci, dans lequel au moins une occurrence de R⁴ est un alcényle éventuellement substitué, dans lequel au moins une occurrence de R⁴ est -OR^{A}, et R^{A} est un atome d'hydrogène, un C₁₋₆ alkyle éventuellement substitué, ou un aryle éventuellement substitué, ou dans lequel au moins une occurrence de R⁴ est -NHC(=O)R^{x}, et R^{x} est un C₁₋₆ alkyle éventuellement substitué ou un alcényle éventuellement substitué.

4. Composé selon l'une quelconque des revendications 1 à 3, ou un sel pharmaceutiquement acceptable, un solvate, un hydrate, un polymorphe, un co-cristal, un tautomère, un stéréoisomère ou un dérivé marqué isotopiquement de celui-ci, dans lequel x vaut 0, 1 ou 2.

5. Composé selon l'une quelconque des revendications 1 à 4, ou un sel pharmaceutiquement acceptable, un solvate, un hydrate, un polymorphe, un co-cristal, un tautomère, un stéréoisomère ou un dérivé marqué isotopiquement de celui-ci, dans lequel R¹ est un atome d'hydrogène, ou
R^{1B} est un C₁₋₆ alkyle éventuellement substitué ou un carbocyclyle éventuellement substitué ; ou
au moins une occurrence de R² est un C₁₋₆ alkyle éventuellement substitué ; ou
au moins une occurrence de R² est un méthyle non substitué ; ou
les deux occurrences de R² sont un méthyle non substitué.

6. Composé selon l'une quelconque des revendications 1 à 4, ou un sel pharmaceutiquement acceptable, un solvate, un hydrate, un polymorphe, un co-cristal, un tautomère, un stéréoisomère ou un dérivé marqué isotopiquement de celui-ci, dans lequel
R¹ est un atome d'hydrogène et R^{1B} est un C₁₋₆ alkyle éventuellement substitué ou un carbocyclyle éventuellement substitué ; ou
R¹ est un atome d'hydrogène, R^{1B} est un C₁₋₆ alkyle éventuellement substitué ou un carbocyclyle éventuellement substitué et au moins une occurrence de R² est un C₁₋₆ alkyle éventuellement substitué.

7. Composé selon l'une quelconque des revendications 1 à 4, ou un sel pharmaceutiquement acceptable, un solvate, un hydrate, un polymorphe, un co-cristal, un tautomère, un stéréoisomère ou un dérivé marqué isotopiquement de celui-ci, dans lequel
R¹ est un atome d'hydrogène, R^{1B} est un C₁₋₆ alkyle éventuellement substitué ou un carbocyclyle éventuellement substitué et au moins une occurrence de R² est un méthyle non substitué ; ou
R¹ est un atome d'hydrogène, R^{1B} est un C₁₋₆ alkyle éventuellement substitué ou un carbocyclyle éventuellement substitué et les deux occurrences de R² sont un méthyle non substitué.

8. Composé qui est : 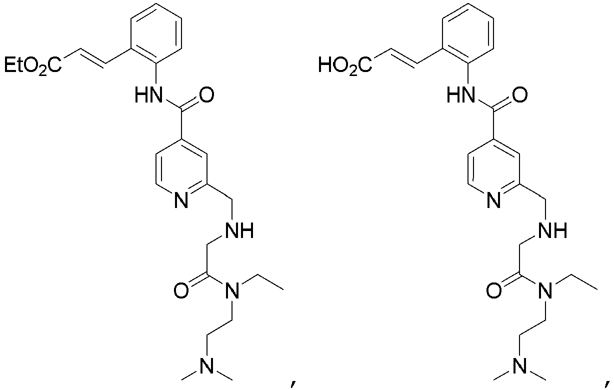 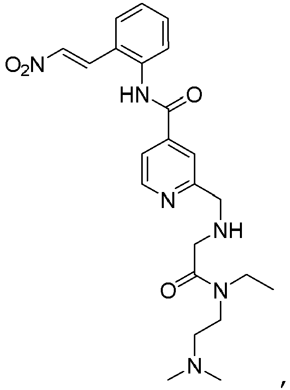 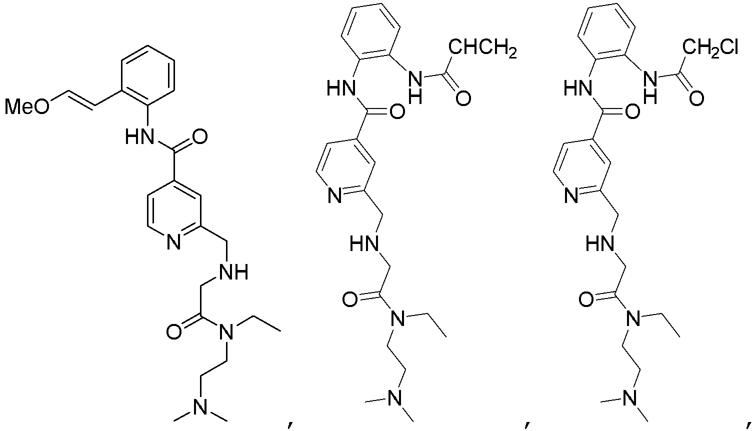 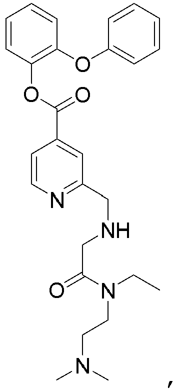 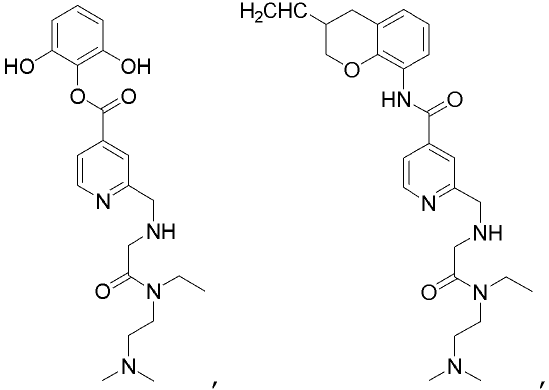 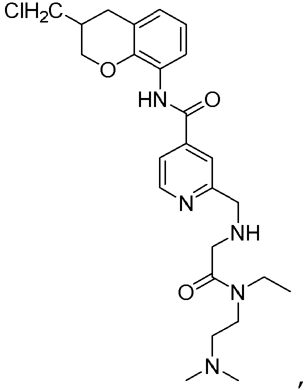 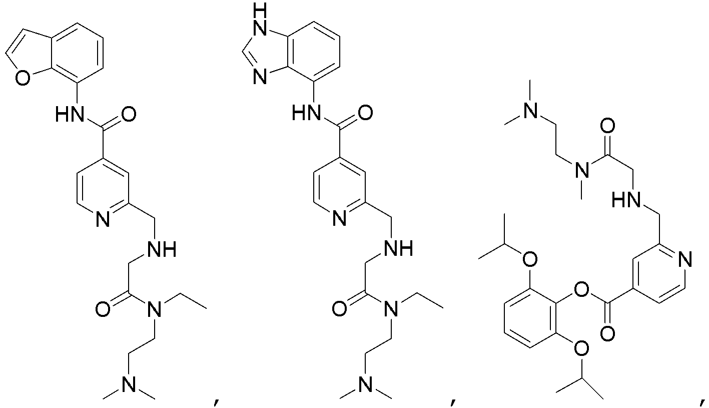 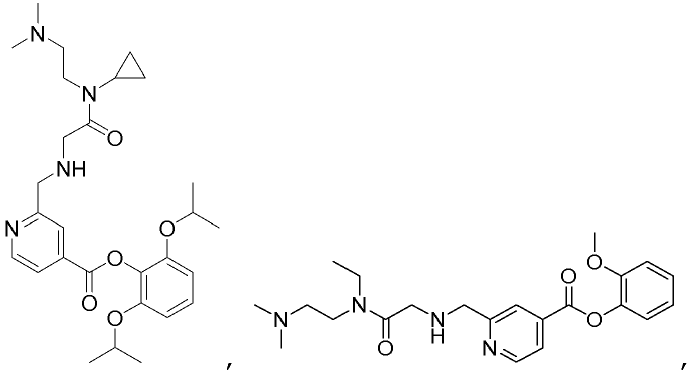 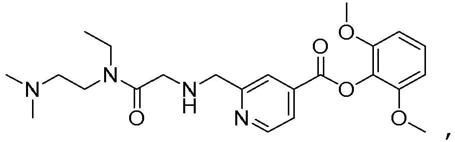 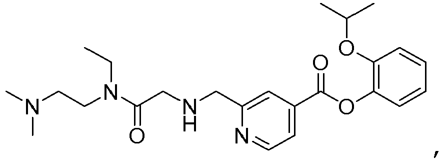 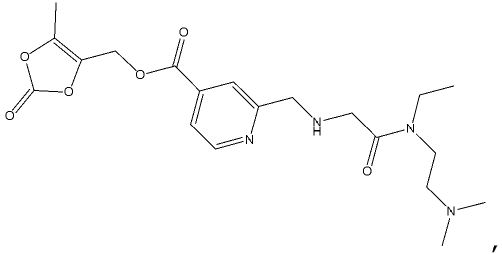 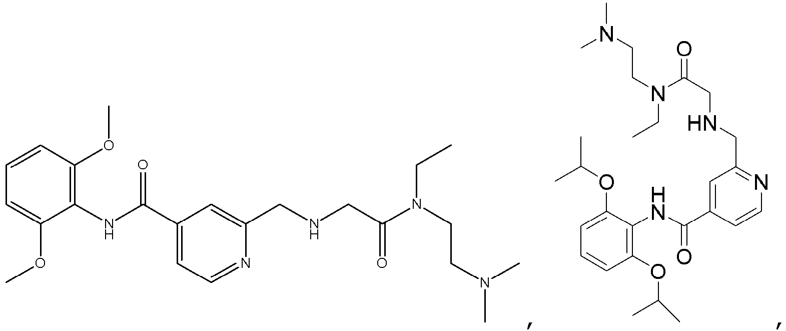 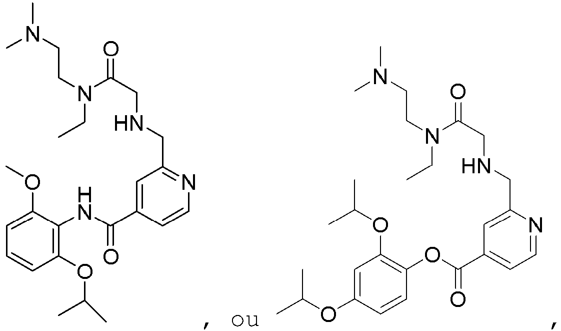 ou un sel pharmaceutiquement acceptable, un solvate, un hydrate, un polymorphe, un co-cristal, un tautomère, un stéréoisomère ou un dérivé marqué isotopiquement de celui-ci.

9. Composé selon la revendication 8, qui est : 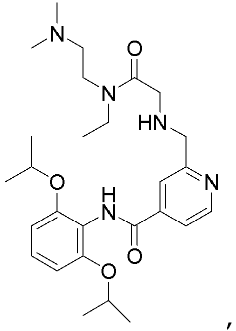 ou un sel pharmaceutiquement acceptable de celui-ci.

10. Composé selon la revendication 8, qui est : 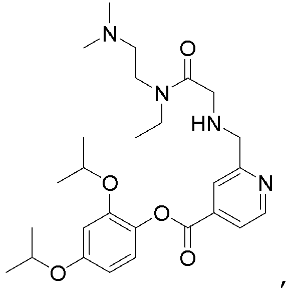 ou un sel pharmaceutiquement acceptable de celui-ci.

11. Composé de formule (II) : 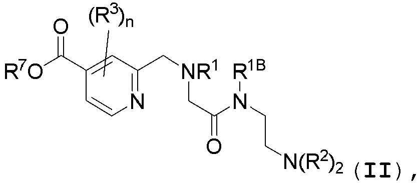 ou un sel pharmaceutiquement acceptable, un solvate, un hydrate, un polymorphe, un co-cristal, un tautomère, un stéréoisomère ou un dérivé marqué isotopiquement, dans lequel :
R¹ est un atome d'hydrogène, un alkyle éventuellement substitué ou un groupe protecteur d'azote ;
R^{1B} est un atome d'hydrogène, un méthyle, un carbocyclyle éventuellement substitué ou un groupe protecteur d'azote ;
chaque occurrence de R² est indépendamment un alkyle éventuellement substitué ou un groupe protecteur d'azote ;
chaque occurrence de R³ est indépendamment un halogène, un acyle éventuellement substitué, un alkyle éventuellement substitué, un alcényle éventuellement substitué, un alcynyle éventuellement substitué, un carbocyclyle éventuellement substitué, un hétérocyclyle éventuellement substitué, un aryle éventuellement substitué, un hétéroaryle éventuellement substitué, -CN, -SCN, -NO₂, -N₃, -OR^{A}, -N(R^{B})₂ ou -SR^{A};
chaque occurrence de R^{A} est indépendamment un atome d'hydrogène, un acyle éventuellement substitué, un alkyle éventuellement substitué, un alcényle éventuellement substitué, un alcynyle éventuellement substitué, un carbocyclyle éventuellement substitué, un hétérocyclyle éventuellement substitué, un aryle éventuellement substitué, un hétéroaryle éventuellement substitué ;
chaque occurrence de R^{B} est indépendamment un atome d'hydrogène, un acyle éventuellement substitué, un alkyle éventuellement substitué, un alcényle éventuellement substitué, un alcynyle éventuellement substitué, un carbocyclyle éventuellement substitué, un hétérocyclyle éventuellement substitué, un aryle éventuellement substitué, un hétéroaryle éventuellement substitué ou un groupe protecteur d'azote ; ou éventuellement deux occurrences de R^{B} sont prises ensemble avec leurs atomes intercalaires pour former un cycle hétérocyclique substitué ou non substitué ou un cycle hétéroaryle substitué ou non substitué ;
n vaut 0, 1, 2 ou 3 ; et
R⁷ est un éthyle ou un propyle ;
où le groupe protecteur d'azote est Bn, BOC, Cbz, Fmoc, trifluoroacétyle, triphénylméthyle, acétyle ou Ts ;
à condition que le composé ne soit pas de formule :

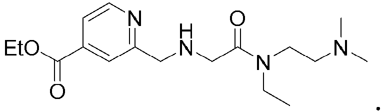

12. Composé selon la revendication 11, dans lequel le composé est de formule : 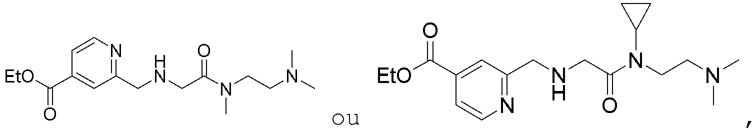 ou un sel pharmaceutiquement acceptable, un solvate, un hydrate, un polymorphe, un co-cristal, un tautomère, un stéréoisomère ou un dérivé marqué isotopiquement de celui-ci.

13. Composition pharmaceutique comprenant un composé selon l'une quelconque des revendications 1-12, ou un sel pharmaceutiquement acceptable, un solvate, un hydrate, un polymorphe, un co-cristal, un tautomère, un stéréoisomère ou un dérivé marqué isotopiquement, et éventuellement un excipient pharmaceutiquement acceptable.

14. Composé selon l'une quelconque des revendications 1 à 12, ou composition pharmaceutique selon la revendication 13, pour utilisation dans le traitement d'une maladie proliférative dans laquelle KDM5 joue un rôle, dans lequel une quantité thérapeutiquement efficace dudit composé ou de ladite composition pharmaceutique doit être administrée à un sujet.

15. Composé pour utilisation ou composition pharmaceutique pour utilisation selon la revendication 14, dans lequel la maladie proliférative est un cancer choisi parmi un carcinome, un sarcome, le cancer du poumon, le cancer du sein, le cancer du foie, le cancer du pancréas, le cancer de l'estomac, le cancer de l'ovaire, le cancer du côlon, la leucémie et le myélome multiple, dans lequel le cancer du poumon est un cancer du poumon non à petites cellules, et le sarcome est un sarcome d'Ewing.
